# EUROPEAN PATENT SPECIFICATION

(11) **EP 2 529 223 B1**
(45) Date of publication and mention of the grant of the patent: **30.03.2016**
(21) Application number: 10844915.8
(22) Date of filing: 24.09.2010
(51) Int. Cl.: G01N 33/53

(54) **BIOMARKERS FOR CIRCULATING TUMOR CELLS**
BIOMARKER FÜR ZIRKULIERENDE TUMORZELLEN
BIOMARQUEURS POUR CELLULES TUMORALES EN CIRCULATION

(30) Priority: 01.03.2010 US 309131 P; 26.02.2010 US 308780 P; 27.01.2010 US 298845 P
(43) Date of publication of application: 05.12.2012
(60) Divisional application: 16152292.5
(73) Proprietor: Duke University, Durham, NC 27705 (US)
(72) Inventor: GARCIA-BLANCO, Mariano, A., Durham, NC 27705 (US); ARMSTRONG, Andrew, J., Durham, NC 27705 (US); GEORGE, Daniel, J., Durham, NC 27705 (US); OLTEAN, Sebastian, Durham, NC 27705 (US)
(74) Representative: Grünecker Patent- und Rechtsanwälte PartG mbB
(86) International application number: PCT/US2010/050223
(87) International publication number: WO 2011/093927

(56) References cited:
- WO-A1-2009/036968
- US-A1- 2009 305 963
- US-B1- 6 566 063
- AKTAS BAHRIYE ET AL: "Stem cell and epithelial-mesenchymal transition markers are frequently overexpressed in circulating tumor cells of metastatic breast cancer patients", BREAST CANCER RESEARCH, CURRENT SCIENCE, LONDON, GB, vol. 11, no. 4, 9 July 2009 (2009-07-09), page R46, XP021061311, ISSN: 1465-5411, DOI: 10.1186/BCR2333
- TOMASKOVIC-CROOK EVA ET AL: "Epithelial to mesenchymal transition and breast cancer.", BREAST CANCER RESEARCH : BCR 2009, vol. 11, no. 6, 2009, page 213, XP55063063, ISSN: 1465-542X
- POLLACK V ET AL: "Oncostatin M-induced effects on EMT in human proximal tubular cells: differential role of ERK signaling", AMERICAN JOURNAL OF PHYSIOLOGY: RENAL, FLUID AND ELECTROLYTEPHYSIOLOGY, AMERICAN PHYSIOLOGICAL SOCIETY, US, vol. 293, no. 5, 1 November 2007 (2007-11-01), pages F1714-F1726, XP002581193, ISSN: 0363-6127, DOI: 10.1152/AJPRENAL.00130.2007 [retrieved on 2007-09-19]

## Description

### CROSS-REFERENCE TO RELATED APPLICATIONS

This application claims the benefit of priority to United States Provisional Patent Application No. 61/298,845 filed January 27, 2010; United States Provisional Patent Application No. 61/308,780 filed February 26, 2010; and United States Provisional Patent Application No. 61/309,131 filed March 1, 2010.

### STATEMENT REGARDING FEDERALLY SPONSORED RESEARCH

This invention was made with government support under federal grant number 5R33CA097502 from the NIH (NCI), and federal grant number 5K12CAlO063904 from the NIH (NCI). The U.S. Government has certain rights to this invention.

### SEQUENCE LISTING

The sequence listing is filed with the application in electronic format only. The sequence listing text file "B2442027.txt" was created on September 24, 2010 and is 131,287 bytes in size.

### FIELD

The disclosure relates to methods for the detection and prognosis of cancer. Moreover, the disclosure provides methods for detecting circulating tumor cells (CTCs) that include the identification, detection, and optional enumeration of one or more biomarkers associated with CTCs that can be used in methods relating to a prognosis, diagnosis, or the treatment of cancer in a subject.

### BACKGROUND

Most metazoan cells can be classified as either epithelial or mesenchymal based on morphology, behavior and molecular signatures. Epithelial cells are generally polar in the apico-basal direction, adherent to adjacent cells in the plane perpendicular to the polarity, and non-motile in the polar direction. Mesenchymal cells, in contrast, lack polarity, do not form tight interactions with neighboring cells, and are motile. In adult animals epithelial and mesenchymal cells remain stably in one state or the other; that is, an epithelial cell does not change its properties and become mesenchymal. During development, however, epithelial cells of the early embryo give rise to all three embryonal layers (endoderm, mesoderm and ectoderm), which include mesenchymal cells (Hay, E.D., et al. Am. J. Kidney Dis. 1995, 26, 678-690). Therefore, these early embryonal cells have the ability to transition between epithelial and mesenchymal states, a property sometimes referred to as epithelial plasticity. Embryos have been shown to undergo epithelial-mesenchymal transitions (EMTs) as well as mesenchymal-epithelial transitions (METs) (Acloque, H., et al. J. Clin. Invest. 2009, 119, 1438-1449).

Circulating tumor cells (CTCs) are cells that have detached from a primary tumor and circulate in the bloodstream. CTCs may constitute seeds for subsequent growth of additional tumors (metastasis) in different tissues. Thus, detection of CTCs can provide for diagnosis and/or prognosis for overall survival and therapeutic implications in subjects with cancers such as metastatic prostate and breast cancer. The number of CTCs in any patient sample (e.g., a blood sample) can be very small, which can make detection difficult. Current methods for detecting CTCs are based on the detection of epithelial cell adhesion molecule (EpCAM) expression, which is a biomarker associated with epithelial cells. Such methods can under-detect CTCs under circumstances where cells undergo a decrease or loss of EpCAM expression, such as biologic processes including EMT. Because of the important role CTCs can play in the diagnosis, monitoring, and prognosis of disease in patients having cancer, any shortcoming in the detection technology needs to be addressed by the art.

Accordingly, there is a need for methods and systems for detecting CTCs that do not rely on existing capture technologies, and methods for correlating CTC detection to diagnosis, monitoring, and prognosis of disease in cancer Aktas Bahriye et al. (Breast Cancer Research, 2009), discloses the detection of circulating tumour cells using among others Twist, Akt2 and PI3K as EMT markers.

### SUMMARY

In an aspect, the invention provides a method for detecting a circulating tumor cell (CTC) in a biological sample, the method comprising detecting at least one epithelial mesenchymal transition (EMT) biomarker in the biological sample, wherein the at least one EMT biomarker is vimentin.

In an aspect, the disclosure provides a kit for detecting a circulating tumor cell (CTC) in a biological sample, the kit comprising an antibody to at least one EMT biomarker and instructions for use.

In an aspect, the disclosure provides a method of predicting responsiveness of a subject having cancer to a course of cancer treatment, the method comprising: determining the level or presence of expression of at least one EMT biomarker to obtain an EMT biomarker profile and/or optionally a gene expression pattern for a CTC; and predicting the responsiveness of the subject to the cancer drug based on the EMT biomarker profile and/or optional gene expression pattern. In some embodiments the method includes: determining the level or presence of expression of at least one EMT biomarker in a sample from the subject to obtain a biomarker profile and optionally a gene expression pattern in a CTC for the subject; identifying the type of cancer from the biomarker profile and/or optional gene expression pattern, and optionally characterizing the stage of the cancer; and predicting responsiveness of the subject to the cancer drug based on any one of the biomarker pattern, the optional gene expression pattern, the type of cancer, or the stage of the cancer. Embodiments of this aspect can include detecting a number of cells captured and enumerated from a blood sample using at least one EMT biomarker applied to a sample from the subject. These cells that express the EMT biomarker are thereby captured using the EMT biomarker and could then be used to obtain a gene expression pattern in CTCs for the subject; to predict responsiveness of the subject to the cancer drug based on the obtained gene expression pattern, and for the detection of other biomarkers in these CTCs to assist in guiding therapy of that subject. These cells could also be used to measure the level of the specified EMT biomarker or other EMT biomarkers.

In an aspect, the disclosure provides a method of assessing the number of CTCs using both the traditional EpCAM based capture methodology and an EMT-marker based capture methodology. This EMT-based capture may replace or complement existing CTC capture technologies. The further capture, enumeration, and characterization of these CTCs using EMT antigen capture may further targeting delivery of a cancer drug in a subject having cancer comprising administering to the subject a cancer drug linked to an antibody specific for at least one EMT biomarker or specific drugs based on a gene expression profile or presence of this EMT biomarker.

In an aspect, the disclosure provides a method of estimating the prognosis of a subject with cancer as well as permitting a further characterization of CTCs that may predict for therapeutic responsiveness, the method comprising: determining the level of or presence of expression of at least one EMT biomarker in a sample from the subject to determine the number of CTCs in the subject and to obtain a gene expression pattern for the subject; and providing a prognosis to the subject based on the gene expression or biomarker profile pattern obtained.

In an aspect, the disclosure provides a method for monitoring progression of cancer in a subject undergoing therapeutic treatment, the method comprising detecting the level of expression or presence of expression of at least one EMT biomarker and the quantification of CTCs captured using this method in blood samples taken from the subject at a first and a second time; and comparing the first and second levels of expression; wherein a detected difference in the level of expression of the at least one EMT biomarker in the first and second samples over time indicates a change in the progression status of the cancer.

In an aspect, the disclosure provides a method for detecting cancer in a subject, the method comprising determining the presence of CTCs that express at least one EMT biomarker in a sample from the subject as compared to a normal or control sample, wherein an increased level of at least one EMT biomarker indicates presence of cancer progression or metastatic spread in the subject.

In an aspect, the disclosure provides a method of treating cancer in a subject comprising administering to the subject a cancer drug linked to an antibody that specifically binds at least one EMT biomarker.

Other aspects and embodiments of the disclosure will become apparent by consideration of the detailed description and accompanying drawings.

### BRIEF DESCRIPTION OF THE DRAWINGS

Figure 1. (A) depicts a schematic representation of the IIIb and IIIc alternatively spliced isoforms of FGFR2. **(B)** is a schematic of the pRIIIcl² minigene and the fluorescence read-out. **(C)** is an RT-PCR analysis of the reporter (upper panel) and endogenous FGFR2 (lower panel). **(D)** are epifluorescence and phase-contrast pictures of clones AT3-M and AT3-T.
Figure 2. (A) depicts examples of clusters of DsRED positive cells formed by AT3-M cells upon treatment with conditioned media from clone AT3-T. **(B)** depicts flow cytometry analysis of the same experiment.
Figure 3. (A) depicts growth curves for clones AT3-T and AT3-M. **(B)** is graph of growth of AT3-M, AT3-T, and DT cells in soft agar. **(C)** depicts a sacrifice curve for rats injected with AT3-M or AT3-T cells. **(D)** depicts a comparison of tumor volumes resulting from AT3-T and AT3-M injection.
Figure 4**. (A)** a representative example of cells that express both RFP and GFP at the periphery of an AT3-M tumor stably transfected with Gint and pRIIIcl² reporters. **(B)** a representative example of a section from an AT3-T tumor stably transfected with GFP and pRIIIcl² reporters.
**Figure 5** a representative example of cells that express both RFP and GFP at the periphery of an AT3-M tumor stably transfected with Gint and pRIIIcl² reporters.
Figure 6**. (A)** representative pictures of cells for the scratch-wound assay. **(B)** a quantification of migration. **(C)** an invasion assay using Matrigel coated membranes. **(D)** a quantification of invasion assay results.

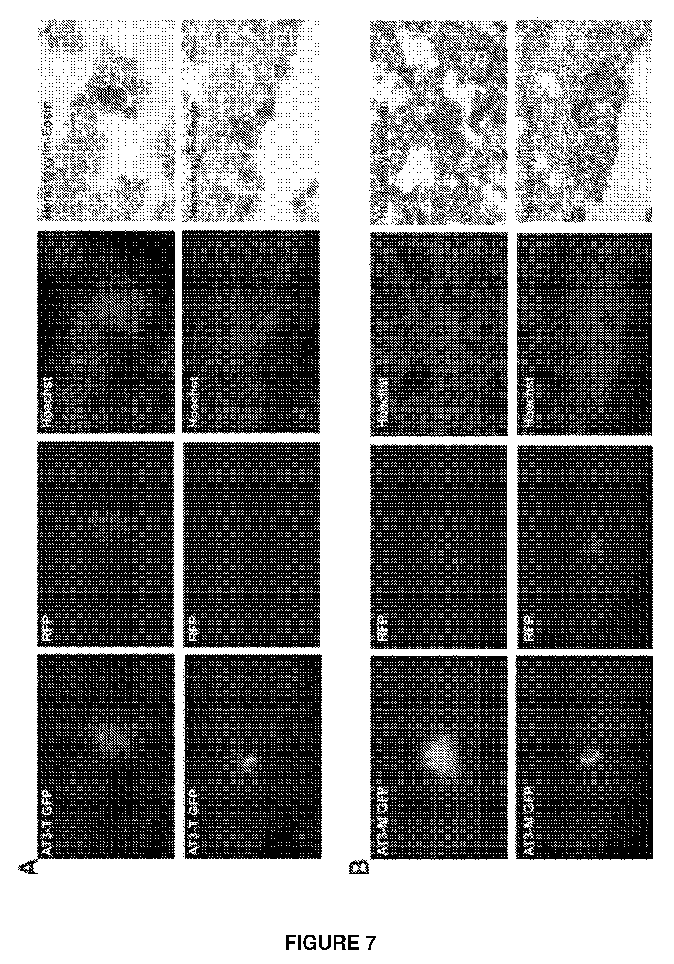

**Figure 7** are metastatic foci in lungs from animals with tumors from either AT3-T or AT3-M clones (stably transfected with GFP and pRIIIcI² reporters). **(A)** (upper panel) is an example of a section exhibiting the pattern for clone AT3-T (i.e. GFP+, DsRED+) in a metastatic focus and (lower panel) an example of a section exhibiting a plastic pattern for clone AT3-T (i.e. GFP+, DsRED-) in a metastatic focus. **(B)** (upper panel) is an example of a section exhibiting the pattern for clone AT3-M (i.e. GFP+, DsRED-) in a metastatic focus and (lower panel) an example of a section exhibiting a plastic pattern for clone AT3-M (i.e. GFP+, DsRED+) in a metastatic focus.
**Figure 8A** a membrane with serial two-fold dilutions of whole cell Iysates cut in half and immunoblotted for CD 133 (upper panel) or β-actin (lower panel). **(B)** a membrane with serial twofold dilutions of whole cell lysates cut in half and immunoblotted for CD44 (upper panel) or β-actin (lower panel).
**Figure 9** depicts a model comparing stem cell-like character and epithelial mesenchymal phenotype.

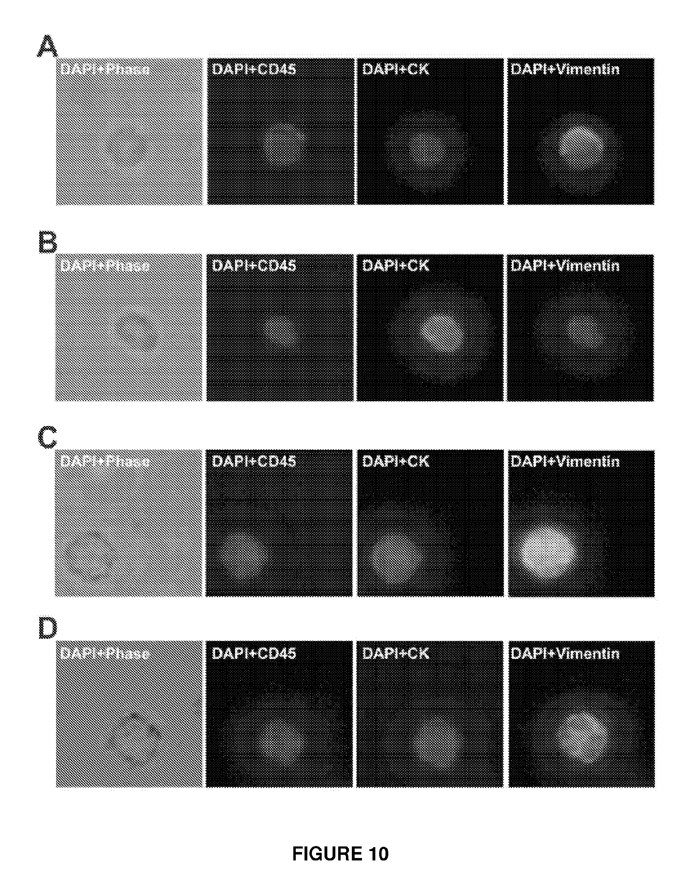

**Figure 10** depicts CTCs from patients with prostate adenocarcinoma. **(A)** illustrates an example of a leukocyte from a human peripheral blood mononuclear cell (PMBC) sample: CD45 (+), CK (-), and vimentin (+). **(B)** illustrates an example of a CD45 (-), CK (+), and vimentin (-) cell from a patient with metastatic breast cancer. **(C)** illustrates an example of a CD45 (-), CK (+), vimentin (+) from a patient with metastatic breast cancer (mBC). **(D)** illustrates an example of a CD45 (-), CK (+), vimentin (+) from a patient with metastatic progressive castrate-resistant prostate cancer (mCRPC).
**Figure 11** depicts immunofluorescent images of CTCs from patients with mCRPC and mBC.
**Figure 12** depicts immunofluorescent images of CTCs from patients with mCRPC and mBC.
**Figure 13** depicts immunofluorescent images of CTCs from patients with mCRPC and mBC.
**Figure 14** depicts immunofluorescent images of CTCs from patients with mCRPC and mBC.
**Figure 15** depicts immunofluorescent images of CTCs from patients with mCRPC and mBC.
**Figure 16** depicts immunofluorescent images of CTCs from patients with mCRPC and mBC.

### DETAILED DESCRIPTION

Before any embodiments are described in detail, it is to be understood that the claims are not limited to the details of construction and the arrangement of components set forth in the following description or illustrated in the included drawings.

In a general sense, the disclosure provides biomarkers that have been identified to be associated with circulating tumor cells (CTCs). As described herein, one or more biomarkers of epithelial mesenchymal transition (EMT) are detectable on CTCs of patients afflicted with common epithelial malignancies. These transitional cells often display stem cell-like characteristics (sternness) and/or plasticity. Further, the disclosure provides description that metastatic propensity and epithelial phenotypic changes correlate with alternative splicing of the FGFR2 gene. The disclosure also provides that, as illustrated in the non-limiting Examples, transitional cells are found in cancer patients where many CTCs co-expressed biomarkers associated with epithelial and mesenchymal cells.

Thus, as described below EMT biomarker expression can be used to detect and quantify CTCs in a biological sample. Accordingly, methods comprising detection of EMT biomarker expression, or detection of CTCs, or a combination thereof, can be used to assess cancer prognosis, tumor invasiveness, risk of metastasis, or to stage tumors. As one of skill in the art will appreciate, any suitable method for evaluating EMT biomarker expression can be used to evaluate EMT biomarker expression according to the methods described herein including, but not limited to, detection with antibodies, real time RT-PCR, Northern analysis, Western analysis, and flow cytometry.

As described herein the ability for a cell to transition easily between epithelial-like and mesenchymal-like states (phenotypic plasticity) is a relevant determinant of malignant fitness more so than the properties of the end states. While these epithelial transitions are phenotypic, the propensity to transition (plasticity) among carcinoma cells may be determined by genotype. The majority of plastic cells may inhabit transitional intermediate states with properties of both epithelium and mesenchyme, and that these transitional cells may be particularly malignant. Such cells may be detected in: (1) tumors where the cancer cells have mixed histology, which indeed have been observed and have been classified as highly aggressive (e.g., clonal sarcomatous carcinomas of epithelial origin, which exhibit an extremely aggressive behavior, such as sarcomatoid renal cell carcinoma and carcinosarcoma of the prostate); and (2) cancer cells co-expressing epithelial and mesenchymal markers, as described herein.

The disclosure, as illustrated by the non-limiting embodiments in the Examples, provides for identification of cells that possess an intermediate phenotype - expressing epithelial and mesenchymal isoforms of FGFR2, having epithelial-like morphology and gene expression patterns, while also displaying mesenchymal cell-like migration, tumor formation, and metastases. In embodiments, these cells are identified in patients with advanced cancer, metastatic adenocarcinoma, and metastatic breast and prostate carcinomas. In some embodiments, the cells comprise CTCs. In some embodiments the CTCs co-expresses biomarkers including, for example, EpCAM, cytokeratin, and vimentin, which identify cells as both epithelial- and mesenchymal-like. In some embodiments, these CTCs in intermediate phenotypic states are identified by detecting EMT biomarkers and provide a diagnosis and/or prognosis of the state and/or degree of malignancy of a cancer.

In an aspect the disclosure provides a method for detecting CTCs in a biological sample, the method comprising detecting at least one epithelial mesenchymal transition (EMT) biomarker in the biological sample. In some embodiments such as illustrated in the Examples, biomarkers of EMT are present on the CTCs of patients with common epithelial malignancies. In some embodiments methods that include detection and identification of alternative splice variants of the FGFR2 gene are used to correlate to metastatic propensity and epithelial phenotypic in a CTC.

Thus, EMT biomarker expression may be used to detect CTCs. EMT biomarker expression, or detection of CTCs, or a combination thereof, may be used to assess cancer prognosis, tumor invasiveness, risk of metastasis, or to stage tumors. As mentioned above, the methods described herein can include any suitable method for evaluating EMT biomarker expression including, but not limited to, detection with antibodies, real time RT-PCR, Northern analysis, magnetic particles (e.g., microparticles or nanoparticles), Western analysis, and any method or system involving flow cytometry. In some embodiments, the methods and EMT biomarkers can be used in a commercially available system such as a system that has been approved by a regulatory agency (e.g., FDA) including, for example, CellSearch® technology (Veridex LLC). Thus, the methods can incorporate standard protocols that are known in the art. For example, embodiments comprising CellSearch® technology can include detecting the presence of an EMT biomarker, and correlated to quantifying the number of circulating tumor cells (CTCs) a biological sample, (e.g., blood collected from women in need of a new treatment regimen for metastatic breast cancer, or men in need of treatment for mCRPC). Typical protocols can include drawing blood sample sizes of about 15 mL that can be collected at any particular time (suitably when the patient starts the new therapy, and then again at three to four week intervals). The number of CTCs can be correlated with disease response or progression as determined by standard radiology studies (e.g., CT scans) performed every nine to 12 weeks.

In an aspect, the invention relates to a method for detecting a circulating tumor cell (CTC) in a biological sample, wherein the method comprises detecting at least one epithelial mesenchymal transition (EMT) biomarker in the biological sample, wherein the at least one EMT biomarker is vimentin. As noted above, a biological sample can be from any tissue or fluid from an organism. In some embodiments the biological sample is from a bodily fluid or tissue that is part of, or associated with, the lymphatic system or the circulatory system of the organism. In some embodiments the biological sample is a blood sample.

The epithelial mesenchymal transition (EMT) and cellular plasticity biomarkers used in the methods described herein are associated with circulating tumor cells (CTCs). Accordingly, in various embodiments the methods include detecting the presence of one or more EMT biomarker and correlating that detection with the presence of a CTC, optionally quantifying the number of CTCs in the sample. As discussed herein, EMT biomarkers can include any detectable biomolecule that is associated with a transitional cell that exhibits characteristics (e.g., phenotype, or surface antigen or gene expression profiles, etc.) of plasticity, stem-like properties, invasiveness, and/or chemo-resistance of a cell. In some non-limiting embodiments, the EMT biomarker includes any of vimentin, N-cadherin, O-cadherin, E-cadherin, FGFR2 splice variant isoforms (such as, for example FGFR2 that includes or excludes either exon IIIc or exon IIIb), or CD133, or any combination of two or more thereof. In some embodiments, the EMT biomarker can include one or more of vimentin (polypeptide SEQ ID NO: 14 encoded by polynucleotide SEQ ID NO: 13), N-cadherin (polypeptide SEQ ID NO: 2 encoded by polynucleotide SEQ ID NO: 1; polypeptide SEQ ID NO: 16 encoded by polynucleotide SEQ ID NO: 15), O-cadherin (polypeptide SEQ ID NO: 4 encoded by polynucleotide SEQ ID NO: 3; polypeptide SEQ ID NO: 18 encoded by polynucleotide SEQ ID NO: 17), E-cadherin (polypeptide SEQ ID NO: 12 encoded by polynucleotide SEQ ID NO: 11; polypeptide SEQ ID NO: 24 encoded by polynucleotide SEQ ID NO: 23), FGFR2 (polypeptide SEQ ID NO: 8 encoded by polynucleotide SEQ ID NO: 7; polypeptide SEQ ID NO: 10 encoded by polynucleotide SEQ ID NO: 9; polypeptide SEQ ID NO: 22 encoded by polynucleotide SEQ ID NO: 21), and CD133 (polypeptide SEQ ID NO: 6 encoded by polynucleotide SEQ ID NO: 5; polypeptide SEQ ID NO: 20 encoded by polynucleotide SEQ ID NO: 19). In some embodiments, the EMT biomarker can include one or more of N-cadherin, for example human N-cadherin (for example SEQ ID NO: 16, CCDS ID No: CCDS11891.1); O-cadherin, for example human O-cadherin (for example SEQ ID NO: 18, CCDS ID No: CCDS10803.0); E-cadherin, for example human E-cadherin (for example SEQ ID NO: 24, CCDS ID No: CCDS10869.1); CD133, for example human CD133 (for example SEQ ID NO: 20, CCDS ID No: CCDS47029.1); FGFR2, for example human FGFR2 (for example SEQ ID NO: 22, CCDS ID No: CCDS31298.1); and vimentin, for example human vimentin (for example SEQ ID NO: 14, Accession No. BC000163). It will be understood by one of skill in the art that when reference is made to polynucleotides that encode polypeptides in the above embodiments as well as embodiments throughtout, the polynucleotide can be disclosed as either an RNA (e.g., mRNA) or a DNA (e.g., cDNA).

The EMT biomarkers can be associated with any organism (ortholog) and in certain embodiments are EMT biomarkers associated with a human. Any portion or the entirety of an EMT biomarker can be used for detecting in the methods described herein such as, for example, an epitope of an EMT biomarker protein that binds to an antibody, or a nucleic acid sequence of an EMT biomarker an expressed or transcribed mRNA molecule that is complementary to a reporter nucleic acid probe or primer. In some embodiments, the methods provide for detecting expression of at least two EMT biomarkers. In certain embodiments, expression of vimentin and E-cadherin are detected. In certain embodiments, expression of N-cadherin and O-cadherin are detected. This measure may be used alone or in combination with another method to detect CTCs. In certain embodiments, the methods described herein may be used as a supplemental method in conjunction with CellSearch@ Circulating Tumor Cell Test (noted above). Thus, embodiments provide for a method as part of a dual or complementary detection system that can be used to detect and optionally quantify CTCs in a sample (e.g., comprising the detection of EpCAM and at least one EMT biomarker). The expression of at least one EMT biomarker may be used to isolate CTCs. The expression of at least one EMT biomarker may be used to count or provide a relative number or amount of CTCs, using any known method for correlating detection of a biomarker to a cell, such as a CTC. CTCs may be detected at the time of, prior to, or after metastasis.

Cancers may include, but are not limited to, breast cancer, colon cancer, lung cancer, prostate cancer, testicular cancer, brain cancer, skin cancer, rectal cancer, gastric cancer, esophageal cancer, sarcomas, tracheal cancer, head and neck cancer, pancreatic cancer, liver cancer, ovarian cancer, lymphoid cancer, cervical cancer, vulvar cancer, melanoma, mesothelioma, renal cancer, bladder cancer, thyroid cancer, bone cancers, carcinomas, sarcomas, and soft tissue cancers. Thus, the disclosure is generally applicable to any type of cancer in which expression of an EMT biomarker occurs. In certain embodiments, the cancer is a solid tumor malignancy. In certain embodiments, the cancer is breast, colon, or prostate cancer.

Expression of at least one EMT biomarker may be detected using any suitable method known in the art, including but not limited to, binding with antibodies or fragment thereof, antibodies tethered to or associated with an imaging agent, expression reporter plasmids, flow cytometry, and any suitable array scanner technology. The antibody or fragment thereof may suitably recognize a particular intracellular protein, protein isoform, or protein configuration.

As used herein, an "imaging agent" or "reporter molecule" is any entity which enhances visualization or detection of the cell to which it is delivered. Any type of detectable reporter molecule/imaging agent can be used in the methods disclosed herein for the detection of one or more EMT biomarker. Such detectable molecules are known in the art and include, for example, magnetic beads, fluorophores, radionuclides, nuclear stains (e.g., DAPI). For example, an imaging agent can include a compound that comprises an unstable isotope (i.e., a radionuclide) or a fluorescent moiety, such as Cy-5, Alexa 647, Alexa 555, Alexa 488, fluorescein, rhodamine, and the like. Suitable radionuclides include both alpha- and beta-emitters. In some embodiments, the targeting vehicle is labeled. In other embodiments, suitable radioactive moieties include labeled polynucleotides and polypeptides which can be coupled to the targeting vehicle. In some embodiments, the imaging agent comprises a radionuclide such as, for example, a radionuclide that emits low-energy electrons (e.g., those that emit photons with energies as low as 20 keV). Such nuclides can irradiate the cell to which they are delivered without irradiating surrounding cells or tissues. Non-limiting examples of radionuclides that are can be delivered to cells include ¹³⁷Cs, ¹⁰³Pd, ¹¹¹In, ¹²⁵I, ²¹¹At, ²¹²Bi and ²¹³Bi, among others known in the art. Further imaging agents suitable for delivery to a cell in accordance with some embodiments include paramagnetic species for use in MRI imaging, echogenic entities for use in ultrasound imaging, fluorescent entities for use in fluorescence imaging (including quantum dots), and light-active entities for use in optical imaging. A suitable species for MRI imaging is a gadolinium complex of diethylenetriamine pentacetic acid (DTPA). For positron emission tomography (PET), ¹⁸F or ¹¹C may be delivered. Other non-limiting examples of reporter molecules are discussed throughout the disclosure.

In an aspect, the disclosure provides a kit for detecting CTCs in a sample. In embodiments, the kit comprises an antibody to at least one EMT biomarker. The antibody in the kit can be connected to or associated with an imaging agent. In embodiments, the kit can comprise an antibody to at least one EMT biomarker, wherein the antibody is associated a magnetic bead. The magnetic bead may be used for ferromagnetic separation and enrichment of CTCs.

Aspects of the disclosure also relate to methods of predicting responsiveness of a subject to a cancer drug. The methods may comprise determining the level of expression of at least one EMT biomarker in a sample from the subject. The level of expression of at least one EMT biomarker may be used to obtain a gene expression pattern in CTCs for the subject. The methods may further comprise predicting responsiveness of the subject to the cancer drug based on the gene expression pattern obtained. Genome variation in CTCs from the subject may also be determined.

Also disclosed are methods of providing a cancer prognosis to a subject. The methods may comprise determining the level of expression of at least one EMT biomarker in a sample from the subject. The level of expression of at least one EMT biomarker may be used to determine the number of CTCs in the sample. The CTCs may be captured using at least one EMT biomarker. The level of expression of at least one EMT biomarker may be used to determine a gene expression pattern in the CTCs for the subject. A prognosis may be provided to the subject based on the gene expression pattern obtained.

Also disclosed are methods for following the progress of cancer in a subject. The methods may comprise determining the level of expression of at least one EMT biomarker in samples from the subject at a first and a second time, and comparing the first and second levels of expression. The level of expression of at least one EMT biomarker in the sample may be determined over time, such as following initiation of a new cancer therapy. The level of expression of at least one EMT biomarker in the sample may be used to determine the number or amount of CTCs. An increase between the first and second levels may indicate progression of the cancer. A decrease between the first and second levels may indicate remission or response of the cancer to the therapy. No difference between the first and second levels may indicate arrest or stability in the progression of the cancer.

Also disclosed are methods of screening for cancer in a subject. The methods may comprise determining the level of expression of at least one EMT biomarker in a sample from the subject. The level of expression of at least one EMT biomarker may be used to determine the amount or number of CTCs in the subject. The level of expression of at least one EMT biomarker may be compared to a normal or control sample. An increased level of at least one EMT biomarker may indicate presence of cancer in the subject.

Also disclosed are methods of arresting cell growth or inducing cell death of a cancer cell expressing an EMT biomarker. The methods include contacting the cancer cell with a conjugate capable of mediating intracellular delivery of an agent, such as the antibodies to EMT markers described herein. The agent is capable of arresting or attenuating the growth of the cell or inducing cell death through any mechanism after agent internalization. The cancer cell may be contacted with the *conjugate in vitro, in vivo,* or *ex vivo.* These methods may be useful in treating cancer by directly targeting cancer cells expressing an EMT biomarker for delivery of agents capable of decreasing or arresting cell growth or inducing cell death.

The disclosure also provides for targeted therapeutic methods and molecules that comprise an anti-cancer agent linked to a binding agent that targets at least one EMT as described herein. In some embodiments the link between the anti-cancer agent and the binding agent is a covalent bond. In some embodiments the link is formed by strong electrostatic interactions (hydrogen bonds, hydrophilic/hydrophobic interaction, or oppositely charged moieties, and the like). Any anti-cancer agent can be used in such molecules and therapeutic methods, and can be selected by one of skill in the art based on the type of cancer to be treated, the progress/stage of the cancer, potential adverse drug interactions, dosage requirements, administration schedule, and the like.

### EXAMPLES

### Example 1. Materials and Methods

*Plasmids and cell culture.* The minigene used (pRIIIcl²) was previously described (S. Oltean et al., Proc Natl Acad Sci USA 2006, 103, 14116). All cell lines were cultured in low glucose DMEM (Invitrogen) with 10% FBS and 15 IJg/mL blasticidin. Single cell progenies were isolated from a population of AT3 cells stably transfected with pRIIIcl² minigene by limiting dilution to produce a concentration of 1 cell/10 wells and plated on 96-well plates. Cells were counted using a hemocytometer to obtain an initial concentration of 1 x 10⁵ cells/mL. Through a series of progressive dilutions a final concentration of 1 cell/mL was obtained and 100 IJI were pipetted in each well of three 96-well plates. All wells were monitored through bright field microscopy, those appearing to contain more than one cell were excluded, and those containing single cells were further cultured into 25 mL flasks. 16 of an expected 27 clones were obtained using this procedure in a first round.

To measure cell population growth *rate in vitro,* cells were plated at 50,000/well in 6-well dishes. Viable cells were counted using Trypan Blue staining at 24, 48, 72, and 96h.

*Animals and tumor cell implantation.* Cells were trypsinized, washed, and resuspended in PBS at a final concentration of 3 x 10⁵ cells/mL, and kept on ice for less than 30 minutes before implantation. Cells (3 x 10⁵) were injected subcutis in both flanks of Copenhagen 2331 rats (Harlan Labs, Indianapolis, IN; 75-90 g, 2 months of age). Animals were continuously monitored for tumor growth. All animal procedures were approved by the Duke University Institutional and Animal Care and Use committee and followed NIH guidelines. Sacrifice curves were compared using a Mantel-Haenszel logrank test. Tumor volume was compared using an unpaired t test. Prism 4.0c for the Macintosh (Graphpad, La Jolla, CA) was used for statistical analyses.

*Histological sections and analysis.* Excised tumors and lungs were washed in PBS at room temperature. Depending on the size of the lungs, they were frozen either together or separately. The tumor sections and the lungs were placed in cryomolds, embedded in optimal-cutting-temperature tissue sectioning medium (Sakura Finetek, Torrance, CA), snap-frozen in liquid nitrogen, and stored at 80°C. Slides for fluorescence imaging were prepared as follows: the tissue was incubated for 2-3 h at -20°C to equilibrate the temperature and then sectioned with a microtome. The sections (15 µm) were placed on glass slides, fixed in 4% (wt/vol) paraformaldehyde for 30 min at room temperature, and rinsed in PBS at room temperature. The slides were mounted with gel/mount media (Biomeda, Foster City, CA). The sections were analyzed by using an Olympus (Melville, NY) IX 71 epifluorescence microscope, and images were acquired by using an Olympus DP70 digital camera. Image processing was done with DP Controller software (Olympus). For hematoxylin-eosin staining after fluorescence imaging, the slides were incubated in warm water for 15-20 minutes for the cover slip to come off, slides were dried, and staining was performed according to standard procedure.

*RNA extraction from tumor sections.* Sections were fixed in 4% (wt/vol) paraformaldehyde for 5 minutes, rinsed in PBS, and imaged. DsRED+ and DsRED- regions of the sections were marked on the slide. The slide was immersed in warm water for 5 minutes to remove the coverslip and the DsRED+ and DsRED- regions scraped off. RNA isolation was further performed as described before (N. Masuda, T. Ohnishi, S. Kawamoto, M. Monden, K. Okubo, Nucleic Acids Res 1999, 27, 4436). Briefly, samples were treated with proteinase K in digestion buffer containing SDS, and further isolation of RNA was performed using the RNeasy kit (QIAGEN, Valencia, CA).

*Immunoblots.* Cells were collected from confluent 25 cm² tissues flasks by scraping, washed in PBS, and lysed in sample buffer. Whole cell lysates were serially diluted in sample buffer, fractionated via 7.5% SDS-PAGE, and transferred to PVDF. Membranes were cut in half. The bottom half was probed with anti-β-actin at 1:1000 or 1:5000 (Santa Cruz Biotechnology, CA, 47778) as an internal loading control, while the top half was probed with anti-CD 133 (Santa Cruz Biotechnology, CA, 30219) at 1:200 or anti-CD44 (Santa Cruz Biotechnology, CA, 7946) at 1:200.

*Gene expression analysis.* Triplicate cultures of AT3-M and AT3-T cells were grown to -60% confluency. Total RNA was isolated using the RNeasy kit (Qiagen, Valencia, CA), and triplicate samples were submitted to the Duke Microarray Facility. Gene expression analysis was performed using the R027K rat spotted arrays 3.0 (Operon, Huntsville, AL). Bioinformatical analysis of expression differences between AT3-M and AT3-T cells was done using the GeneSpring GX software version 7.3.1 (Agilent Technologies, Durham, NC). The data files (representing signals for 26,986 gene probes in all six data points, three for AT3-M and three for AT3-T) were normalized using the feature: per Spot and per Chip - intensity dependent (Iowess) normalization. The resulting gene list was used to determine the significantly differentially expressed genes between AT3-M and AT3-T using the "Filtering on Volcano plot" feature with the following characteristics: (1) Test type: Parametric test, don't assume variances equal; (2) Multiple testing correction: None; (3) Fold Difference: Twofold or greater and a P-value cutoff of 0.05.

*Analysis of human circulating tumor cells.* Patients eligible for the CTC biomarker protocols included (1) men with progressive CRPC, with metastatic progression by PSA (two consecutive rises over nadir separated by >1 week) or radiologic criteria (RECIST or new bone scan lesions), a PSA ≥5, age ≥18 years; or (2) women with mBC with disease progression or with initiation of a new systemic therapy, who were > 18 years of age, and who were at least 7 days from treatment with an anthracycline-containing regimen. Blood (15 mL) was collected from patients and processed within 48 hours at the Duke University CTC lab using the Cell Search System (Veridex, Raritan, NJ). Veridex profile kits were used, which isolate EpCAM positive cells without additional staining. The isolated cells were either processed immediately or stored overnight in 4% paraformaldehyde and processed the next day. Immunostaining was done on teflon coated slides. Briefly, cells were pipetted into the wells of the slides and left to settle for -30 minutes followed by standard immunostaining procedures with careful aspiration to minimize cell loss. An initial ferromagnetic wash using a benchtop magnet was performed to further isolate CTCs, with resuspension of the cell pellet after magnet release 100 uL PBS. Following 4% PFA fixation and permeabilization with PBT (PBS with 2% Triton) and blocking with 10% goat serum for 30 minutes, triple immunostaining was performed using CD45 antibody (AbCam #33533-50) labeled with Alexa 647, cytokeratin (AbD Serotec #MCA 1907HT) labeled with Alexa 555, and Vimentin (BD Biosciences, San Jose, CA #550513) labeled with Alexa 488. Nuclear staining with 4',6-diamidino-2phenylindole (DAPI) was then performed. A CTC was defined as an intact cell by microscopic examination, containing an intact nucleus and expressing cytokeratin but lacking CD45 staining, using appropriate controls (see Table 1 for antibodies and controls). Human peripheral blood mononuclear cells (PBMCs), obtained by Ficoll purification of buffy coats from normal donors, were kindly provided by Micah Luftig (Duke University, Durham NC) and used as control cells for CD45 expression. Linear regression analysis was performed to compare CTC count (standard Cellsearch method) against the proportion of CTCs that co-express vimentin. Goodness of fit was tested by analysis of variance.

**Table 1. EMT/Stemness Antigens to be assessed in CTCs.**

| **Antigen** | **Product** | **Positive Control** | **Negative Control** | **Leukocyte Expression** | **Dilution** |
|---|---|---|---|---|---|
| Vimentin | BD Biosciences, mouse monoclonal IgG1 | PBMCs, PC-3, DU145 | T47D, LnCAP | Yes | 2:225 |
| N-cadherin | DAKO, mouse monoclonal IgG1, 6G11 | Sarcoma, rat brain, PC-3 | DU145, T47D, mock | No | 4:225 |
| Cytokeratin (pan) | AbD Serotec, mouse monoclonal IgG1, MCAI907HT, clone AEI/AE3 | T47D, DU145 | PC-3, PBMCs | No | 2:45 |
| CD45 | Invitrogen, mouse IgG1, HI30, MHCD4500 | PBMC | PC-3, DU145 | Yes | 1:45 |
| CD133 | Santa Cruz mouse monoclonal IgG, sc-130127 | CaCo-2 colon cancer cells | Mock | Variable | 4:225 |

The slides were mounted with gel/mount media (Biomeda, Foster City, CA). The slides were analyzed with an Olympus (Melville, NY) IX 71 epifluorescence microscope, and images were acquired using an Olympus DP70 digital camera. Image processing was done with DP controller software (Olympus). All fields were analysed, with each cytokeratin positive nucleated cell that was CD45 negative being counted as a CTC. Positive control cells for each antibody included PC-3 cells for vimentin, peripheral blood mononuclear cells (PBMCs) for CD45, and T47D breast cancer cell lines for cytokeratin. A similar volume of reaction mix without antibody was used for negative controls.

Media exchange experiments. The cells of AT3-T or AT3-M clones were plated at a concentration of 150,000 cells/2 mL of media in 6-well plates and allowed to incubate for 24 h. The conditioned media was then filtered using a 0.22 µm filter, and then immediately allowed to incubate with cells of the other clone, which was plated at the same concentration and had its media aspirated and cells washed with 2 mL of PBS. All cells with media replaced were incubated for 72 h, and phase and epifluorescent microscopy was used to monitor cell phenotypes 24, 48, and 72 h after treatment. Control plates, in which media was conditioned, cells washed with PBS and media added back to the same cells, were also used.

*Scratch*-*wound assay.* Cells were plated and left to grow to nearly 100% confluency in 6-well dishes. A wound was simulated by scratching the cells with a sterile 200 IJI pipette tip. The wells were washed twice with PBS and fresh media added. Pictures were taken in the same marked spot at 0, 24, and 48 h. Percent migration was calculated as (width at 0 h - width at 24 or 48 h) 1 width at 0 h x 100. Relative migration was compared using two-way analysis of variance via Prism 4.0c for the Macintosh (Graphpad, La Jolla, CA).

*Matrigel assay.* Matrigel assay was performed per manufacturer's indications (BO Biosciences). Briefly, after rehydration, 2 x 10⁵ cells were plated either in the control or in the matrigel-coated inserts and incubated for 22 h. Following incubation, the non-invading cells from the upper-part of the inserts were removed using cotton-tipped swabs. The cells from the lower part of the membrane were stained with hematoxylin-eosin, membranes were removed, placed on a slide and observed under the microscope.

*Immunohistochemical (IHC) analysis of metastases*. Under the same informed consent protocol as the analysis of human circulating tumor cells described above, men undergoing CTC collection additionally consented to have a radiologic-guided metastatic biopsy for analysis of biomarker expression by IHC. Samples were obtained through core needle biopsies during light sedation, and immediately formalin-fixed and paraffin embedded. For analysis, slides were deparaffinized, rehydrated, and endogenous peroxidase was inactivated for 30 min. in 0.3% H₂O₂ (hydrogen-peroxide) in methanol. Specific antigen retrieval steps were performed for individual antigens. Three markers were evaluated by IHC: vimentin (M7020, Dako, 1:150; antigen retrieval with pepsin treatment at 37°C for 15 minutes), cytokeratin cocktail (18-0132, Invitrogen, 1:50 and 349205, BD Biosciences 1:50, antigen retrieval with pepsin treatment at 37°C for 15 minutes), and CD45 (M0701, Dako, 1:200; antigen retrieval with sodium citrate 10 mM, pH 6.0 at 100°C for 30 minutes). Primary antibody was incubated for 60 minutes at room temperature. Dako Envision horseradish peroxidase secondary antibody was used for 30 minutes at room temperature and the signal was detected with DAB reagent (Vector kit SK 4100). Slides were counter stained with hematoxylin and eosin and assessed by a trained pathologist for expression using appropriate positive (localized prostate tissue microarray sections) and negative controls (mock antibody) for each marker.

*Statistical analyses.* To determine the significantly differentially expressed genes between AT3-M and AT3-T the GeneSpring GX "Filtering on Volcano plot" feature was used with the following characteristics: (1) Test type: Parametric test, don't assume variances equal; (2) Multiple testing correction: None; (3) Fold Difference: Twofold or greater and a P-value cutoff of 0.05. To compare CTC count (standard Cellsearch® method) against the proportion of CTCs that co-express vimentin, N-cadherin, or CD133, linear regression analysis was performed. Goodness of fit was tested by analysis of variance.

### Example 2. Isolation of individual AT3 clones that inhabit an intermediate phenotypic state

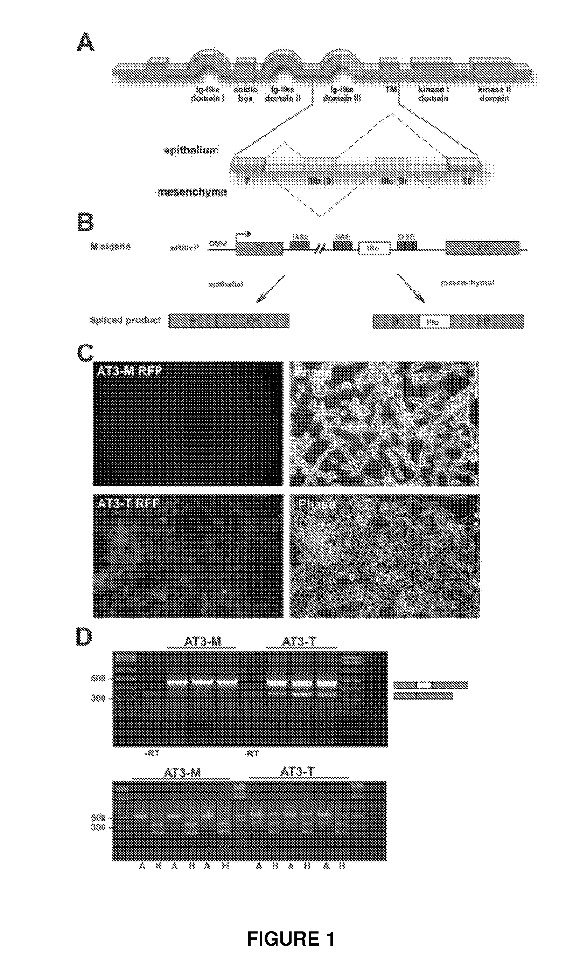

The alternative splicing of FGFR2 transcripts, which produces either FGFR2-IIIb or - IIIc variants in epithelial and mesenchymal cells respectively, is exquisitely regulated (Figure 1A). In Figure 1A is a schematic representation of the IIIb and IIIc alternatively spliced isoforms of FGFR2. FGFR2 contains an extracellular domain (with three IgG-like domains), a transmembrane domain (TM), and two intracellular tyrosine kinase domains. The IIIb isoform is found in epithelial cells while the IIIc isoform in mesenchymal cells. Exons IIIb and IIIc are regulated coordinately to provide mutually exclusive expression of the two isoforms and transcripts including both exons are destabilized by nonsense-mediated decay. We have previously used FGFR2 alternative splicing reporters, in particular constructs that measure the epithelial-specific silencing of exon IIIc (e.g., pRIIIcl² in Figure 1B), to report on the phenotypic state of cells *in vitro* and *in vivo.* In Figure 1B is a schematic of the pRIIIcl² minigene and the fluorescence read-out. The minigene contains the DsRED open reading frame interrupted by exon IIIc and flanking introns of the FGFR2 gene. In epithelial cells exon IIIc is skipped, DsRED open reading frame is formed and results in fluorescence signal. In mesenchymal cells, exon IIIc is included and the DsRED open reading frame is disrupted, resulting in low or close-to-background fluorescence signal. The pRIIIcl² splicing reporter, which produces a variant red fluorescence protein (DsRED) when exon IIIc is silenced, revealed MET in primary tumors derived from AT3 cells implanted in the flanks of Copenhagen white rats. While most tumors contained MET foci, each tumor had very few foci and these were not randomly distributed but rather were associated with collagenous stroma. In contrast to the low frequency of MET in primary tumors, a high incidence of MET among lung metastases in these animals was observed, suggesting an unexpected association between the more epithelial phenotype and aggressive behavior. These studies could not ascertain whether the epithelial-like AT3 cells found in the lungs had undergone MET in the primary tumors or during the process of metastasis.

In an attempt to find post-MET cells *in vitro*, limiting dilution was used to obtain clones from AT3 cells stably transfected with the pRIIIcl² reporter. A total of 16 clones of a maximum calculated recovery of 27 were obtained, which is ∼ 60% cloning efficiency. Eleven of these sixteen clones expressed RIIIcl² transcripts (italicized in Table 2), and of these, eight expressed DsRED (Table 2). Some of the clones had an epithelial-like morphology (cells with cobblestone appearance and adherent to each other), while others had a mesenchymal-like morphology (spindle-shaped), as well as clones that displayed a mixed phenotype. It is important to note that given the high cloning efficiency and the high frequency of DsRED+ clones, it is highly unlikely for these epithelial-like clones to come from a very small population within the parental AT3 cells. Rather, the process of subcloning induced a phenotypic transition in a significant number of the AT3 cells.

**Table 2. Properties of AT3 clones.**

| **AT3 Clones** | **Cellular morphology³** | **DsRED expression²** | **Detection of exon IIIc skipping among RIIIcl3 transcripts¹** | **FGFR2 transcipts detected³** |
|---|---|---|---|---|
| *1* | *Epithelial* | *High* | + | *IIIc* |
| 2 | *Epithelial* | *High* | + | *IIIc > IIIb* |
| *3* | Epithelial | Low | ND | IIIc > *IIIb* |
| *4* | Epithelial | Low | ND | IIIc |
| 5 | *Epithelial* | *High* | + | *IIIc > IIIb* |
| 6 | Mesenchymal | Low | ND | IIIc |
| 7 | Mixed | Low | ND | IIIc |
| *8* | *Mixed* | *High* | + | *IIIc* |
| 9 | Mixed | Low | ND | IIIc |
| *10* | *Mixed* | *High* | + | *IIIc* |
| *11* | *Mesenchymal* | *Low* | - | *IIIc* |
| *12* | Mesenchymal | *Low* | - | *IIIc* |
| *13* | *Epithelial* | *High* | *-1* | *IIIc > IIIb* |
| 14 | *Epithelial* | *Low* | - | *IIIc* |
| *15* | *Epithelial* | *High* | + | *IIIc* |
| *16* | *Mixed* | *High* | -2 | *IIIc* |

| | | | | |
|---|---|---|---|---|
| ¹See Figure 1C. A "+" indicates detection of RIIIcl² transcripts missing exon IIIc, a "-" all RIIIcI² transcripts include exon IIIc, ND means that no RIIIcl² transcripts were detected. ²Determined by epifluorescence microscopy (high is defined as fluorescence above background of naive AT3 cells and low undistinguishable from the same cells). ³Discussed further herein and illustrated in Figure 1C. | | | | |

All of the clones obtained by limiting dilution were analyzed to determine the splicing status of RIIIcl² and endogenous FGFR2 transcripts. We could not detect exon IIIc skipping among pRIIIcl² transcripts or any evidence of exon *IIIb* inclusion among endogenous FGFR2 transcripts in clones with a mesenchymal-like morphology (Figure 1C and Table 2). Figure 1C shows RT-PCR analysis of the reporter (upper panel) and endogenous FGFR2 (lower panel). Primers used for the reporter are designed in the DsRED regions flanking exon IIIc. RT-PCR shows a higher percentage of the skipped product in clone AT3-T compared to clone AT3M. Reactions that did not include RT (-RT) reveal a contaminating product that is out-competed by the presence of a bona fide cDNA template (AT3-M lanes). Since exons IIIb and IIIc differ in size by only 3 nucleotides, analysis of the presence of IIIb or IIIc exons in FGFR2 gene was done by using primers in the flanking exons and specific restriction digestion of the resulting RT-PCR products. Exon IIIb is digested by Aval (A) and IIIc by HincII (H). There is a higher percentage of exon IIIb in clone AT3-T. The RT-PCR are replicates from three different cultures of the two clones. These clones did not express detectable levels of DsRED (Figure 1D and Table 2). Figure 1D shows epifluorescence and phase-contrast pictures of clones AT3-M and AT3-T shows the difference in fluorescence intensity and morphology between the two clones. Epifluorescence pictures were taken at the same exposure. All pictures were acquired at 200X magnification. While the skipping of exon IIIc among pRIIIcl² transcripts from epithelial-like clones could be expected, the observation that all of these clones both skipped and included exon IIIc was unexpected (Figure 1 C, Table 2 and data not shown). Analysis of endogenous FGFR2 transcripts revealed that four of the clones with epithelial morphology and DsRED expression had clear evidence of coexpression of both IIIb and IIIc isoforms (Table 2, and Figures 1C and 1D). As shown in Figure 1, AT3-T cells expressed epithelial and mesenchymal isoforms of FGFR2. The expression of DsRED in all the cells suggested that each cell in the culture was expressing both isoforms (Figure 1C).

We followed two clones with epithelial morphology, high DsRED levels and coexpression of FGFR2-IIIb and -IIIc transcripts (clone 2 and clone 5 (clone 5 herein AT3-T)) and noted that the phenotypic characteristics described above were stable for over six months. Equally, we followed clone 11 (clone 11 herein AT3-M) and clone 12 for six months, and noted that the mesenchymal morphology, undetectable DsRED expression and exclusive production of FGFR2-IIIc were also stable. We concluded from these observations that AT3 cells were plastic and were coaxed by sub-cloning to populate intermediate phenotypic states, with properties of epithelial and mesenchymal cells.

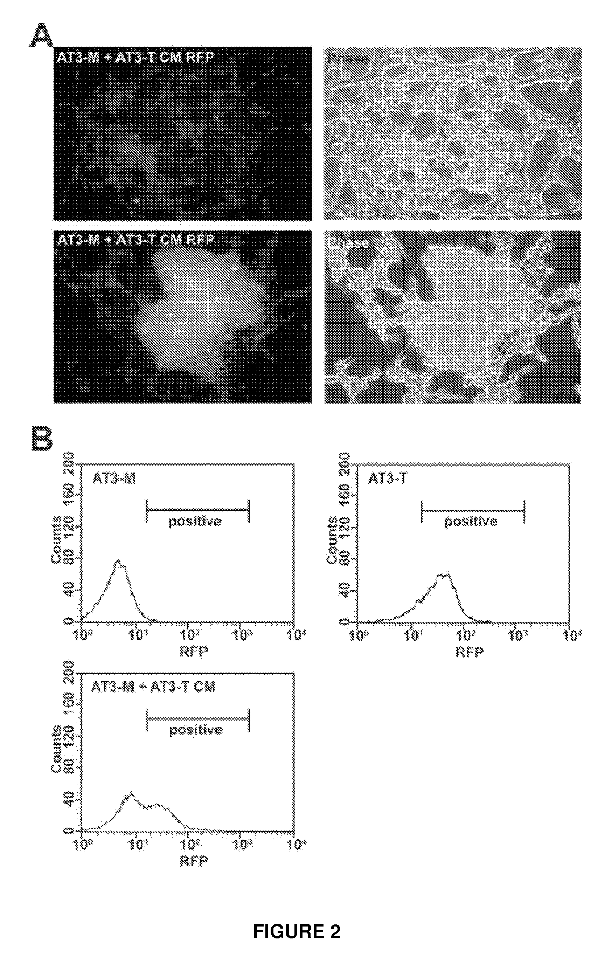

A media exchange experiment was used to investigate whether or not the splicing of RIIIcI² transcripts in the DsRED expressing clones was regulated by soluble factors.. Media conditioned by DsRED expressing clones (clone 5 in Table 2) was filtered and added to DsRED negative clones (clone 11 in Table 2). DsRED+ cells were observed among DsRED-cells incubated with DsRED+ conditioned media (Figure 2). Figure 2A shows examples of clusters of DsRED positive cells formed by AT3-M cells upon treatment with conditioned media from clone AT3-T. Media was conditioned for 24 h, filtered and added on AT3-M cells. Pictures (acquired at 200X) are taken 48 h following media exchange. Figure 2B shows results from flow cytometry analysis of the same experiment. Left upper panel represents clone AT3-M conditioned with media from the same clone, as a negative control. Right upper panel represents clone AT3-T, which is DsRED positive. The lower panel represents clone AT3-M 48 h after conditioned media from clone AT3-T was added. Different lots of fetal bovine serum caused variation in this effect. This effect was quantified by flow cytometry and these data suggested that about half of the DsRED- cells were induced to express DsRED at levels equivalent to those seen in DsRED+ cells (Figure 2). The changes observed were not due to prolonged culture of the cells in the same wells because conditioned media from a separate DsRED- culture did not induce DsRED expression. As shown in Figure 2, AT3-T conditioned media induced AT3-M cells to express DsRED. These observations suggest that soluble factors secreted by the DsRED+ clones or dilution of factors extant in the DsRED- conditioned media may contribute to plasticity.

### Example 3. AT3-M and AT3-T cells are tumorogenic

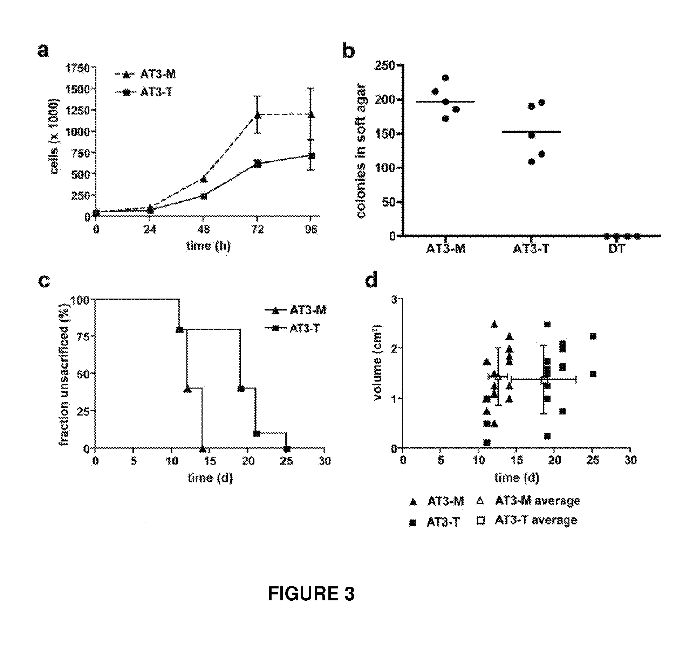

The initial characterization of the AT3-T revealed that these transitional cells grew slower and reached a lower confluent density than the AT3-M (Figure 3A). Figure 3A shows growth curves for clones AT3-T and AT3-M. Cells were plated at 0 h time-point, trypsinized, and counted at the indicated times. Data are the mean ± S.D. (n = 3). To investigate their growth *in vivo* AT3-M and AT3-T cells were co-transfected with pGint a plasmid that expresses EGFP (herein GFP) in both mesenchymal and epithelial cells, and sorted stable populations of each cell line using flow cytometry for uniform GFP intensity. The GFP expressing cells maintained the morphological characteristics, the differential DsRED expression, and the differences in the splicing of pRIIIcl² and FGFR2 transcripts first observed after sub-cloning.

We injected 3 x 10⁵ GFP-expressing AT3-T or AT3-M cells subcutis in both flanks of Copenhagen white 2331 male rats. All of the animals developed bilateral tumors, indicating that both AT3-M and AT3-T cells were highly tumorogenic in these syngeneic rats. As a humane endpoint, rats were sacrificed when tumor length estimated by palpation reached 1 cm. The *in vivo* growth curves for the AT3-M and AT3-T tumors were significantly different, as determined by a logrank test (p = 0.0020; Figure 3B). Figure 3B is a sacrifice curve for rats injected with AT3-M or AT3-T cells. Figure 3C shows comparison of tumor volumes resulting from AT3-T and AT3-M injection. The Y-axis represents tumor volumes at the time of sacrifice of the animals and the X-axis days from the time of implantation to the time of sacrifice. Average tumor volumes and average days until sacrifice are represented with S.D. bars. Some points represent more than one tumor with the same volume on the same day. Tumor volume was measured (Figure 3C) and although most AT3-T animals were sacrificed later, there was no significant difference in tumor size (p = 0.76). As shown in Figure 3, AT3-T cells grew more slowly than the mesenchymal-like AT3-M cells *in vitro* and *in vivo,* but both were equally tumorogenic. We concluded that whereas AT3-T cells grew more slowly *in vitro* and *in vivo* relative to their more mesenchymal siblings, these transitional cells were capable of forming tumors.

### Example 4. Both AT3-M and AT3-T are plastic

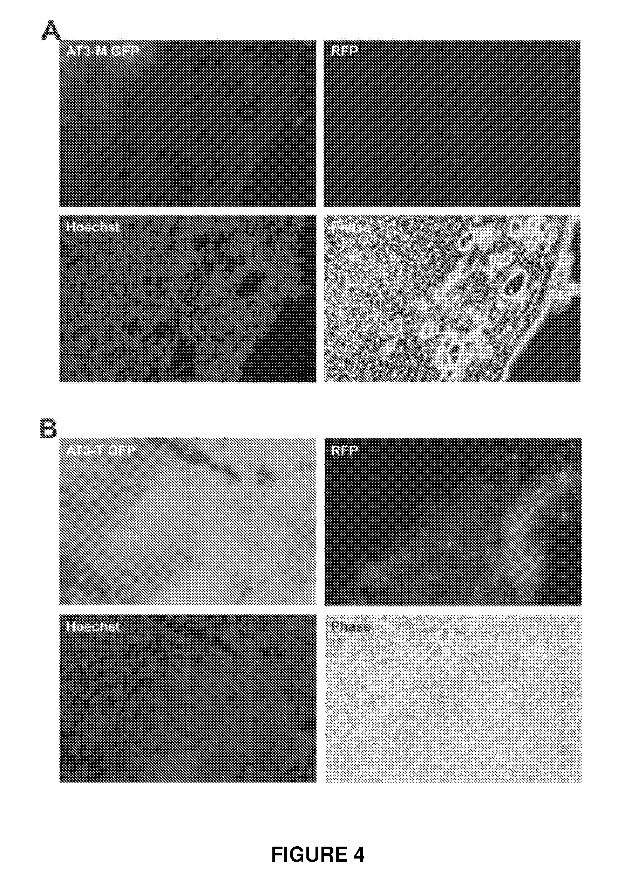

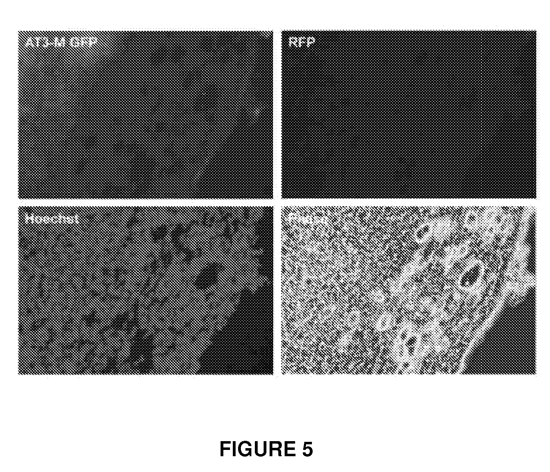

Since the implanted AT3-M and AT3-T cells could be tracked by GFP expression, and epithelial character could be interrogated by DsRED expression, the plasticity of the tumors were able to investigated. The overwhelming majority of cells in AT3-M tumors expressed GFP but not DsRED (Figure 4A). As shown in Figure 4, tumors from both AT3-T and AT3-M clones have evidence of plasticity. Figure 4A shows representative example of cells that express both RFP and GFP at the periphery of an AT3-M tumor stably transfected with Gint and pRIIIcl² reporters. Pictures were taken at 200X magnification. To compensate for a low RFP signal, the color curve of the entire picture was adjusted. Nonetheless, groups of cells were observed expressing both GFP and DsRED in many AT3-M tumor sections, especially near the tumor capsule, (Figure 4A; see also Figure 5). Figure 5 shows a representative example of cells that express both RFP and GFP at the periphery of an AT3-M tumor stably transfected with Gint and pRIIIcI² reporters. Pictures were taken at 200X magnification. In this version, overall RFP signal was not adjusted via color curve after the image was captured. RFP positive cells were clearly above background level.

Many sections from AT3-T tumors co-expressed GFP and DsRED; however, large areas were observed that expressed GFP but not DsRED in all 64 sections surveyed (Figure 4B). Figure 4B shows representative example of a section from an AT3-T tumor stably transfected with GFP and pRIIIcl² reporters. Pictures were taken at 200X magnification. RNA extracted from these regions of AT3-T tumors confirmed the presence of the pRIIIcl² transcripts. Both AT3-T and AT3-M cells were plastic and produced tumors with cells that displayed a range of epithelial-mesenchymal properties.

### Example 5. AT3-T cells are motile in vitro and metastatic in vivo

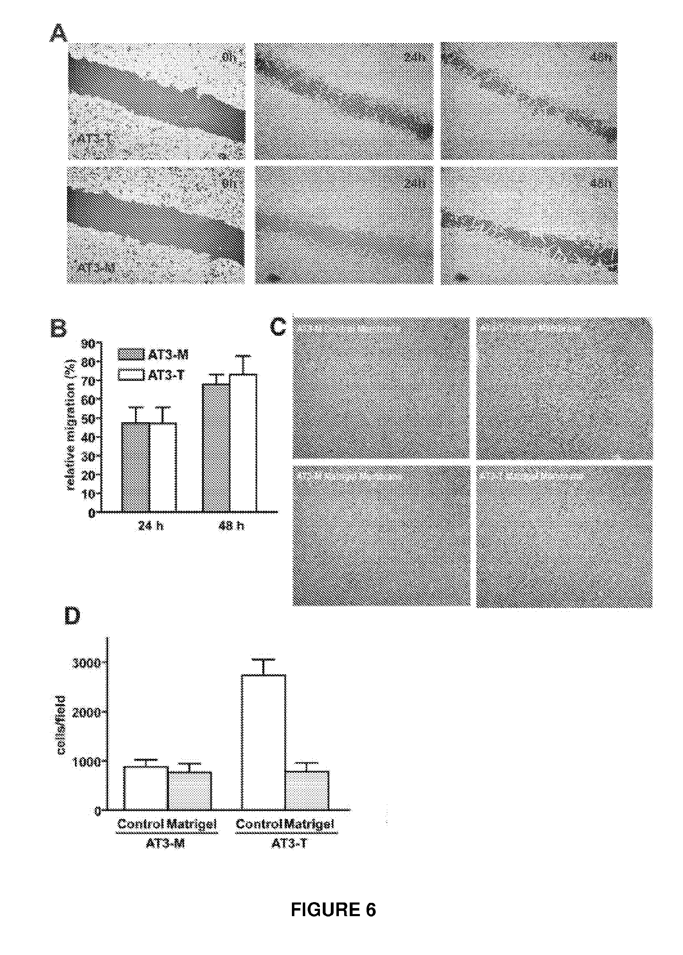

Comparison of AT3-T and AT3-M mobility and invasive potential was performed in culture. Motility was measured in culture by a "wound closure" assay, and no significant motility difference (p = 0.59) was found between cell lines 24 and 48 hours after a scratch-wound had been made in the cultures (Figure 6). Figure 6A shows representative pictures for the scratch-wound assay (experiment done in triplicate for each clone). Pictures were taken at 40X magnification. Figure 6B shows quantification of migration as explained in Methods. Mean and SO values were derived from triplicate experiments. Figure 6C shows invasion assay using Matrigel coated membranes. Representative pictures of each clone and for both control membranes and Matrigel-coated membranes (n = 5). Cells were stained with hematoxylin-eosin. Pictures were taken at 40X magnification. Figure 6D shows quantification of invasion assay results. Mean and SD values were derived from five individual experiments. To gauge invasive properties of the cells we measured the number of cells traversing through Matrigel membranes in a 22-hour period. The same number of AT3-T and AT3M cells was observed on the Matrigel membranes suggesting that the two cell lines were equally capable of invading this membrane (Figure 6). While a higher number of cells from clone AT3-T were observed on the control membrane compared to clone AT3-M, these studies nevertheless indicated that the more epithelial AT3-T cells had similar motility and invasive potential as the AT3-M cells. As shown in Figure 6, AT3-M and AT3-T cells exhibited similar migration *in vitro.*

In order to assess *invasiveness in vivo* lungs from the twenty animals harboring AT3-M and AT3-T tumors were examined for presence of metastatic foci. No macroscopic metastatic nodules were observed in any of the lungs, which was likely due to the sacrificing protocol used on the animals when the tumors reached a specified size instead of using survival as the end-point. The GFP expression from the Gint reporter was examined to evaluate the presence of micrometastases by epifluorescence microscopy. To assure a comprehensive evaluation, 7-8 equally spaced sections from each lung were surveyed (total of 150 sections for each clone). The presence of metastatic foci was determined by GFP fluorescence, followed by counter-staining of the sections with hematoxylineosin (Figure 7). Figure 7A shows (upper panel) an example of a section exhibiting the expected pattern for clone AT3-T (i.e. GFP+, DsRED+) in a metastatic focus, and (lower panel) an example of a section exhibiting a plastic pattern for clone AT3-T (i.e. GFP+, DsRED-) in a metastatic focus. Figure 7B shows (upper panel) an example of a section exhibiting the expected pattern for clone AT3-M (i.e. GFP+, DsRED-) in a metastatic focus, and (lower panel) an example of a section exhibiting a plastic pattern for clone AT3-M (i.e. GFP+, DsRED+) in a metastatic focus. As shown in Figure 7, metastatic foci in lungs from animals with tumors from either AT3Tor AT3-M clones (stably transfected with GFP and pRIIIcI² reporters) had evidence of plasticity. Metastatic foci were found in 7 out of 10 lungs for clone AT3-M and 6 out of 10 lungs for clone AT3-T.

Evaluation of the plasticity of the metastatic foci using the combined output of the GFP and DsRED reporters revealed plastic foci (DsRED+ for AT3-M and DsRED- for AT3-T) in the case of both clones: 3 out of 12 for clone AT3-T and 13 out 16 for clone AT3-M (Figure 7). These studies indicated phenotypic plasticity for the AT3-M cells and suggested it for the AT3-T cells. Importantly, both cell lines were metastatic despite differences in the original epithelial vs. mesenchymal phenotype.

*Plasticity and metastatic behavior of cancer cells.* Both the mesenchymal AT3-M and the more epithelial AT3-T cells metastasized efficiently. The drivers of metastasis, however, may be different in these two cells. The gene expression comparison between the AT3-M and AT3-T clones revealed at least one intriguing possibility: microarray analysis showed a 12-fold increase in the expression of junctional adhesion molecule C (JAM-C) in AT3-T compared to AT3-M, and this was confirmed by RT-PCR and immunoblot analysis. JAMs were present in leukocytes and at the tight junctions of epithelial and endothelial cells and have been shown to be involved in transendothelial migration of monocytes. JAM-C is expressed in several cell lines with high metastatic potential and knock-down of this molecule in the HT1080 human fibrosarcoma line significantly decreases its metastatic properties *in vivo.* Moreover, JAM-C is also present in the gene sets associated with stemness that had significant overlaps with genes that define clone AT3-T. Therefore clone AT3-T, by over-expression of different adhesion molecules may acquire metastatic capabilities. In addition, the overexpression of the downstream Hedgehog pathway effector GLI3 may be significantly upregulated in the more epithelial and stem cell-like AT3-T cells as compared to the more mesenchymal AT3-M cells. Hedgehog signaling has been linked to EMT, stemness, and metastasis/aggressiveness in several tumor types, and thus differential expression or regulation of developmental programs may underly these phenotypical differences across these cell lines. Increased expression of Patched, a Hedghog pathway component, has been linked to prostate tumors during progression to androgen independence and in circulating tumor cells of men with metastatic castration-resistant prostate cancer.

### Example 6. AT3-T cells display a stem cell-like gene expression signature

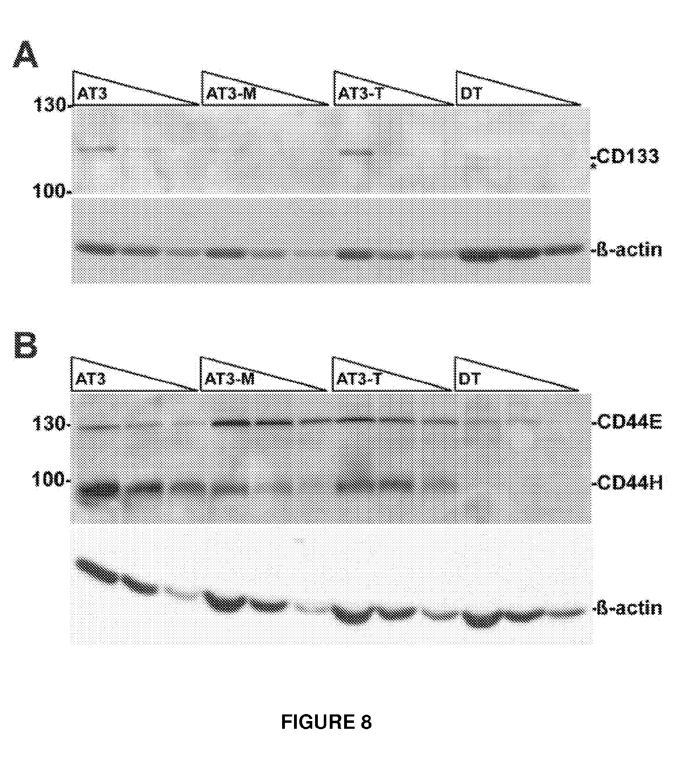

AT3-T cells sometimes formed tight clusters resembling protospheres. While sphere formation is not an exclusive property of stem cells, it has been associated with stemness in many different systems. Given these observations and the high tumorogenicity of AT3-T and AT3-M cells, they were tested for the expression of markers associated with cancer stem-like cells. Also included were the parental AT3 cells and another Dunning tumor cell line, DT cells, which display epithelial markers and are only weakly tumorogenic in Copenhagen white rats. The DT cells expressed very low levels of CD44 and CD133, which are associated with highly malignant cancer stem-like cells (Figure 8). CD133 was detectable in DT lysates only when four fold more lysate was loaded. The mesenchymal-like AT3 cells expressed much higher levels of both CD44 and CD133 than the DT cells (note that the lanes for the DT samples are overloaded in Figure 8A), which is consistent with recent reports that EMT induces stemness in mammary epithelial carcinoma cells. Figure 8A shows a membrane with serial twofold dilutions of whole cell lysates was cut in half and immunoblotted for CD133 (upper panel) or β-actin (lower panel). Size markers are in kDa. A faster migrating CD 133 band repeatably detected only in DT lysates is marked (*), suggesting possible post-translational regulation. Figure 8B shows a membrane with serial twofold dilutions of whole cell lysates was cut in half and immunoblotted for CD44 (upper panel) or β-actin (lower panel). Representative blots from two independent sets of lysates are shown. AT3-T expressed CD44 and CD133. Interestingly, the AT3-T cells expressed overall higher levels of CD44 and CD133 than the more mesenchymal AT3-M. Moreover, AT3-T cells expressed a higher ratio of CD44H to CD44E when compared to AT3-M cells. The CD44H isoform has been associated with malignancy while CD44E is not. This suggests a more complex relationship between epithelial transitions and acquisition of stem cell-like properties. Consistent with expression of stem-like markers, both AT3-M and AT3-T cells formed colonies in soft agar and tumors when injected into Copenhagen white rats, and these tumors led to extensive metastases similar to parental AT3 cells (Figure 3B).

To further explore these connections between transitions and stemness, global gene expression in AT3-M and AT3-T cells was compared. This analysis showed that 422 genes were differentially expressed (≥ 2-fold; p-value < 0.05) in these two cells (Table 3). Many of the genes that were upregulated in AT3-T relative to AT3-M were preferentially expressed in epithelial cells and vice versa for those preferentially expressed in mesenchymal cells (Table 4). There were exceptions to this, however. Expression of the gene disintegrin-like and metalloprotease was consistent with a mesenchymal phenotype, but this mRNA level was 4-fold higher in AT3-T compared to AT3-M. Integrin β-4, normally associated with epithelial-like cells, was expressed 3-fold lower in AT3-T compared to AT3-M. These observations were consistent with the characterization of AT3-T cells as displaying more epithelial features than AT3-M cells and as populating an intermediate phenotypic state.

**Table 3.**

| **x Fold change (AT3-T/AT3-M)** | **Gene Symbol (Human)** | **Gene Symbol (Rat)** |
|---|---|---|
| 0.00771 | P2RX5 | P2rx5 |
| 0.011 | CCNB11P1 | #N/A |
| 0.0296 | STRA6 | Stra6 |
| 0.0327 | G0S2 | G0s2 |
| 0.0835 | SERPINF1 | Serpinf1 |
| 0.101 | GSTA1 | #N/A |
| 0.107 | RSNL2 | Clip4 |
| 0.115 | ADAMTS7 | #N/A |
| 0.134 | GZMB | #N/A |
| 0.137 | SPON2 | #N/A |
| 0.156 | MMP3 | #N/A |
| 0.191 | ATP8A1 | #N/A |
| 0.197 | EVPL | Evpl |
| 0.21 | LGALS3BP | Lgals3bp |
| 0.216 | SERPINB2 | Serpinb2 |
| 0.219 | NETO2 | Neto2 |
| 0.223 | PTX3 | #N/A |
| 0.23 | SERPINB7 | Serpinb7 |
| 0.233 | RASIP1 | #N/A |
| 0.235 | OMD | #N/A |
| 0.239 | HLA-G | #N/A |
| 0.239 | HLA-A | #N/A |
| 0.247 | CD97 | Cd97 |
| 0.251 | GJA4 | Gja4 |
| 0.254 | DSU | #N/A |
| 0.257 | MGLL | Mgll |
| 0.261 | SPHK1 | #N/A |
| 0.268 | HRBL | Zcwpw1 |
| 0.268 | ZCWPW1 | Zcwpw1 |
| 0.27 | ENPP3 | Enpp3 |
| 0.275 | PTGS1 | Ptgs1 |
| 0.278 | RAMP1 | Ramp1 |
| 0.281 | DHRS3 | Dhrs3 |
| 0.282 | FAM117A | Fam117a |
| 0.284 | TUBB2A///TUBB2B | Tubb2b |
| 0.284 | TUBB2B | Tubb2b |
| 0.285 | C10orf10 | LOC500300 |
| 0.289 | SYTL2 | #N/A |
| 0.291 | SLC39A4 | Slc39a4 |
| 0.292 | CHRD | Chrd |
| 0.292 | GIP | Gip |
| 0.293 | CKLF | Cklf |
| 0.294 | PLAU | Plau |
| 0.295 | GUF1 | #N/A |
| 0.307 | CGI-38 | Tppp3 |
| 0.311 | LECT2 | Lect2 |
| 0.318 | NQO2 | #N/A |
| 0.32 | C11orf75 | RGD1309410 |
| 0.324 | DOCK2 | #N/A |
| 0.325 | LGALS2 | #N/A |
| 0.326 | CASP4 | Casp1 |
| 0.326 | LTBP4 | Ltbp4 |
| 0.334 | HSPB1 | Hspb1 |
| 0.335 | ITGB4 | Itgb4 |
| 0.34 | BPHL | Bphl |
| 0.341 | FOXF2 | #N/A |
| 0.345 | MYH1 | #N/A |
| 0.345 | SMAD6 | Smad6 |
| 0.348 | TGFB1 | Tgfb1 |
| 0.351 | MMP10 | #N/A |
| 0.363 | MMP9 | Mmp9 |
| 0.363 | COL18A1 | Coll8al |
| 0.366 | HES1 | #N/A |
| 0.369 | SLC35D2 | #N/A |
| 0.377 | ADORA2B | Adora2b |
| 0.377 | COL3A1 | Col3a1 |
| 0.379 | DPEP2 | Dpep2 |
| 0.382 | GPR153 | Gpr153 predicted |
| 0.383 | LOC55908 | #N/A |
| 0.389 | SELPLG | #N/A |
| 0.394 | P2RX1 | Atp2a3 |
| 0.394 | ATP2A3 | Atp2a3 |
| 0.394 | ADD3 | Add3 |
| 0.395 | TSPAN9 | Tspan9 |
| 0.399 | LOC54103 | #N/A |
| 0.4 | BFSP2 | #N/A |
| 0.4 | FLJ14213 | RGD1309969 |
| 0.4 | PGGT1B | Pggt1b |
| 0.401 | HCN2 | Hcn2 |
| 0.403 | C2orf33 | RGD1310230 |
| 0.404 | TMEPAI | #N/A |
| 0.405 | INHA | Inha |
| 0.406 | HPSE | #N/A |
| 0.409 | CRY1 | Cry1 |
| 0.413 | IL3RA | 113ra |
| 0.413 | CDC42EP1 | #N/A |
| 0.416 | ARG1 | Arg1 |
| 0.417 | MAPK14 | Mapk14 |
| 0.419 | FLJ22028 | #N/A |
| 0.421 | GALR2 | Galr2 |
| 0.422 | TSPAN8 | Tspan8 |
| 0.422 | FAM77C | RGD1561205 |
| 0.422 | USP2 | Usp2 |
| 0.422 | LAMA3 | #N/A |
| 0.424 | CCNE1 | Ccne1 |
| 0.424 | NSF | Nsf |
| 0.428 | ST3GAL5 | St3gal5 |
| 0.429 | SYNJ2 | Synj2 |
| 0.43 | ADA | Ada |
| 0.43 | PCBP3 | Pcbp3 |
| 0.433 | ZNF43 | #N/A |
| 0.433 | C14orf130 | Ubr7 |
| 0.436 | SOS2 | #N/A |
| 0.436 | RASSF3 | #N/A |
| 0.436 | GLMN | Glmn |
| 0.438 | OSR2 | Osr2 |
| 0.44 | AGTPBP1 | Agtpbp1 |
| 0.444 | DBNDD2 | RGD1311642 |
| 0.445 | SGCB | #N/A |
| 0.446 | HBLD2 | Isca1 |
| 0.448 | SCARB1 | Scarb1 |
| 0.448 | EVI2A | Evi2a |
| 0.448 | AP4M1 | #N/A |
| 0.451 | IGF2BP3 | #N/A |
| 0.452 | FLJ10404 | Ddx41 |
| 0.454 | TGFB2 | Tgfb2 |
| 0.459 | PASK | Pask |
| 0.461 | C19orf37 | Zfp428 |
| 0.462 | BMP1 | Bmp1 |
| 0.464 | PTPN13 | Ptpn13 |
| 0.47 | PTPRG | #N/A |
| 0.47 | EFNB1 | Efnb1 |
| 0.472 | PER2 | Per2 |
| 0.472 | IRS3L /// LOC442715 | Irs3 |
| 0.472 | HRBL | Irs3 |
| 0.472 | MAP3K3 | Kcnh6 |
| 0.472 | WDR68 | Kcnh6 |
| 0.472 | KCNH6 | Kcnh6 |
| 0.472 | CCDC44 | Kcnh6 |
| 0.473 | CIB2 | Cib2 |
| 0.475 | MPZL1 | Mpzl1 |
| 0.475 | FADS2 | #N/A |
| 0.48 | ZNF185 | #N/A |
| 0.482 | SLC29A1 | Slc29a1 |
| 0.487 | RUNX3 | Runx3 |
| 0.488 | NINJ1 | Ninj1 |
| 0.489 | RASL11B | Ras111b |
| 0.49 | ECE2 | Ece2 |
| 0.49 | TNNC2 | Tnnc2 |
| 0.491 | WASPIP | Wipf1 |
| 0.492 | FN1 | Fn1 |
| 0.494 | NDE1 | Nde1 |
| 0.494 | CAMK2G | Camk2g |
| 0.495 | CUTL1 | Cux1 |
| 0.495 | ABHD6 | Abhd6 |
| 0.495 | PTPN14 | Ptpn14 |
| 0.497 | FLJ13946 | #N/A |
| 0.498 | BAIAP2 | Baiap2 |
| 0.499 | MSL3L1 | Msl311 |
| 0.499 | DYNLT1 | Dynlt1 |
| 0.499 | GSTM3 | Gstm5 |
| | | |
| 2 | CHES1 | Foxn3 |
| 2.004 | AQR | Agr /// Znf770 |
| 2.006 | EPN1 | Epn1 |
| 2.011 | PPBP | Ppbp |
| 2.019 | SLC35D1 | #N/A |
| 2.022 | PTPRC | #N/A |
| 2.031 | USP47 | Usp47 |
| 2.041 | DHX29 | #N/A |
| 2.047 | HMOX1 | #N/A |
| 2.05 | CAV1 | Cav1 |
| 2.053 | BUB1B | Bub1b |
| 2.069 | KCNIP4 | #N/A |
| 2.072 | -- | #N/A |
| 2.072 | ADAM10 | #N/A |
| 2.073 | KIAA115 | #N/A |
| 2.074 | PSTPIP2 | #N/A |
| 2.083 | MAML1 | #N/A |
| 2.084 | RAB32 | #N/A |
| 2.089 | FAM111A | #N/A |
| 2.095 | ATRNL1 | #N/A |
| 2.101 | PPIC | Ppic |
| 2.101 | CHD4 | Chd4 |
| 2.109 | IDE | Ide |
| 2.117 | PITPNM3 | #N/A |
| 2.121 | NFE2L1 | Nfe211 |
| 2.121 | MFSD1 | #N/A |
| 2.133 | KITLG | Kitlg |
| 2.161 | ING3 | Ing3 |
| 2.167 | CD24 | #N/A |
| 2.169 | IDS | #N/A |
| 2.177 | MGC3196 | LOC686289 /// LOC690285 |
| 2.185 | FBXL11 | Fbx111 |
| 2.185 | -- | Fbx111 |
| 2.191 | ZC3H12A | #N/A |
| 2.195 | RKHD2 | #N/A |
| 2.201 | LAMC2 | Lamc2 |
| 2.217 | KIF11 | Kif11 |
| 2.242 | SNAPC5 | Snapc5 |
| 2.252 | THRAP3 | #N/A |
| 2.261 | HS6ST1 | #N/A |
| 2.264 | OXCT1 | #N/A |
| 2.266 | TEK | #N/A |
| | HIST2H4///H4/o/// LOC648164 | |
| 2.268 | | #N/A |
| 2.271 | TMF1 | Tmf1 |
| 2.273 | ZBTB7B | Zbtb7b |
| 2.274 | CAMSAP1L1 | RGD1310950 |
| 2.279 | CYP3A5 | Cyp3ai |
| 2.279 | CYP3A7 | Cyp3a9 |
| 2.279 | CYP3A4 | Cyp3a9 |
| 2.282 | PENK | Penk1 |
| 2.283 | KIAA2010 | Smek1 |
| 2.284 | CHRNA1 | #N/A |
| 2.299 | BAT3 | Bat3 |
| 2.302 | ROM1 | Rom1 |
| 2.306 | HOXB8 | #N/A |
| 2.309 | KLK14 | #N/A |
| 2.31 | SUV39H1 | #N/A |
| | LOC440354///BOLA2/// LOC595101 | |
| 2.315 | | RGD1564579 |
| 2.315 | UBN1 | Ubn1 |
| 2.323 | C1orf103 | #N/A |
| 2.333 | EYA2 | Eya2 |
| 2.347 | MT2A | #N/A |
| 2.353 | KIAA1815 | Ermp1 |
| 2.355 | SETD1B | #N/A |
| 2.369 | MPHOSPH1 | Kif20b |
| 2.38 | EFNA1 | Efna1 |
| 2.392 | ABCF2 | Abcf2 |
| 2.397 | LIMA1 | Lima1 |
| 2.418 | EXTL3 | Extl3 |
| 2.418 | ARL6IP2 | Arl6ip2 |
| 2.442 | GRAMD3 | Gramd3 |
| 2.456 | JARID1A | Jarid1a |
| 2.476 | ARHGEF9 | Arhgef9 |
| 2.485 | CAD | Cad |
| 2.493 | RAI17 | #N/A |
| 2.526 | KIAA0284 | #N/A |
| 2.529 | SGPP1 | Sgpp1 |
| 2.531 | ABCB1 | #N/A |
| 2.531 | ABCB1///ABCB4 | #N/A |
| 2.542 | KIF1C | #N/A |
| 2.553 | KIAA0020 | LOC499339 |
| 2.563 | ADAM 15 | Adam15 |
| 2.577 | UBE1 | Uba1 |
| 2.577 | INE1 | Uba1 |
| 2.58 | GRIP2 | Grip2 |
| 2.59 | PPEF1 | #N/A |
| 2.619 | SC65 | Sc65 |
| 2.62 | FER1L3 | #N/A |
| 2.62 | NOC3L | #N/A |
| 2.62 | RBP4 | #N/A |
| 2.645 | SPINK4 | Spink4 |
| 2.653 | ATXN2L | #N/A |
| 2.711 | AHCYL1 | Ahcy11 |
| 2.723 | TUBB3 | Tubb3 |
| 2.723 | MC1R | Tubb3 |
| 2.729 | AGPAT7 | Lpcat4 |
| 2.749 | HOXC11 | #N/A |
| 2.766 | APH1A | Aph1a |
| 2.785 | CNOT1 | RGD1308009 |
| 2.785 | CSNK2A2 | RGD1308009 |
| 2.794 | STAC | #N/A |
| 2.904 | STAG1 | #N/A |
| 2.942 | MBNL1 | #N/A |
| 2.982 | MNT | Mnt |
| 3.007 | RANBP5 | Ipo5 |
| 3.014 | HERC1 | Herc1 |
| 3.065 | ALDOC | Aldoc |
| 3.122 | KIAA0460 | - |
| 3.174 | FLT3 | #N/A |
| 3.278 | CXCL6 | Cxcl6 |
| 3.366 | GLI3 | #N/A |
| 3.489 | SSR3 | #N/A |
| 3.585 | BCAN | Bcan |
| 3.824 | FKBP10 | Fkbp10 |
| 3.903 | GSTK1 | Gstk1 |
| 3.931 | PSCDBP | #N/A |
| 3.974 | ALCAM | Alcam |
| 4.056 | ADAMTS13 | |
| 4.203 | SPRR2B | #N/A |
| 4.276 | GPR126 | #N/A |
| 5.169 | SULF1 | Sulf1 |
| 5.529 | TFF1 | Tff1 |
| 6.52 | PTN | Ptn |
| 8.591 | MLF1 | Mlfl |
| 9.012 | THBS2 | Thbs2 |
| 10.79 | HEPH | Heph |
| 12.53 | JAM3 | Jam3 |

**Table 4. Examples of epithelial or mesenchymal genes in the expression data analysis of clones AT3-T and AT3-M.**

| **Gene name** | **x Fold change in AT3-T vs. AT3-M** |
|---|---|
| Junctional adhesion molecule C | 12.53 |
| Disintegrin-like and metalloprotease | 4.05 |
| Activated leukocyte cell adhesion molecule | 3.97 |
| Tubulin | 2.73 |
| Epithelial protein lost in neoplasm | 2.39 |
| Laminin | 2.20 |
| TGFβ2 | 0.45 |
| MMP9 | 0.36 |
| Collagen, type XVIII | 0.36 |
| MMP10 | 0.35 |
| Integrin β4 | 0.33 |
| TGFβ1 | 0.31 |
| Urokinase plasminogen activator | 0.29 |
| MMP3 | 0.15 |

Two gene sets were assembled: one composed of gene products upregulated in AT3-T (relative to AT3-M) and the second of those downregulated in AT3-T (relative to AT3-M). The two gene sets were compared for overlap with 5,452 gene sets from the Molecular Signature Database collections (Gene Set Enrichment Analysis (GSEA) http://www.broad.mit.edu/gsea/). Analysis of genes over-expressed in AT3-T relative to AT3-M for overlap with 5,452 gene sets from the Molecular Signature Database collections via Gene Set Enrichment Analysis (GSEA) did not show any significant enrichment of sets associated with EMT or MET. In this regard, both AT3-M and AT3-T resembled the mesenchymal-like, parental AT3 line. Among the 15 most significant overlaps for the genes overexpressed in AT3-T there were three sets of genes activated in hematopoetic stem cells (p = 3.24 x 10⁻⁸), neural stem cells (p = 3.07 x 10⁻⁷) and embryonal murine stem cells (p = 5.14 x 10⁻⁶), (Table 5) while among the 20 most significant overlaps for the genes that are relatively downregulated in AT3-T cells were two gene sets associated with development of mature cell types. Expression of the downstream hedgehog pathway effector GL13 was found to be 3.4-fold overexpressed in AT3-T cells compared to AT3-M cells, indicating that regulation of this developmental/stemness pathway in prostate cancer may be tied to the underlying phenotypic state during EMT/MET, similar to what has been reported in other tumors. These data indicated that AT3-T cells have gene expression profiles similar to stem cells, and, in concordance with the analysis of CD44 and CD 133 protein expression, suggested that AT3-T cells exist in a more stem cell-like state than the more mesenchymal AT3-M cells.

**Table 5.**

| GSEA Collections: | C1, C3, C2, C5, C4 | | | | |
|---|---|---|---|---|---|
| # overlaps shown: | 20 | | | | |
| # gene sets in collections: | 5452 | | | | |
| # genes in comparison (N) | 127 | | | | |
| # genes in collections (N) | 39655 | | | | |
| | | | | | |
| gene set name | # genes in gene set (k) | Description | # genes in overlap (k) | k/K | p value |
| TATAAA_V$TATA_O1 | 1333 | Genes with promoter regions [- 2kb,2kb] around transcription start site containing the motif TATAAA which matches annotation for TAF<br> TATA | 20 | 0.015 | 8.07E-09 |
| STEMCELL_HEMATOPOIETIC_UP | 1452 | Enriched in mouse hematopoietic stem cells, compared to differentiated brain and bone marrow cells | 20 | 0.0138 | 3.24E-08 |
| GNF2 RAP1B | 37 | Neighborhood of RAP1B | 5 | 0.1351 | 1.23E-07 |
| STEMCELL_NEURA L_UP | 1838 | Enriched in mouse neural stem cells, compared to differentiated brain and bone marrow cells | 21 | 0.0114 | 3.07E-07 |
| module 2 | 383 | Genes in Module 2 | 10 | 0.0261 | 4.34E-07 |
| CTTTGA_V$LEF1_Q2 | 1270 | Genes with promoter regions [-2kb,2kb] around transcription start site containing the motif CTTTGA which matches annotation for LEF 1: lymphoid enhancer-binding factor 1 | 17 | 0.0134 | 5.48E-07 |
| SIGNAL_TRANSDUCTION | 1637 | Genes annotated by the GO term GO:0007165. The cascade of processes by which a signal interacts with a receptor, causing a change in the level or activity of a second messenger or other downstream target, and ultimately effecting a change in the functioning of the cell. | 19 | 0.0116 | 9.33E-07 |
| module_385 | 28 | Genes in module 385 | 4 | 0.1429 | 1.91E06 |
| V$MYCMAX_O1 | 261 | Genes with promoter regions [-2kb,2kb] around transcription start site containing the motif NNACCACGTGG TNN which matches annotation for MYC: v-myc myelocytomatosis viral oncogene homolog (avian)<br> MAX: MYC associated factor X | 8 | 0.0307 | 1.98E06 |
| GGGCGGR V$SP1_Q 6 | 3053 | Genes with promoter regions [-2kb,2kb] around transcription start site containing the motif GGGCGGR which matches annotation for SP1: Sp1 transcription factor | 26 | 0.0085 | 2.59E-06 |
| AACTTT_UNKNOW N | 1963 | Genes with promoter regions [-2kb,2kb] around transcription start site containing motif AACTTT. Motif does not match any known transcription factor | 20 | 0.0102 | 3.29E-06 |
| V$AP1 C | 281 | Genes with promoter regions [-2kb,2kb] around transcription start site containing the motif NTGASTCAG which matches annotation for JUN: jun oncogene | 8 | 0.0285 | 3.38E-06 |
| MEMBRANE PART | 1673 | Genes annotated by the GO term GO:0044425 Any constituent part of a membrane, a double layer of lipid molecules that encloses all cells, and, in eukaryotes, many organelles; may be a single or double lipid bilayer; also includes associated proteins. | 18 | 0.0108 | 5.09E-06 |
| STEMCELL_EMBRYONIC_UP | 1344 | Enriched in mouse embryonic stem cells, compared to differentiated brain and bone marrow cells | 16 | 0.0119 | 5.14E-06 |
| INTRINSIC_TO_MEMBRANE | 1350 | Genes annotated by the GO term GO:0031224. Located in a membrane such that some covalently attached portion of the gene product, for example part of a peptide sequence or some other covalently attached moiety such as a GPI anchor, spans or is embedded in one or both leaflets of the membrane. | 16 | 0.0119 | 5.43E-06 |
| CELL SURFACE | 79 | Genes annotated by the GO term GO:0009986. The external part of the cell wall and/or plasma membrane. | 5 | 0.0633 | 5.58E-06 |
| UVC_XPCS_8HR_DN | 408 | Down-regulated at 8 hours following treatment of XPB/CS fibroblasts with 3 J/m^2 UVC | 9 | 0.0221 | 6.35E-06 |
| NOTCH_SIGNALING PATHWAY | 12 | Genes annotated by the GO term GO:0007219. The series of molecular signals initiated by binding of an extracellular ligand to a Notch receptor on the surface of the target cell. | 3 | 0.25 | 6.86E-06 |
| LEI_MYB_REGULATED GENES | 325 | Myb-regulated genes | 8 | 0.0246 | 9.62E-06 |
| MORF_DDB1 | 246 | Neighborhood of DDB1 | 7 | 0.0285 | 1.40E-05 |

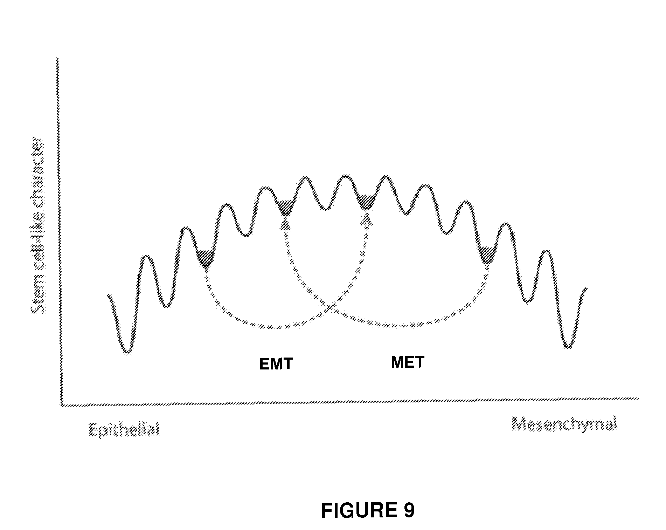

*Epithelial plasticity and sterns cell-like behavior.* It is well appreciated that cells induced to undergo EMT activate stem cell pathways. Work presented here shows that AT3 cells that transitioned towards a more epithelial state, i.e. were involved in MET, also activated expression of stem cell-like markers. This finding suggested a broader relationship between plasticity and stem cell-like character or stemness, which was modeled using a Gibbs free energy diagram (Figure 9). Figure 9 shows a model comparing stem cell-like character and epithelial-mesenchymal phenotype. The x-axis represents the spectrum of epithelial to mesenchymal phenotypes and the y-axis represents the stem cell-like character of the cells. The left arrow represents an EMT and the right arrow represents an MET. The model posits that as cells transition back and forth along the epithelial and mesenchymal x-axis they course through states of varying stemness, and this property peaks at intermediate states between epithelial and mesenchymal phenotypes. The number of different states and the exact height of the barriers between states are speculative and are not meant to be taken as proportional. Two phenotypic transitions are shown, the first is a partial EMT (left arrow) and the second is a partial MET (right arrow). Both of these transitions result in states with higher stem cell-like character. It should be noted that the model also predicts that some EMTs, and equally some METs, will result in a decrease in stemness and indeed this has been observed when the highly aggressive human DKAT basal-type breast cancer cell line is induced to undergo EMT (N. D'Amato and V. Seewaldt, personal communication). The model also suggests a link between stemness, plasticity, and metastatic propensity, perhaps explained by activation of certain oncogenic pathways (e.g., PI3 kinase/Akt) and developmental pathways.

The model also predicts that cells with maximal stem-cell character, which by definition will be highly malignant, should display both epithelial and mesenchymal traits, because they inhabit intermediate states in the epithelialmesenchymal axis. The highly malignant rat adenocarcinoma AT3-T cells are in this type of state. Importantly, in humans with metastatic breast and prostate carcinomas many CTCs also exist in these intermediate states. These cells correlate with disease progression and are believed to be highly aggressive. A population of cells enriched in CTCs expressed RNAs encoding mesenchymal markers; however, the data did not indicate whether or not epithelial and mesenchymal markers were co-expressed in the same cell. Another clinical example of cells in intermediate states is found in sarcomatoid renal cell carcinomas, which have been shown to co-express epithelial markers, such as epithelial membrane antigen, and mesenchymal ones, like vimentin. These tumors, though rare (1-8% of renal tumors) are highly aggressive and difficult to treat. A similar situation may be found in carcinosarcomas of both the prostate and breast, highly aggressive, rare tumors with mixed epithelial and mesenchymal components but of clonal origin. It is not completely clear whether or not single cells in these tumor co-express epithelial and mesenchymal markers and are thus truly in intermediate states.

Finally, the model suggests that as sarcomas undergo MET they will activate stem cell-like pathways and become more aggressive. Indeed, there are many descriptions of sarcomas with mixed epithelial and mesenchymal components in close proximity as seen in some synovial- and osteo- sarcomas. New genetically-defined mouse models of soft tissue sarcoma should shed light on the existence and importance of cells intermediate cell states in progression of these tumors.

### Example 7. Phenotypic plasticity among human circulating tumor cells

The experiments described above indicated that Dunning rat prostate adenocarcinoma cells that inhabit an intermediate phenotypic state are tumorogenic, metastatic, and possess stem cell-like antigens and cellular programs. To investigate whether or not similar transitional cells could play a role in human cancer, cancer cells isolated from blood of men with metastatic castrate resistant progressive prostate cancer (CRPC) or women with progressive metastatic breast cancer (mBC) were examined. Circulating tumor cells (CTCs) represent an ideal source of tissue to investigate evidence of this plasticity *in vivo,* given that these cells are likely to be in circulation prior to and during metastatic colonization. CTCs have both independent prognostic and predictive significance in multiple epithelial malignancies, including breast and prostate cancer. These cells can be collected, isolated, and analyzed for a variety of biomarkers relevant to cancer biology.

It was tested whether there was a high likelihood of finding transitional cells within a population of CTCs captured by FDA-approved EpCAM (Epithelial Cell Adhesion Molecule)-targeted ferromagnetic antibodies. These cells were interrogated for expression of CD45 (expressed in many leukocytes; Figure 10A), cytokeratin (CK; an epithelial marker), and vimentin (a mesenchymal marker) by immunofluorescence. CTCs were defined as CD45-negative and CK-positive nucleated intact cells (Figure 10B) and transitional CTCs were so defined if they additionally co-expressed vimentin (Figure 10C-D). Figure 10 shows that CTCs from patients with prostate adenocarcinoma stained positive for epithelial and mesenchymal markers. Triple staining was performed using anti-CD45 antibody labeled with Alexa 647, anti-cytokeratin (CK) antibody labeled with Alexa 555, and anti-vimentin antibody labeled with Alexa 488. Nuclei were labeled with DAPI. Figure 10A shows an example of a leukocyte from a human peripheral blood mononuclear cell sample: CD45 (+), CK (-), and vimentin (+). Additionally, CD45 (+), CK (-), and vimentin (-) cells were observed. Figure 10B shows an example of a CD45 (-), CK (+), and vimentin (-) cell from a patient with metastatic breast cancer. Such cells were counted as vimentin (-) CTCs in Table 6. Figure 10C shows an example of a CD45 (-), CK (+), vimentin (+) from a patient with metastatic breast cancer. Such cells were counted as vimentin (+) CTCs in Table 6. Figure 10D shows an example of a CD45 (-), CK (+), vimentin (+) from a patient with metastatic progressive castrate-resistant prostate cancer. Such cells were counted as vimentin (+) CTCs in Table 6.

Transitional CTCs co-expressed vimentin and CK in many of the patients with elevated CTC counts (≥5 CTCs/7.5 mL by standard testing) (Table 6, Figure 10). In fact, among nine patients with progressive metastatic CRPC and eight patients with progressive mBC, it was found that approximately 75% (range 0-100%, 85.5% in CRPC, 54% in mBC) of the CTCs stained for both CK and vimentin (Figure 10C-D), indicating a transitional phenotype. These data indicated that circulating tumor cells in patients with metastatic breast and prostate cancer co-express epithelial (EpCAM and cytokeratin) and mesenchymal (vimentin) markers, and thus exist in a transitional phenotypic state, similar to that observed in our preclinical models.

**Table 6. Circulating tumor cell (CTC) counts and vimentin expression in patients with metastatic castration resistant prostate or metastatic breast cancer.**

| Subject Number | CTC Count (Cellsearch)* | Ratio: vimentin (+) CTCs/Total CTC Count |
|---|---|---|
| Castrate-Resistance Metastatic Prostates Cancer | | |
| 1 | 5 | 4/6 |
| 2 | 41 | 11/11 |
| 3 | 45 | 6/10 |
| 4 | 626 | 5/8 |
| 5 | 110 | 17/21 |
| 6 | 182 | 5/6 |
| 7 | 17 | 13/16 |
| 8 | 19 | 33/34 |
| 9 | 34 | 12/12 |
| Total | | 106/124 (85.5%) |
| Metastatic Breast Cancer | | |
| 1 | 21 | 0/6 |
| 2 | 7 | 2/2 |
| 3 | 8 | 4/4 |
| 4 | 21 | 1/2 |
| 5 | 12 | 2/2 |
| 6 | 188 | 21/22 |
| 7 | 138 | 8/20 |
| 8 | 377 | 6/23 |
| Total | | 44/81 (54.3%) |
| Overall Total | -- | 150/205 (73.1%) |

| | | |
|---|---|---|
| *Column 2 represents the CTC count as determined by the standard Cellsearch EpCAM based method for each subject, while column 3 represents the number and proportion of CTCs counted manually that were found to express cytokeratin and co-express vimentin, expressed as a ratio and percentage. | | |

*Plasticity and CTCs.* The identification of plasticity among CTCs in a significant subset of patient samples offers several important clinical opportunities. Expression of plasticity may have prognostic or predictive value in patients with metastatic cancers, especially mBC where a significant range of values were shown for plasticity. Thus, the subset of patients with very high plasticity may have a more aggressive natural history and exhibit greater resistance to systemic treatments. In terms of diagnosis and utility as predictive biomarkers the data suggested that in addition to cells expressing both epithelial and mesenchymal markers there may be an unknown number of CTCs that have moved further towards the mesenchymal pole and are EpCAM negative. These cells will be missed by the FDA approved CellSearch® System and also by the Adna Test (AdnaGen AG) system and current microfluidic technologies, which enrich for CTCs by immunoabsorbtion of cells expressing MUC1 or EpCAM. Indeed, recent studies in breast cancer have suggested that "normal" type breast cancer cell lines that overexpress both EMT and stem cell antigens (CD44+, CD24-) may lack EpCAM and are thus not detectable by currently approved CTC detection systems. Therefore it is possible that the number of CTCs in patients with metastatic cancer is much higher than currently appreciated. Identification of this additional subset of CTC can provide greater prognostic value than CTC counts as currently determined, as well as earlier detection of CTCs and the metastatic potential in patients with earlier stage disease.

Furthermore, CTCs in intermediate states, which comprise the 50-75% of cells isolated herein from patients with metastatic breast and prostate cancer as well as those cells that may go undetected because they have undergone a more complete EMT, represent a therapeutic problem. It has been well documented that EMT alters drug sensitivity of lung cancer cells and it has been challenging to direct therapy to cancer cells with stem cell-like properties, perhaps because of their recalcitrance to undergo apoptosis.

While recent studies suggest both a screening method and actual compounds (e.g., salinomycin) that can selectively target cancer stem cells, these aggressive cells still represent a formidable therapeutic challenge. Thus, molecules comprising a binding agent that has binding specificity to an EMT biomarker described herein and linked to an anti-cancer agent provide additional therapeutic options.

### Example 8. CTCs from patients with metastatic breast and prostate cancer express vimentin and N-cadherin

Eligible men had progressive metastatic CRPC (progression despite testosterone < 50 ng/dL) and were about to begin a new systemic therapy. Eligible women had progressive metastatic breast cancer (mBC) and were about to begin a new systemic therapy. Baseline characteristics of patients (n = 29) are presented in Table 7.

**Table 7. Baseline characteristics of patients (n = 29)**

| | Metastatic Prostate (n = 17) | Metastatic Breast (n=12) |
|---|---|---|
| DEMOGRAPHICS | | |
| Age, median | 69 (59-82) | 61.5 (48-81) |
| Race, Ethnicity | | |
| White, non-hispanic | 76% | 58% |
| Other, non-hispanic | 23 % | 42% |

| BASELINE DISEASE HISTORY | | |
|---|---|---|
| Gleason Score, median | 7 (7-9) | --- |
| ER/PR, % | --- | 75% / 67% |
| Baseline median PSA, Range | 396.4 (14-13,419.5) | --- |
| Baseline Pain Score (0-10), median | 1 (0-7) | 0 (0-6) |
| Karnofsky Performance Status, median | 90 (70-100) | 90 (70-100) (n=6) |
| # of Prior Hormonal Therapies | 2 (0-5) | 2 (0-4) |
| Prior Chemotherapy | 47% | 83% |
| Baseline CTC Count, median | 40 (4-828) | 13(0-1062) |

| METASTATIC SITES | | |
|---|---|---|
| Lymph Node | 65% | 50% |
| Liver | 24% | 50% |
| Lung | 47% | 42% |
| Bone | 94% | 75% |

CTCs were drawn into standard FDA-approved Cellsave tubes and processed within 48 hours using the CellSearch® methodology using EpCAM-based ferromagnetic capture. A CTC was defined as an intact nucleated (DAPI+) cell that expressed pan-CK and lacked expression of the leukocyte antigen CD45, and was enumerated using standard methods. A second Cellsearch® tube was collected and processed using EpCAM capture, and isolated cells were stained for CK (IgG1, AbD Serotec) labeled with Alexa 555, CD45 (IgG1, AbCam) labeled with Alexa 647, and either vimentin (IgG1, BD Biosciences) or N-Cadherin (IgG1, DAKO) using immunoflouresent labeling with Alexa 488. The proportion of CTCs staining positive for an EMT antigen was calculated from the total number of CTCs manually scored from the second tube. Positive controls using American Red Cross-derived PBMCs (CD45), PC3 prostate cancer cells (vimentin, N-cadherin), and T47D breast cancer cells (CK) were used for each marker. Negative controls using mock antibody were used to optimize the staining/scoring of each antigen.

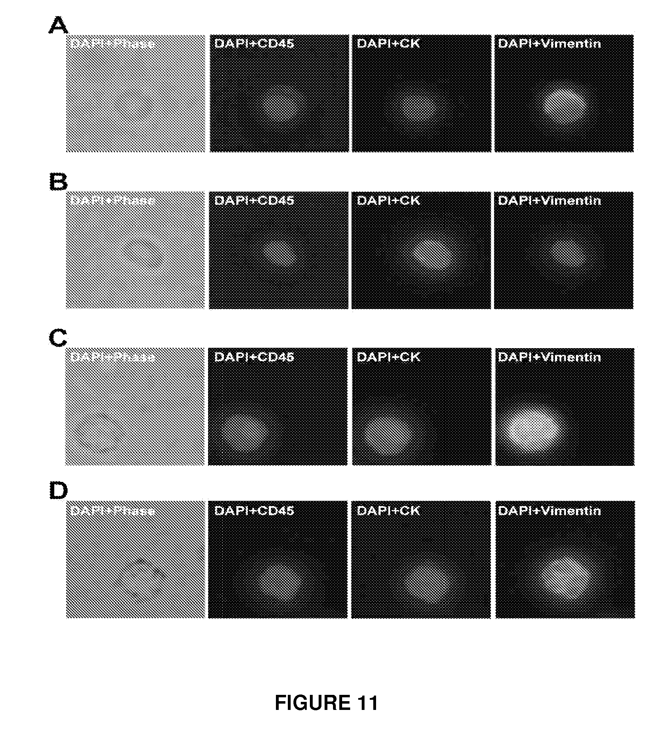

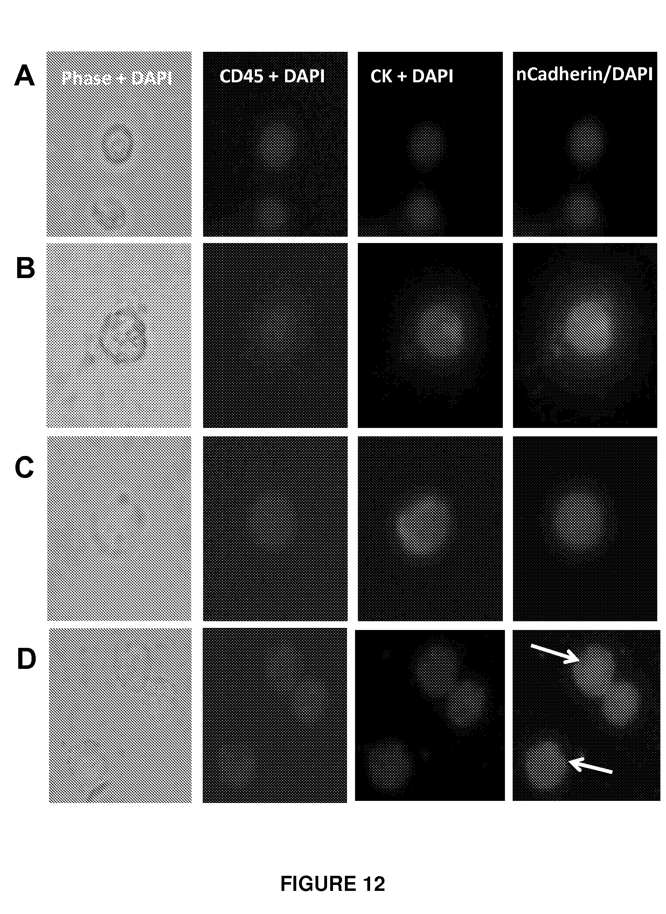

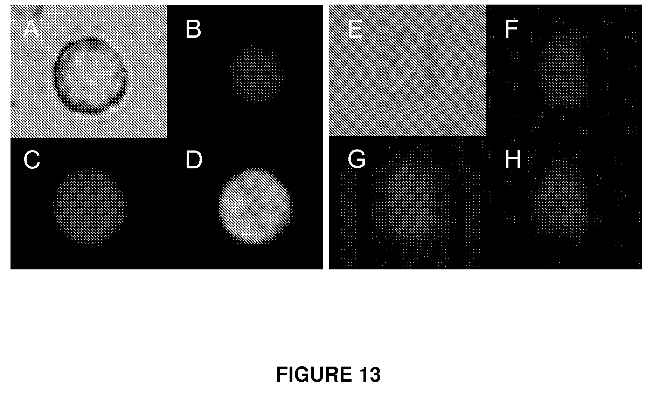

Prevalence of vimentin and CK co-expression in CTCs, and prevalence of N-cadherin and CK co-expression in CTCs are presented in Tables 8 and 9, respectively. Vimentin co-expression was detected in 17/20 (85%) patients with mCRPC or mBC and 78% of all N-Cadherin co-expression was detected in 8/9 (89%) patients and 81% of Immunofluorescent images of CTCs from patients with mCRPC and mBC are shown in Figure 11 (A, a leukocyte; B, vimentin negative CTC (CRPC); C, vimentin positive CTC (BC); and D) vimentin positive CTC (CRPC)). Immunofluorescent images of CTCs from patients with mCRPC and mBC are shown in Figure 12 (A, leukocyte; B, Ncad positive CTC (BC); C, Ncad negative CTC (BC); and D, two NCad positive CTCs (arrows) and 1 Ncad negative CTC (CRPC)). Immunofluorescent images of CTCs from patients with mCRPC and mBC are shown in Figure 13 (A, Phase/DAPI; B, CD45/DAPI; C, CK/DAPI; D, Vimentin/DAPI positivity in a man with mCRPC; E, Phase/DAPI; F, CD45/DAPI; G, CK/DAPI; and H, Vimentin/DAPI negativity in a second man with mCRPC).

The data showed the co-expression of cytokeratin with the EMT antigens vimentin and N-cadherin in CTCs from men with metatastic CRPC and women with metastatic breast cancer. A majority of CTCs examined co-expressed CK and EMT proteins by immunofluorescent labeling. The majority of patients in this study had CTCs that co-expressed vimentin or N-cadherin suggesting potential epithelial plasticity during metastasis. The data suggests that CTCs can lack epithelial markers and provide methods for assessing patients with breast and prostate cancer as well as for the optimal detection of circulating tumor cells in other common malignancies.

**Table 8.**

| **Subject Number** | | **CTC Count (Cellsearch)** | **Ratio of: Vimentin (+) CTCs / Total Manual CTC Count** |
|---|---|---|---|
| | 1 | 5 | 4/6 |
| | 2 | 4 | 2/2 |
| | 3 | 54 | 11/11 |
| castrate-resistant metastatic prostate cancer | 4 | 45 | 6/10 |
| | 5 | 626 | 5/8 |
| | 6 | 110 | 17/21 |
| | 7 | 182 | 5/6 |
| | 8 | 17* | 13/16 |
| | 9 | 19 | 33/34 |
| | 10 | 34 | 12/12 |
| Total | | 1127 | 108/126 (86%) |
| | 1 | 13 | 0/6 |
| | 2 | 85 | 2/2 |
| | 3 | 8 | 4/4 |
| | 4 | 21 | 1/2 |
| metastatic breast cancer | 5 | 12 | 2/2 |
| | 6 | 188 | 21/22 |
| | 7 | 324** | 29/33 |
| | 8 | 377 | 6/23 |
| | 9 | 0 | 0/0 |
| | 10 | 3 | 0/3 |
| Total | | 884 | 65/97 (67%) |
| Overall Total | | -- | 173/223 (78%) |

**Table 9.**

| **Subject Number** | | **CTC Count (Cellsearch)** | **Ratio of: N-Cadherin (+) CTCs / Total Manual CTC Count** |
|---|---|---|---|
| | 1 | 45 | 13/19 |
| | 2 | 12 | 5/7 |
| castrate-resistant metastatic prostate cancer | 3 | 10 | 8/8 |
| | 4 | 5 | 8/9 |
| | 5 | 12 | 4/4 |
| | 6 | 221 | 11/13 |
| | 7 | 828 | 81/96 |
| Total | | 1132 | 130/156 (83%) |
| metastatic breast cancer | 1 | 1062 | 9/13 |
| | 2 | 2 | 0/3 |
| Total | | 1064 | 9/16 (56%) |
| Overall Total | | -- | 139/172 (81%) |

| | | | |
|---|---|---|---|
| *Count from 3 months prior to baseline (no intervening therapy) **Count from time point #2 | | | |

In a second trial to test for the existence of transitional CTCs, blood was collected from 31 men with mCRPC and 16 women with mBC (see baseline characteristics for the patients in Table 10 and Table 11). CTCs were processed using the CellSearch® EpCAM-based immunocapture method and profiled for expression of CD45 (PTPRC) (a leukocyte marker), cytokeratins (CK) (epithelial markers), vimentin (VIM) and N-cadherin (CDH2) (mesenchymal markers), and CD133 (a stem cell marker) by immunofluorescence (IF) (Table 2). Leukocytes were defined as nucleated (DAPI positive), CD45-positive and CK-negative cells, whereas CTCs were defined as nucleated (DAPI positive), CD45-negative and CK-positive cells. Among CTCs we identified transitional cells as those that additionally expressed vimentin or N-cadherin.

**Table 10. Baseline demographic and clinical characteristics of the men with metastatic CPRC.**

| **DEMOGRAPHICS** | n=31 |
|---|---|
| Age, years (range) | 71 (59-89) |
| Race, Ethnicity | |
| White, non-Hispanic | 71 % |
| Black, non-Hispanic | 29% |

| **BASELINE DISEASE HISTORY** | |
|---|---|
| Median Gleason Score (range) | 8 (5-10) |
| Median Baseline PSA¹ (ng/dl, range) | 267.5 (14.0-13,419.5) |
| Median Baseline Pain (range)² | 1 (0-7) |
| Median Karnofsky Performance Status (range) | 90 (60-100) |
| Median Number of Prior Hormonal Therapies (range) | 3 (0-5) |
| Prior Chemotherapy | 65 % |
| Prior Bisphosphonates | 71 % |

| **SITES OF METASTATIC DISEASE** | |
|---|---|
| Visceral (lung + liver) | 35% |
| Lymph Node Only | 0% |
| Bone metastatic: | |
| Bone Metastatic With Lymph Nodes (no visceral metastases) | 39% |
| Bone Metastatic Without Lymph Nodes (no visceral metastases) | 26% |

| | |
|---|---|
| ¹ PSA: prostate specific antigen. ² Pain is scored as a linear analog scale (0-10 range). | |

**Table 11. Baseline characteristics of mBC patients.**

| **DEMOGRAPHICS** | n = 16 |
|---|---|
| Median age (range) | 61 (48-81) |
| Race, Ethnicity | |
| White, non-Hispanic | 44% |
| Black, non-Hispanic | 50% |
| Asian, non-hispanic | 6% |

| **BASELINE DISEASE HISTORY** | |
|---|---|
| ER and/or PR positive disease | 56% |
| HER2 positive disease (HER2 3+) | |
| | 0% |
| Median Karnofsky Performance Status (range) | 90 (70-90) |
| Median Number of Prior EndocrineTherapies (range) | 1 (0-4) |
| Median Number of Prior Chemotherapies | 2 (0-7) |

| **SITES OF METASTATIC DISEASE** | |
|---|---|
| Visceral (lung or liver) | 75% |
| Lymph Node Only | 0% |
| Lymph Node, soft tissue, or contralateral breast only Bone metastases only: | 13% |
| Bone Metastatic With Lymph Nodes (no visceral metastases) | 0% |
| Bone Metastatic Without Lymph Nodes (no visceral metastases) | 13% |

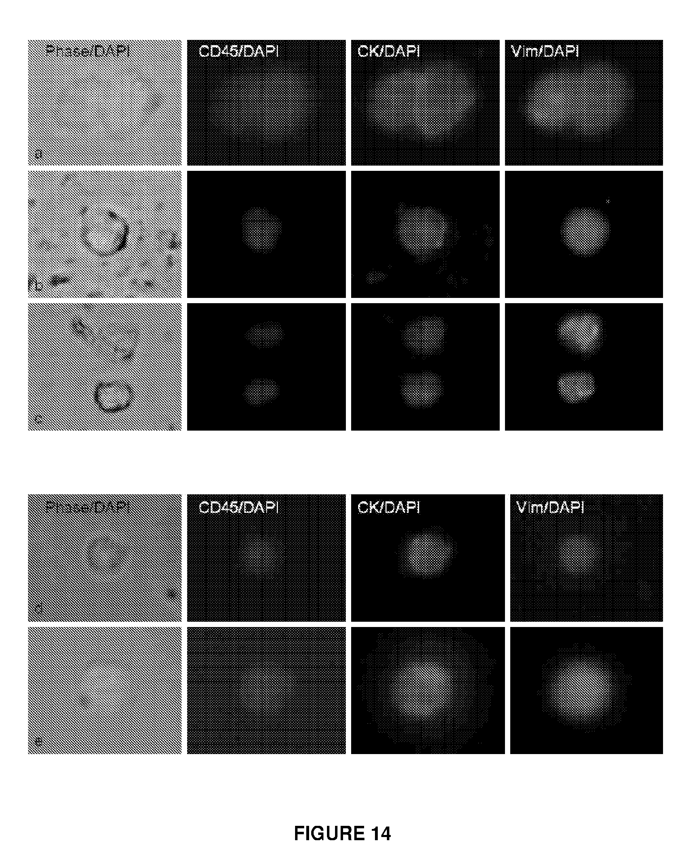

Among ten men with mCRPC, CTCs co-expressed vimentin and CK in 10/10 (100%) patients, and by this criterion 108/126 (86%) of enumerated CTCs were transitional (Table 12, Figure 14). Biopsies of bony metastases performed within one week of CTC collection in two of these patients revealed no vimentin expression in the CK positive tumor foci, but strong vimentin expression in the surrounding bone stroma, which lacks CK expression. These same patients had CTCs taken at the same time as the CT-guided tumor biopsy that commonly expressed co-expressed CK and vimentin. These findings are consistent with invasion and metastasis by transitional CTCs that subsequently undergo MET; alternatively, vimentin expression may be heterogeneously expressed in metastases, similar to CTC expression.

**Table 12. Circulating tumor cell (CTC) and transitional CTCs in patients with metastatic CRPC.**

| Subject Number | CTC Count (Cellsearch)ⁱ | Ratio: Vimentin (+) CTCs / Total Manual CTC Countⁱⁱ |
|---|---|---|
| 1 | 5 | 4/6 |
| 2 | 4 | 2/2 |
| 3 | 54 | 11/11 |
| 4 | 45 | 6/10 |
| 5 | 626 | 5/8 |
| 6 | 110 | 17/21 |
| 7 | 182 | 5/6 |
| 8 | 17 | 13/16 |
| 9 | 19 | 33/34 |
| 10 | 34 | 12/12 |
| Total | 1127 | 108/126 (86%) |

| Subject Number | CTC Count (Cellsearch) | Ratio: N-Cadherin (+) CTCs/ Total Manual CTC Count |
|---|---|---|
| 11 | 45 | 13/19 |
| 12 | 12 | 5/7 |
| 13 | 10 | 8/8 |
| 14 | 5 | 7/8 |
| 15 | 12 | 3/4 |
| 16 | 220 | 11/13 |
| 17 | 828 | 81/96 |
| 18 | 26 | 6/11 |
| 19 | 12 | 18/22 |
| 20 | 42 | 15/18 |
| Total | 1224 | 167/206 (81%) |

| Subject Number | CTC Count (Cellsearch) | Ratio: CD133 (+) CTCs/ Total Manual CTC Count |
|---|---|---|
| 21 | 485 | 38/38 |
| 22 | 16 | 6/11 |
| 23 | 91 | 15/21 |
| 24 | 6 | 0/0 |
| 25 | 36 | 29/29 |
| 26 | 27 | 9/9 |
| 27 | 43 | 10/15 |
| 28 | 2 | 0/0 |
| 29 | 23 | 12/14 |
| 30 | 38 | 23/26 |
| 31 | 30 | 12/17 |
| Total | 797 | 154/180 (86%) |

| | | |
|---|---|---|
| i The middle column represents the CTC Count from the FDA-approved Cellsearch® enumeration of CTCs for each subject. ii Right column represents the ratio of vimentin (co-expression of vimentin ranged from 60-100% of cells in a given individual and did not correlate with CTC count (R²=0.11)), N-cadherin (Co-expression of N-cadherin ranged from 55-100% of cells in a given individual, and did not correlate with CTC count (R²=-0.09)), or CD133 (CD133 co-expression ranged from 55-100% of evaluable cells in a given individual and did not correlate with CTC number (R²=0.04)) expressing CTCs among the total number of CTCs that were manually enumerated. A CTC was defined as an intact DAPI positive (nucleated) cell that lacked CD45 expression and expressed cytokeratin. | | |

**Table 13. CTCs and transitional CTCs in patients with mBC.**

| Subject Number | CTC Count (Cellsearch)i | Ratio: Vimentin (+) CTCs / Total Manual CTC Countii |
|---|---|---|
| 1 | 21 | 0/6 |
| 2 | 7 | 2/2 |
| 3 | 8 | 4/4 |
| 4 | 21 | 1/2 |
| 5 | 12 | 2/2 |
| 6 | 188 | 21/22 |
| 7 | 324 | 29/33 |
| 8 | 377 | 6/23 |
| 9 | 0 | 0/0 |
| 10 | 3 | 0/3 |
| Total | 961 | 65/97 (67%) |

| Subject Number | CTC Count (Cellsearch) | Ratio: N-Cadherin (+) CTCs/ Total Manual CTC Count |
|---|---|---|
| 11 | 1062 | 9/13 |
| 12 | 2 | 0/3 |
| 13 | 147 | 52/59 |
| 14 | 6 | 2/5 |
| 15 | 33 | 15/15 |
| 16 | 2 | 0/0 |
| Total | 1252 | 78/95 (82%) |

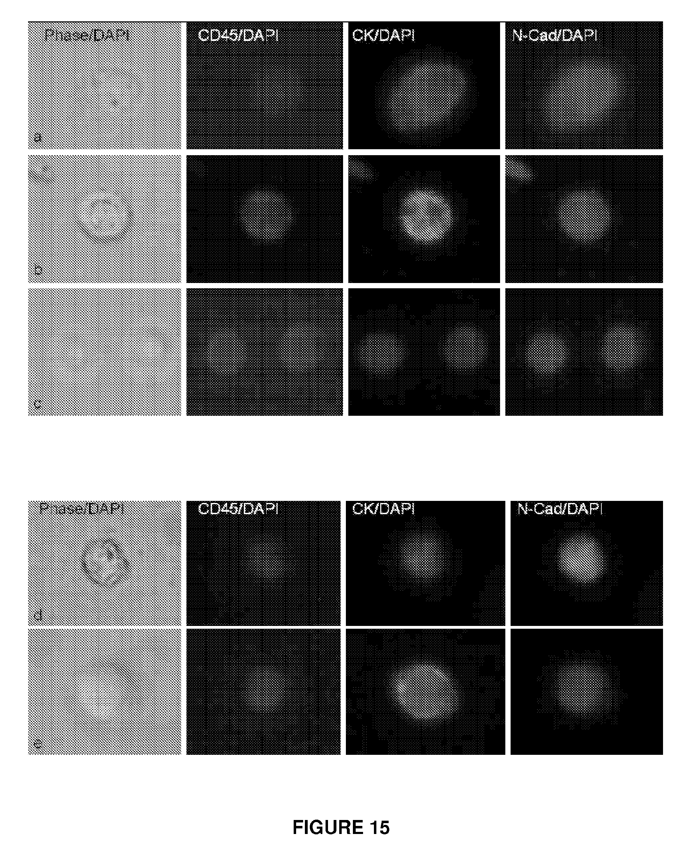

Among the next cohort of 10 men with mCRPC, CTCs co-expressed N-cadherin and CK in 10/10 (100%) patients, and by this criterion 167/206 (81 %) of CTCs were identified as transitional (Table 12, Figure 15). Among 10 women with mBC, nine had detectable CTCs and of these, we found evidence of vimentin co-expression in seven (78%) patients, and 55/88 CTCs overall (63%) co-expressed vimentin (Table 13, Figure 14). Among another six women with detectable CTCs and mBC, four had evidence of CK and N-cadherin co-expression, and overall 78/95 CTCs (82%) had N-cadherin expression, with significant heterogeneity in expression in a given individual (Table 13, Figure 15). These data indicate that many CTCs in patients with mBC and mCRPC co-express epithelial (EpCAM and cytokeratin) and mesenchymal (vimentin, N-cadherin) markers, and thus exist in a transitional phenotypic state, similar to that observed in our preclinical models.

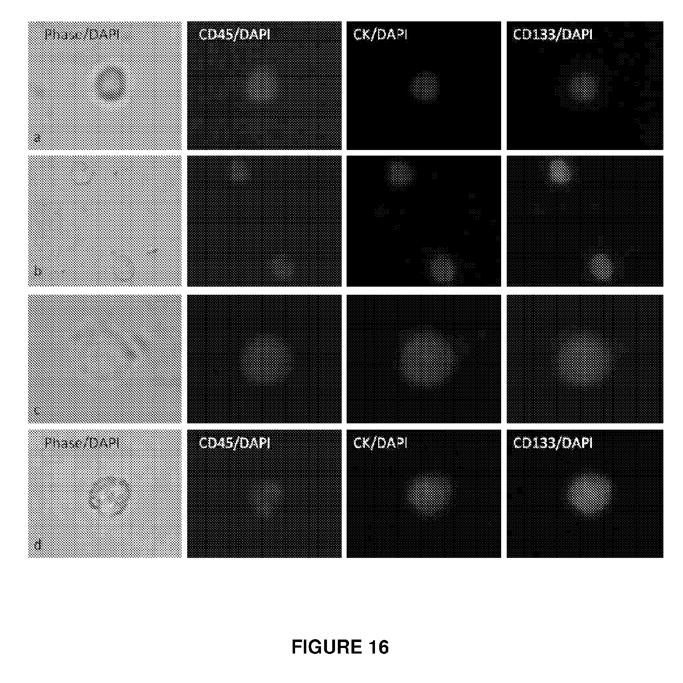

Given the expression of the stem cell associated antigen CD 133 in transitional AT3-T cells, CD 133 expression in CTCs from men with mCRPC was evaluated. CD 133 was expressed in 11/11 (100%) men with CTCs, and in 154/180 (86%) of CTCs from these men (Table 12, Figure 16). These data suggest that CTCs from patients with common epithelial malignancies inhabit transitional states characterized by co-expression of epithelial and mesenchymal markers as well as CD133, biomarkers that have been associated with stem-like properties, invasiveness, and chemoresistance.

### SEQUENCES

SEQ ID NO: 1
   N-cadherin (also known as cadherin-2, cdh2)
   From Mus musculus
   Gene No. 12558, Accession No. AB008811
   nucleotide (mRNA), 4321 bp
SEQ ID NO: 2
   N-cadherin (also known as cadherin-2, cdh2)
   From Mus musculus
   Gene No. 12558, Accession No. AB008811
   polypeptide, translation of SEQ ID NO: 1
SEQ ID NO: 3
   O-cadherin (also known as cadherin-11, cdh11, or ob-cadherin)
   From Xenopus laevis
   Gene No. 100337621, Accession No. AF002983
   nucleotide (RNA), 3237 bp
SEQ ID NO: 4
   O-cadherin (also known as cadherin-11, cdh11, or ob-cadherin)
   From Xenopus laevis
   Gene No. 100337621, Accession No. AF002983
   polypeptide, translation of SEQ ID NO: 3
SEQ ID NO: 5
   CD133 (also known as PROM-1, prominin-1), isoform 2
   From Mus musculus
   Gene No. 19126, Accession No. BC028286
   nucleotide (mRNA), 3701 bp
SEQ ID NO: 6
   CD133 (also known as PROM-1, prominin-1), isoform 2
   From Mus musculus
   Gene No. 19126, Accession No. BC028286
   polypeptide, translation of SEQ ID NO: 5
SEQ ID NO: 7
   FGFR2 IIIc
   From Mus musculus
   Gene No. 14183, Accession No. M86441
   nucleotide (mRNA), 3306 bp
SEQ ID NO: 8
   FGFR2 IIIc
   From Mus musculus
   Gene No. 14183, Accession No. M86441
   polypeptide, translation of SEQ ID NO: 7
SEQ ID NO: 9
   FGFR2 IIIb
   From Mus musculus
   Gene No. 14183, Accession No. M63503
   nucleotide (mRNA), 3037 bp
SEQ ID NO: 10
   FGFR2 IIIb
   From Mus musculus
   Gene No. 14183, Accession No. M63503
   polypeptide, translation of SEQ ID NO: 9
SEQ ID NO: 11
   E-cadherin (also known as cadherin-1, cdh1)
   From Xenopus (Silurana) tropicalis
   Gene No. 779546, Accession No. XM_002935997
   nucleotide (mRNA), 3344 bp
SEQ ID NO: 12
   E-cadherin (also known as cadherin-1, cdh1)
   From Xenopus (Silurana) tropicalis
   Gene No. 779546, Accession No. XM_002935997
   polypeptide (translation of SEQ ID NO: 11), 872 amino acids
SEQ ID NO: 13
   Vimentin
   From homo sapiens
   Accession No. BC000163
   nucleotide (mRNA), 1862 bp
SEQ ID NO: 14
   Vimentin
   From homo sapiens
   Accession No. BC000163
   polypeptide (translation of SEQ ID NO: 13), 466 amino acids
SEQ ID NO: 15
   N-cadherin
   From homo sapiens
   CCDS ID No. CCDS11891.1
   nucleotide, 2721 bp
SEQ ID NO: 16
   N-cadherin
   From homo sapiens
   CCDS ID No. CCDS11891.1
   polypeptide (translation of SEQ ID NO: 15), 906 amino acids
SEQ ID NO: 17
   O-cadherin (also known as ob-cadherin)
   From homo sapiens
   CCDS ID No. CCDS 10803.1
   nucleotide, 2391 bp
SEQ ID NO: 18
   O-cadherin (also known as ob-cadherin)
   From homo sapiens
   CCDS ID No. CCDS10803.1
   polypeptide (translation of SEQ ID NO: 17), 796 amino acids
SEQ ID NO: 19
   CD 133 (also known as PROM1)
   From homo sapiens
   CCDS ID No. CCDS47029.1
   nucleotide, 2598 bp
SEQ ID NO: 20
   CD 133 (also known as PROM1)
   From homo sapiens
   CCDS ID No. CCDS47029.1
   poplypeptide (translation of SEQ ID NO: 19), 865 amino acids
SEQ ID NO: 21
   FGFR2, isoform 1
   From homo sapiens
   CCDS ID No. CCDS31298.1
   nucleotide, 2466 bp
SEQ ID NO: 22
   FGFR2, isoform 1
   From homo sapiens
   CCDS ID No. CCDS31298.1
   polypeptide (translation of SEQ ID NO: 21), 821 amino acids
SEQ ID NO: 23
   E-cadherin (also known as CDH1)
   From homo sapiens
   CCDS ID No. CCDS10869.1
   nucleotide, 2649 bp
SEQ ID NO: 24
   E-cadherin (also known as CDH1)
   From homo sapiens
   CCDS ID No. CCDS10869.1
   polypeptide (translation of SEQ ID NO: 23), 882 amino acids

## Claims

1. A method for detecting a circulating tumor cell (CTC) in a biological sample, the method comprising detecting at least one epithelial mesenchymal transition (EMT) biomarker in the biological sample, wherein the at least one EMT biomarker is vimentin.

2. The method of claim 1, wherein the sample is a blood sample.

3. The method of any one of the preceding claims, wherein the method is performed at the time of or prior to cancer metastasis.

4. The method of any one of the preceding claims, wherein the at least one EMT biomarker is detected by flow cytometry, ferromagnetic enrichment, ferromagnetic sorting, or EMT antigen-antibody binding.

5. The method of any one of the preceding claims, comprising detecting at least two EMT biomarkers.

6. The method of any one of the preceding claims, wherein the at least one EMT biomarker further includes N-cadherin, O-cadherin, E-cadherin, FGFR2 splice variant isoforms, CD133, or any combination of two or more thereof.

## Patentansprüche

1. Ein Verfahren zur Detektion einer zirkulierenden Tumor Zelle (ZTZ) in einer biologischen Probe, wobei das Verfahren das Detektieren wenigstens eines epithelial-mesenchymalen Transitions- (EMT-) Biomarkers in der biologischen Probe umfasst, wobei der wenigstens eine EMT-Biomarker Vimentin ist.

2. Das Verfahren von Anspruch 1, wobei die Probe eine Blutprobe ist.

3. Das Verfahren von einem beliebigen der vorangegangenen Ansprüche, wobei das Verfahren zum Zeitpunkt einer oder vor einer Krebsmetastase ausgeführt wird.

4. Das Verfahren von einem beliebigen der vorangegangenen Ansprüche, wobei der wenigstens eine EMT-Biomarker mittels Durchflusszytometrie, ferromagnetischer Anreicherung, ferromagnetischer Sortierung oder EMT-Antigen-Antikörperbindung detektiert wird.

5. Das Verfahren von einem beliebigen der vorangegangenen Ansprüche, umfassend das Detektieren von wenigstens zwei EMT-Biomarkern.

6. Das Verfahren von einem beliebigen der vorangegangenen Ansprüche, wobei der wenigstens eine EMT-Biomarker ferner N-Cadherin, O-Cadherin, E-Cadherin, FGFR2 Spleißvariant-Isoformen, CD133, oder eine jegliche Kombination von zwei oder mehreren von diesen einschließt.

## Revendications

1. Procédé de détection d'une cellule tumorale circulante (CTC) dans un échantillon biologique, le procédé comprenant la détection d'au moins un biomarqueur de transition épithélio-mésenchymateuse (EMT) dans l'échantillon biologique, dans lequel ledit au moins un biomarqueur EMT est la vimentine.

2. Procédé selon la revendication 1, dans lequel l'échantillon est un échantillon de sang.

3. Procédé selon l'une quelconque des revendications précédentes, dans lequel le procédé est mis en oeuvre lors d'une métastase cancéreuse ou avant celle-ci.

4. Procédé selon l'une quelconque des revendications précédentes, dans lequel ledit au moins un biomarqueur EMT est détecté par cytométrie en flux, enrichissement ferromagnétique, tri ferromagnétique, ou liaison EMT antigène-anticorps.

5. Procédé selon l'une quelconque des revendications précédentes, comprenant la détection d'au moins deux biomarqueurs EMT.

6. Procédé selon l'une quelconque des revendications précédentes, dans lequel ledit au moins un biomarqueur EMT comprend en outre la N-cadhérine, l'O-cadhérine, l'E-cadhérine, des isoformes d'épissage du gène FGFR2, le CD133, ou toute combinaison de deux ou plusieurs de ceux-ci.


### SEQUENCE LISTING

<110> DUKE UNIVERSITY
<120> BIOMARKERS FOR CIRCULATING TUMOR CELLS
<130> 028193-9082 WO00
<140> New International Application
   <141> 2010-09-24
<150> 61/298,845
   <151> 2010-01-27
<150> 61/308,780
   <151> 2010-02-26
<150> 61/309,131
   <151> 2010-03-01
<160> 24
<170> PatentIn version 3.5
<210> 1
   <211> 4321
   <212> DNA
   <213> Mus musculus
<400> 1
   cacacacaca cgcacacaca cacacacaca cacttctcgg cgcgcacgac gcccgccctt 60
   ctccccgccc cctccccagc tccttgatct cccgtctgtt ttattactcc tggtgcgagt 120
   ccggcggact ccgaggcccg ctatttgtta ccaactcgct ctcattggcg gggaggagag 180
   cagcggagaa gggggtgggg aggggagggg aagggaaggg gtggccactg ccggagccga 240
   ctccgcgctg ctgttggtgc cgctgccgct tctgctgcct ctgctgccgc cgccgccgcc 300
   tccggctcct cgctcggccc ctctccgcct ccatgtgccg gatagcggga gcgccgcgga 360
   ccctgctgcc gcttctggcg gccttgcttc aggcgtctgt ggaggcttct ggtgaaattg 420
   cattatgcaa gactggattt cctgaagatg tttacagcgc agtcttaccg aaggatgtgc 480
   acgaaggaca gccccttctc aatgtgaaat tcagcaactg caatagaaaa aggaaagttc 540
   agtatgaaag cagcgagcca gcagatttca aggtggacga ggacggcacg gtgtatgctg 600
   tgagaagctt ccctctcact gcagagcagg caaagttcct gatatatgcc caagacaaag 660
   aaacccagga aaagtggcag gtagctgtaa acctgagccg ggagccaacc ctgactgagg 720
   agcctatgaa ggaaccacat gaaattgaag aaatagtatt ccctagacaa cttgccaagc 780
   acagtggagc tctacaaagg cagaagagag actgggtcat cccgccaatc aacttgccag 840
   aaaactccag aggacccttt cctcaagagc ttgtcagaat caggtctgat agagataaaa 900
   acctttccct gagatacagc gtcactgggc caggagctga ccagcctcca acgggcatct 960
   tcattatcaa ccccatctca ggacagctgt cagtcacaaa gcctctggat cgagagctga 1020
   tagcccggtt tcacttgaga gcacatgcag tggacatcaa tggcaatcaa gtggagaacc 1080
   ccattgacat tgtcatcaat gttattgaca tgaatgataa cagacctgag tttctgcacc 1140
   aggtttggaa tgggtctgtt ccagagggat caaagcctgg gacgtatgtg atgacggtca 1200
   ctgccattga tgcggatgat ccaaatgccc tgaatggaat gctgcggtac aggatcctgt 1260
   cccaggcgcc cagcacacct tcacccaaca tgtttacaat caacaatgag actggggaca 1320
   tcatcactgt ggcagctggt ctggatcgag agaaagtgca acagtatacg ttaataattc 1380
   aagccacaga catggaaggc aatcccactt atggcctttc aaacacagcc acagccgtca 1440
   tcacggtgac agatgtcaat gacaatcctc cagagtttac tgccatgact ttctacggag 1500
   aagtccctga gaacagggtg gacgtcattg tagccaacct aactgtcacg gacaaagatc 1560
   agccccacac gccggcctgg aatgcggcat acagaatcag tggtggagac cctacaggaa 1620
   ggtttgccat cctgacagac cccaacagca atgatgggct agtcacagtg gtaaaaccaa 1680
   ttgactttga aacgaatagg atgtttgtcc ttactgttgc tgcagaaaac caagtgccat 1740
   tagctaaagg cattcagcac ccacctcagt cgacagccac tgtgtctgtg acagttattg 1800
   atgtcaatga aaatccttat tttgccccaa atcctaaaat cattcgccaa gaggaaggcc 1860
   tccacgcagg taccatgctg accacgctca ctgctcagga ccccgatcga tatatgcaac 1920
   agaatatcag atacacaaaa ttgtctgatc ctgccaactg gctgaaaata gaccccgtga 1980
   atgggcagat cactactatt gccgttttgg acagagaatc gccaaatgta aaaaacaaca 2040
   tctataatgc taccttcctt gcttctgaca atggaatccc gcctatgagt gggacaggaa 2100
   cactgcaaat ctatttactt gatatcaatg acaacgcccc tcaggtgtta cctcaagagg 2160
   cggagacctg tgaaactcca gaacccaact caattaacat cacagcactt gattatgaca 2220
   tagacccaaa cgccgggccg ttcgcgtttg atcttccctt atctccagtg actattaaaa 2280
   gaaactggac catcaaccgg cttaatggtg attttgctca gctcaattta aagataaaat 2340
   ttttggaagc tggtatctat gaagttccca tcattatcac agattcaggg aatcccccca 2400
   agtccaacat ttccatcctg cgtgtgaaag tttgtcagtg tgactccaat ggagactgca 2460
   cggacgtgga caggatcgtg ggtgcagggc ttggcacggg cgccatcatc gctatccttc 2520
   tgtgtatcat catcctgctg atccttgttc tcatgtttgt ggtatggatg aaacggcggg 2580
   ataaagagcg ccaagccaag cagcttttaa ttgacccaga agatgatgta agagataata 2640
   tattgaaata tgatgaagaa ggtggaggag aagaagacca ggactatgac ttgagccagc 2700
   tccagcaacc agatactgtg gagcctgatg ccatcaagcc cgtgggaatc agacggctag 2760
   acgagaggcc tatccatgct gagccacagt acccagtccg atccgcagcc ccacaccctg 2820
   gggatattgg ggacttcatt aatgagggcc ttaaagctgc tgacaacgac cccacggcgc 2880
   caccgtatga ctccctctta gtctttgact acgagggcag cggctccacg gctggctcct 2940
   tgagctccct caactcctcc agtagcggtg gggaccagga ctatgactac ctgaatgact 3000
   ggggaccccg cttcaagaaa ctggcggaca tgtacggcgg tggtgacgac tgaacggcag     3060
   gacggacttg gcttttggac aagtatgaac agtttcacct gatattccca aaaaaaagca     3120
   tacagaagct aggctttaac tctgtagtcc actagcaccg tgcttgctgg aggctttggc     3180
   gtaggctgcg aaccagtttg ggctcccagg gaatatcagt gatccaatac tgtctggaaa     3240
   acaccgagct cagctacact tgaattttac agtaaagaag cactgggatt tatgtgcctt     3300
   tttgtacctt tttcagattg gaattagttt tctgtttaag gctttaatgg tactgatttc     3360
   tgaaatgata aggaaaagac aaaatatttt gtggcgggag cagaaagtta aatgtgatac     3420
   gcttcaaccc acttttgtta caatgcattt gcttttgtta agatacagaa cgaaacaacc     3480
   agattaaaaa aaattaactc atggagtgat tttgttacct ttggggtggg ggggatgaga     3540
   ccacaagata ggaaaatgta cattacttct agttttagac tttagatttt tttttttcac     3600
   taaaatctta aaacttacgc agctggttgc agataaaggg agttttcata tcaccaattt     3660
   gtagcaaaat gaattttttc ataaactaga atgttagaca cattttggtc ttaatccatg     3720
   tacacttttt tattttctgt attttttcca cctcgctgta aaaatggtgt gtgtacataa     3780
   tgtttatcag catagactat ggaggagtgc agagaactcg gaacatgtgt atgtattatt     3840
   tggactttgg attcaggttt tttgcatgtt aatatctttc gttatgggta aagtatttac     3900
   aaaacaaagt gacatttgat tcaactgttg agctgtagtt agaatactca atttttaatt     3960
   ttttaatttt ttttaaattt ttttattttc tttttgtttg tttcgttttg gggaggggta     4020
   aaagttctta gcacaatgtt ttacataatt tgtaccaaaa aaattacaca caaaaaaaaa     4080
   aaaaagaaaa gaaaagaaaa gtgaaagggg tggcctgttt cttgcagcac tagcaagtgt     4140
   gtgtttttaa aaaacaaaac aaacaaacaa aaaaataaat aaaaagagga aaaagaaaaa     4200
   aaaaaaagct tttaaactgg agagacttct gaaacagctt tgcgtctgtg ttgtgtacca     4260
   gaatacaaac aatacacctc tgaccccagc gttctgaata aaaagctaat tttggatctg     4320
   g                                                                     4321
<210> 2
   <211> 906
   <212> PRT
   <213> Mus musculus
<400> 2
   Met Cys Arg Ile Ala Gly Ala Pro Arg Thr Leu Leu Pro Leu Leu Ala
   1               5                   10                  15
   Ala Leu Leu Gln Ala Ser Val Glu Ala Ser Gly Glu Ile Ala Leu Cys
               20                  25                  30
   Lys Thr Gly Phe Pro Glu Asp Val Tyr Ser Ala Val Leu Pro Lys Asp
           35                  40                  45
   Val His Glu Gly Gln Pro Leu Leu Asn Val Lys Phe Ser Asn Cys Asn
       50                  55                  60
   Arg Lys Arg Lys Val Gln Tyr Glu Ser Ser Glu Pro Ala Asp Phe Lys
   65                  70                  75                  80
   Val Asp Glu Asp Gly Thr Val Tyr Ala Val Arg Ser Phe Pro Leu Thr
                   85                  90                  95
   Ala Glu Gln Ala Lys Phe Leu Ile Tyr Ala Gln Asp Lys Glu Thr Gln
               100                 105                 110
   Glu Lys Trp Gln Val Ala Val Asn Leu Ser Arg Glu Pro Thr Leu Thr
           115                 120                 125
   Glu Glu Pro Met Lys Glu Pro His Glu Ile Glu Glu Ile Val Phe Pro
       130                 135                 140
   Arg Gln Leu Ala Lys His Ser Gly Ala Leu Gln Arg Gln Lys Arg Asp
   145                 150                 155                 160
   Trp Val Ile Pro Pro Ile Asn Leu Pro Glu Asn Ser Arg Gly Pro Phe
                   165                 170                 175
   Pro Gln Glu Leu Val Arg Ile Arg Ser Asp Arg Asp Lys Asn Leu Ser
               180                 185                 190
   Leu Arg Tyr Ser Val Thr Gly Pro Gly Ala Asp Gln Pro Pro Thr Gly
           195                 200                 205
   Ile Phe Ile Ile Asn Pro Ile Ser Gly Gln Leu Ser Val Thr Lys Pro
       210                 215                 220
   Leu Asp Arg Glu Leu Ile Ala Arg Phe His Leu Arg Ala His Ala Val
   225                 230                 235                 240
   Asp Ile Asn Gly Asn Gln Val Glu Asn Pro Ile Asp Ile Val Ile Asn
                   245                 250                 255
   Val Ile Asp Met Asn Asp Asn Arg Pro Glu Phe Leu His Gln Val Trp
               260                 265                 270
   Asn Gly Ser Val Pro Glu Gly Ser Lys Pro Gly Thr Tyr Val Met Thr
           275                 280                 285
   Val Thr Ala Ile Asp Ala Asp Asp Pro Asn Ala Leu Asn Gly Met Leu
       290                 295                 300
   Arg Tyr Arg Ile Leu Ser Gln Ala Pro Ser Thr Pro Ser Pro Asn Met
   305                 310                 315                 320
   Phe Thr Ile Asn Asn Glu Thr Gly Asp Ile Ile Thr Val Ala Ala Gly
                   325                 330                 335
   Leu Asp Arg Glu Lys Val Gln Gln Tyr Thr Leu Ile Ile Gln Ala Thr
               340                 345                 350
   Asp Met Glu Gly Asn Pro Thr Tyr Gly Leu Ser Asn Thr Ala Thr Ala
           355                 360                 365
   Val Ile Thr Val Thr Asp Val Asn Asp Asn Pro Pro Glu Phe Thr Ala
       370                 375                 380
   Met Thr Phe Tyr Gly Glu Val Pro Glu Asn Arg Val Asp Val Ile Val
   385                 390                 395                 400
   Ala Asn Leu Thr Val Thr Asp Lys Asp Gln Pro His Thr Pro Ala Trp
                   405                 410                 415
   Asn Ala Ala Tyr Arg Ile Ser Gly Gly Asp Pro Thr Gly Arg Phe Ala
               420                 425                 430
   Ile Leu Thr Asp Pro Asn Ser Asn Asp Gly Leu Val Thr Val Val Lys
           435                 440                 445
   Pro Ile Asp Phe Glu Thr Asn Arg Met Phe Val Leu Thr Val Ala Ala
       450                 455                 460
   Glu Asn Gln Val Pro Leu Ala Lys Gly Ile Gln His Pro Pro Gln Ser
   465                 470                 475                 480
   Thr Ala Thr Val Ser Val Thr Val Ile Asp Val Asn Glu Asn Pro Tyr
                   485                 490                 495
   Phe Ala Pro Asn Pro Lys Ile Ile Arg Gln Glu Glu Gly Leu His Ala
               500                 505                 510
   Gly Thr Met Leu Thr Thr Leu Thr Ala Gln Asp Pro Asp Arg Tyr Met
           515                 520                 525
   Gln Gln Asn Ile Arg Tyr Thr Lys Leu Ser Asp Pro Ala Asn Trp Leu
       530                 535                 540
   Lys Ile Asp Pro Val Asn Gly Gln Ile Thr Thr Ile Ala Val Leu Asp
   545                 550                 555                 560
   Arg Glu Ser Pro Asn Val Lys Asn Asn Ile Tyr Asn Ala Thr Phe Leu
   565 570 575
   Ala Ser Asp Asn Gly Ile Pro Pro Met Ser Gly Thr Gly Thr Leu Gln
   580 585 590
   Ile Tyr Leu Leu Asp Ile Asn Asp Asn Ala Pro Gln Val Leu Pro Gln
   595 600 605
   Glu Ala Glu Thr Cys Glu Thr Pro Glu Pro Asn Ser Ile Asn Ile Thr
   610 615 620
   Ala Leu Asp Tyr Asp Ile Asp Pro Asn Ala Gly Pro Phe Ala Phe Asp
   625 630 635 640
   Leu Pro Leu Ser Pro Val Thr Ile Lys Arg Asn Trp Thr Ile Asn Arg
   645 650 655
   Leu Asn Gly Asp Phe Ala Gln Leu Asn Leu Lys Ile Lys Phe Leu Glu
   660 665 670
   Ala Gly Ile Tyr Glu Val Pro Ile Ile Ile Thr Asp Ser Gly Asn Pro
   675 680 685
   Pro Lys Ser Asn Ile Ser Ile Leu Arg Val Lys Val Cys Gln Cys Asp
   690 695 700
   Ser Asn Gly Asp Cys Thr Asp Val Asp Arg Ile Val Gly Ala Gly Leu
   705 710 715 720
   Gly Thr Gly Ala Ile Ile Ala Ile Leu Leu Cys Ile Ile Ile Leu Leu
   725 730 735
   Ile Leu Val Leu Met Phe Val Val Trp Met Lys Arg Arg Asp Lys Glu
   740 745 750
   Arg Gln Ala Lys Gln Leu Leu Ile Asp Pro Glu Asp Asp Val Arg Asp
   755 760 765
   Asn Ile Leu Lys Tyr Asp Glu Glu Gly Gly Gly Glu Glu Asp Gln Asp
   770 775 780
   Tyr Asp Leu Ser Gln Leu Gln Gln Pro Asp Thr Val Glu Pro Asp Ala
   785 790 795 800
   Ile Lys Pro Val Gly Ile Arg Arg Leu Asp Glu Arg Pro Ile His Ala
   805 810 815
   Glu Pro Gln Tyr Pro Val Arg Ser Ala Ala Pro His Pro Gly Asp Ile
   820 825 830
   Gly Asp Phe Ile Asn Glu Gly Leu Lys Ala Ala Asp Asn Asp Pro Thr
   835 840 845
   Ala Pro Pro Tyr Asp Ser Leu Leu Val Phe Asp Tyr Glu Gly Ser Gly
   850 855 860
   Ser Thr Ala Gly Ser Leu Ser Ser Leu Asn Ser Ser Ser Ser Gly Gly
   865 870 875 880
   Asp Gln Asp Tyr Asp Tyr Leu Asn Asp Trp Gly Pro Arg Phe Lys Lys
   885 890 895
   Leu Ala Asp Met Tyr Gly Gly Gly Asp Asp
   900 905
<210> 3
   <211> 3237
   <212> DNA
   <213> Xenopus laevis
<400> 3
   tcggcacgag ctggagtgta caggactttt aagatgctgc tgggtgtctg cactgtgtcc      60
   atgtgaatgt ggcattttta ttttgaattc cctccggaga caagatttca tcaagagttt     120
   cctttggata ttaagtcaaa gtgcaagcaa tggagattct ctataagaag gcaataatct     180
   gggggattta ctaaaattaa acaaacagat tgacattcgc tggatttatc aagcaatttt     240
   gcatttacaa cactaccaaa aatgaagaaa gacttttgct tacacggttt acttttatgt     300
   ttgggaattg cgtattgtag tcatgccaca tctttaagaa aaaacaataa actaaggcaa     360
   tcattccatg gtcaccatga aaaaggcaaa gaagggcaag ttttacatag gtcaaagaga     420
   ggatgggttt ggaatcaatt ttttgtaata gaagaataca ccggaccaga tcctgtactc     480
   gttggacggc ttcactcaga tgttgactct ggagattgga agataaaata catactctca     540
   ggagagggtg ctgggaccat ttttgtcatt gatgacaaat cagggaatat ccatgcaacc      600
   aagaccctgg atcgagaaga aagggctcag tataccttaa tggctcaggc agttgacaga     660
   gaaacaaata aaccactgga accaccatca gagtttatcg ttaaagttca agacataaat     720
   gataatcccc cggagttctt gcatgaaaac taccacgcaa atgtgcctga gatgtccaat     780
   gtgggtacat cagtaattca agtaacagcc tctgatgcag atgatccaac atatggaaac     840
   agcgctaagc ttgtgtatag tattctcgaa gggcagccat atttttcagt cgaagcacaa     900
   tcaggaatca ttaggactgc ccttccaaac atggacagag aagccaagga agaataccat     960
   gttgttattc aagcaaagga tatgggagga catatgggag gactctcagg gacaactaaa    1020
   gtgacaataa cgctgacaga tgtcaatgac aatccaccaa agtttccaca aagtgcgtac    1080
   cccatgtctg tgtcagaagc tgctgtccca ggggaagagg ttggcagaat aaaagctaaa    1140
   gatccagaca ttggagaaaa tggcttaata aagtaccgta ttcttgaagg agatggggca 1200
   gagatgtttg aaatcacagc tgattatgta actcaggaag gcgttgtaaa gctaaaaaag 1260
   gtggtggatt atgaaaccaa gaagttctac agtatgaagg ttgaagctgt caacgttcat 1320
   attgatccca gattccttag ccggggacca ttcaaagaca ctgctactgt taagatctca 1380
   gtagaggatt ttgatgaacc gcctattttc ttagaaagaa gttacatttt ggaagtatat 1440
   gaaaatgctc catcggatac tgtggtcgga agagtgcacg ctaaagaccc agatgctgct 1500
   aacagcccaa ttaggtattc aatcgatcgc cacactgacc ttgacagatt cttcagcatc 1560
   aacccagagg atggtgtcat caaaaccaca aagggtttgg atagagagga aagcccttgg 1620
   cacaacatct cagtcattgc aactgaagtc cacaatcgaa ttcatgaaac tagagttcca 1680
   gtagctatta aagtcttgga taagaatgac aatgctccgg aatttgcaaa gccctatgaa 1740
   gcttttgtct gtgaaaatgc tccaatcaat caggagtttt tgaccatcac tgcagtagat 1800
   aaagatgata cagccaatgg acttcgtttt ctctttagtt tccccccaga aattgtacat 1860
   ccaaatccaa atttcaccat aatagacaaa cgagataaca cagcaagcat ccgtgttggc 1920
   cgtggagttt tcagccgaca gaaacaagac ttgtatttgg ttcctattgt tataagtgat 1980
   gggggaagcc caccgatgag cagcaccaat accctttctg tccgaatctg cagttgcaat 2040
   agtgatggat cccaactatc ttgtaatgct gaaccccaat cccttaacgc tggactcagt 2100
   actggagcac tgattgcaat ccttgcttgc attgtaattt tattagtgat tgtggttttg 2160
   tttgtgactc tgaggagaga gaagaaggaa cctctaattg tctttgaaga ggaagatatc 2220
   cgggaaaata taattacata tgatgatgaa ggtggtggag aggaagacac cgaagcattt 2280
   gacattgcaa cactgcagaa tcctgatggg attaatggat ttatgccacg gaaagatatc 2340
   aaacccgaat ttcaatataa ccccagagat attggaataa gaccagcacc aaacagtgtt 2400
   gacgttgatg acttcattaa cacaaggata catgaggccg ataatgaccc tgcagctccg 2460
   ccttatgact ccattcagat ctatggatac gaagggagag gttctgtggc tggctctctt 2520
   agttcattag agtcagcctc tacagattca gatttggact atgattatct acaaaactgg 2580
   ggacctcgat ttaagaaact agcaaattta tatgggtcca aagacacttg tgaagatgat 2640
   tcttaacaaa taagttctga atttggcctt atgaactgca taatgtactg aaatatccag 2700
   agtaaacatt aacaggtatt tttttaaagg aaaacatgaa aaaggcttct ttaaccttcc 2760
   aaggtttaca aacaggattc cttccaaaac aagaactgtt aaatggtggt ggatactgtg 2820
   aaaaccctat ggcctgtgta gaagttgtgt attcattttt ttttttgttt tttgtttttt 2880
   ttccaagaaa ccacttgtaa aatgcagcct atttaaggga atggaaatgc aggaaaaacg 2940
   caacaaaaaa ggggaatctt tacagtatta aacataacca tcaaatcttc tcaaacaaag 3000
   cttccacaca aaaaaaaaaa aagataacag ttttgagctg taatttcgcc ttaaactatg 3060
   gacactttat atgtagtgca tttttaaact tgaaaaaaat atatatataa tatccagcca 3120
   gcttcaatcc atataatgta tgtacagtaa aatgtacaat tattctgtct cttgagcatc 3180
   agacttgtta ctgctgattc ttgtaaatct tttttgctta taatcccctc gtgccga 3237
<210> 4
   <211> 794
   <212> PRT
   <213> Xenopus laevis
<400> 4
   Met Lys Lys Asp Phe Cys Leu His Gly Leu Leu Leu Cys Leu Gly Ile
   1               5                   10                  15
   Ala Tyr Cys Ser His Ala Thr Ser Leu Arg Lys Asn Asn Lys Leu Arg
               20                  25                  30
   Gln Ser Phe His Gly His His Glu Lys Gly Lys Glu Gly Gln Val Leu
           35                  40                  45
   His Arg Ser Lys Arg Gly Trp Val Trp Asn Gln Phe Phe Val Ile Glu
       50                  55                  60
   Glu Tyr Thr Gly Pro Asp Pro Val Leu Val Gly Arg Leu His Ser Asp
   65                  70                  75                  80
   Val Asp Ser Gly Asp Trp Lys Ile Lys Tyr Ile Leu Ser Gly Glu Gly
                   85                  90                  95
   Ala Gly Thr Ile Phe Val Ile Asp Asp Lys Ser Gly Asn Ile His Ala
               100                 105                 110
   Thr Lys Thr Leu Asp Arg Glu Glu Arg Ala Gln Tyr Thr Leu Met Ala
           115                 120                 125
   Gln Ala Val Asp Arg Glu Thr Asn Lys Pro Leu Glu Pro Pro Ser Glu
       130                 135                 140
   Phe Ile Val Lys Val Gln Asp Ile Asn Asp Asn Pro Pro Glu Phe Leu
   145                 150                 155                 160
   His Glu Asn Tyr His Ala Asn Val Pro Glu Met Ser Asn Val Gly Thr
                   165                 170                 175
   Ser Val Ile Gln Val Thr Ala Ser Asp Ala Asp Asp Pro Thr Tyr Gly
               180                 185                 190
   Asn Ser Ala Lys Leu Val Tyr Ser Ile Leu Glu Gly Gln Pro Tyr Phe
           195                 200                 205
   Ser Val Glu Ala Gln Ser Gly Ile Ile Arg Thr Ala Leu Pro Asn Met
       210                 215                 220
   Asp Arg Glu Ala Lys Glu Glu Tyr His Val Val Ile Gln Ala Lys Asp
   225                 230                 235                 240
   Met Gly Gly His Met Gly Gly Leu Ser Gly Thr Thr Lys Val Thr Ile
                   245                 250                 255
   Thr Leu Thr Asp Val Asn Asp Asn Pro Pro Lys Phe Pro Gln Ser Ala
               260                 265                 270
   Tyr Pro Met Ser Val Ser Glu Ala Ala Val Pro Gly Glu Glu Val Gly
           275                 280                 285
   Arg Ile Lys Ala Lys Asp Pro Asp Ile Gly Glu Asn Gly Leu Ile Lys
       290                 295                 300
   Tyr Arg Ile Leu Glu Gly Asp Gly Ala Glu Met Phe Glu Ile Thr Ala
   305                 310                 315                 320
   Asp Tyr Val Thr Gln Glu Gly Val Val Lys Leu Lys Lys Val Val Asp
                   325                 330                 335
   Tyr Glu Thr Lys Lys Phe Tyr Ser Met Lys Val Glu Ala Val Asn Val
               340                 345                 350
   His Ile Asp Pro Arg Phe Leu Ser Arg Gly Pro Phe Lys Asp Thr Ala
           355                 360                 365
   Thr Val Lys Ile Ser Val Glu Asp Phe Asp Glu Pro Pro Ile Phe Leu
       370                 375                 380
   Glu Arg Ser Tyr Ile Leu Glu Val Tyr Glu Asn Ala Pro Ser Asp Thr
   385                 390                 395                 400
   Val Val Gly Arg Val His Ala Lys Asp Pro Asp Ala Ala Asn Ser Pro
                   405                 410                 415
   Ile Arg Tyr Ser Ile Asp Arg His Thr Asp Leu Asp Arg Phe Phe Ser
               420                 425                 430
   Ile Asn Pro Glu Asp Gly Val Ile Lys Thr Thr Lys Gly Leu Asp Arg
           435                 440                 445
   Glu Glu Ser Pro Trp His Asn Ile Ser Val Ile Ala Thr Glu Val His
       450                 455                 460
   Asn Arg Ile His Glu Thr Arg Val Pro Val Ala Ile Lys Val Leu Asp
   465                 470                 475                 480
   Lys Asn Asp Asn Ala Pro Glu Phe Ala Lys Pro Tyr Glu Ala Phe Val
                   485                 490                 495
   Cys Glu Asn Ala Pro Ile Asn Gln Glu Phe Leu Thr Ile Thr Ala Val
               500                 505                 510
   Asp Lys Asp Asp Thr Ala Asn Gly Leu Arg Phe Leu Phe Ser Phe Pro
           515                 520                 525
   Pro Glu Ile Val His Pro Asn Pro Asn Phe Thr Ile Ile Asp Lys Arg
       530                 535                 540
   Asp Asn Thr Ala Ser Ile Arg Val Gly Arg Gly Val Phe Ser Arg Gln
   545                 550                 555                 560
   Lys Gln Asp Leu Tyr Leu Val Pro Ile Val Ile Ser Asp Gly Gly Ser
                   565                 570                 575
   Pro Pro Met Ser Ser Thr Asn Thr Leu Ser Val Arg Ile Cys Ser Cys
               580                 585                 590
   Asn Ser Asp Gly Ser Gln Leu Ser Cys Asn Ala Glu Pro Gln Ser Leu
           595                 600                 605
   Asn Ala Gly Leu Ser Thr Gly Ala Leu Ile Ala Ile Leu Ala Cys Ile
       610                 615                 620
   Val Ile Leu Leu Val Ile Val Val Leu Phe Val Thr Leu Arg Arg Glu
   625                 630                 635                 640
   Lys Lys Glu Pro Leu Ile Val Phe Glu Glu Glu Asp Ile Arg Glu Asn
                   645                 650                 655
   Ile Ile Thr Tyr Asp Asp Glu Gly Gly Gly Glu Glu Asp Thr Glu Ala
               660                 665                 670
   Phe Asp Ile Ala Thr Leu Gln Asn Pro Asp Gly Ile Asn Gly Phe Met
           675                 680                 685
   Pro Arg Lys Asp Ile Lys Pro Glu Phe Gln Tyr Asn Pro Arg Asp Ile
       690                 695                 700
   Gly Ile Arg Pro Ala Pro Asn Ser Val Asp Val Asp Asp Phe Ile Asn
   705                 710                 715                 720
   Thr Arg Ile His Glu Ala Asp Asn Asp Pro Ala Ala Pro Pro Tyr Asp
   725 730 735
   Ser Ile Gln Ile Tyr Gly Tyr Glu Gly Arg Gly Ser Val Ala Gly Ser
   740 745 750
   Leu Ser Ser Leu Glu Ser Ala Ser Thr Asp Ser Asp Leu Asp Tyr Asp
   755 760 765
   Tyr Leu Gln Asn Trp Gly Pro Arg Phe Lys Lys Leu Ala Asn Leu Tyr
   770 775 780
   Gly Ser Lys Asp Thr Cys Glu Asp Asp Ser
   785 790
<210> 5
   <211> 3701
   <212> DNA
   <213> Mus musculus
<400> 5
   gtccaatcag tgcgctcaga ctcagagccc taggctcctg ctctttaaat taccgagcct 60
   tgtggagacc ccggcacctg gccttaagct cagccctgag gatggtactt tgagtgaatg 120
   accaccttgg agaccgttct tctgtttccc ttgttaccag ccaggaggca gaagagtcca 180
   ccggtccagg aaagacccat ttcccttgag tttccagaaa gtacctcatg cttgagagat 240
   caggccaaca actatggctc tcgtcttcag tgccctgctg ttactggggc tgtgtggaaa 300
   gatctcttca gaaggtcagc ctgcattcca taacactcct ggggctatga attatgaatt 360
   gcctaccacc aaatatgaga cccaagatac cttcaatgct gggattgttg gccctctcta 420
   caaaatggtg cacatcttcc tcaacgtggt ccagccgaat gacttccctc tagatttgat 480
   caaaaaactc atacagaaca agaactttga catctcagtt gattccaagg agccagaaat 540
   catagtcttg gctctgaaga ttgccctcta tgagatcgga gtccttatct gcgccatcct 600
   gggactgctg ttcattatcc tcatgcctct ggtgggctgc ttcttttgta tgtgccgttg 660
   ctgcaacaaa tgcggcggag agatgcacca gcggcagaag cagaatgcgc catgcaggag 720
   gaagtgcttg ggcctctccc tcctggtgat ttgtctgctc atgagccttg gcattatata 780
   tggctttgtg gctaaccagc agaccaggac tcggatcaaa gggacccaga aactggcaaa 840
   gagcaatttc agagactttc aaacactcct gactgaaaca ccaaagcaaa ttgactatgt 900
   agtggagcag tacaccaaca ccaagaacaa ggcattctca gacctggatg gcatcggctc 960
   cgtgctggga ggcagaataa aggaccaact aaaacccaaa gtaactcctg tcctcgaaga 1020
   gattaaggcc atggcgacag ccatcaaaca gaccaaggat gccctgcaga acatgagcag 1080
   cagcctgaaa agtctccaag atgcagccac ccagctcaat accaacctga gctctgtgag 1140
   aaacagcatc gagaattcgc tcagcagcag tgactgtacc tcagatccag ccagcaagat 1200
   ctgcgatagc atcagaccaa gcctaagcag tctggggagc agcctcaatt caagtcagct 1260
   cccatcagtg gatagagaac tcaacactgt tactgaagtc gacaaaactg atctggagag 1320
   cctcgtcaaa agggggtata cgacaattga tgaaataccc aatacaatac aaaaccaaac 1380
   tgtggatgtc atcaaagacg tcaaaaatac cttggactcc attagctcca acattaagga 1440
   catgagccaa agtattccta ttgaggatat gctgttacag gtctcccatt accttaataa 1500
   cagcaacaga tacttaaacc aggagctgcc caagctggaa gaatatgact cgtactggtg 1560
   gctgggtggc ttgattgtct gctttctgct gactctcatt gtgaccttct ttttcctggg 1620
   cttgctgtgt ggtgtgtttg gctatgacaa gcatgccacc ccaactagaa gaggctgtgt 1680
   gtccaacact ggaggcatct tcctcatggc tggggttgga ttcggcttcc ttttttgctg 1740
   gatattgatg atccttgtgg ttcttacgtt tgttgttggt gcaaatgtgg aaaagttgct 1800
   ctgcgaacct tatgaaaaca agaaattatt acaggttttg gacactccct atctgctcaa 1860
   ggaacaatgg caattttatc tttctggcat gctattcaat aacccagaca ttaacatgac 1920
   ctttgagcaa gtctacaggg attgcaaaag aggtcgaggt atatatgctg cttttcagct 1980
   tgagaatgtc gtcaacgtca gtgatcattt caacattgac cagatttctg aaaacataaa 2040
   tacggagttg gaaaacctga atgtgaacat tgatagcatt gaactgttgg ataacacagg 2100
   aaggaagagc ctcgaggact ttgcacattc tgggatagat acaatcgatt attccacata 2160
   cttgaaggag actgagaaat cccctactga agtgaatctg ctgacatttg cctctaccct 2220
   ggaagcaaaa gcaaaccagt tgcctgaagg aaagctgaaa caggccttct tactggatgt 2280
   acagaatata agagccatcc accagcatct cctccctcct gtgcagcaat cactgaaatt 2340
   tgtgagggtg aggaatacgt taagacaaag tgtctggacc ctccagcaaa caagcaacaa 2400
   gttgccggag aaagtgaaga agatccttgc ctctttggac tctgttcagc atttcctcac 2460
   caataacgtt tccctcatcg ttatcgggga aacgaagaag tttgggaaaa caatactagg 2520
   ctactttgaa cattatctgc actgggtctt ttatgccatc acagagaaga tgacatcctg 2580
   caaacccatg gccaccgcga tggactctgc tgttaatggc attctgtgtg gctatgttgc 2640
   ggaccctctg aatttgttct ggttcggcat agggaaagcc acggtgctct tacttccggc 2700
   tgtaatcatt gctatcaagc tggccaagta ctatcgcagg atggattcag aggatgtata 2760
   cgacgacccg tctcgatact gacaactgga gttgaagctg cttgaacaac aagatagtca 2820
   acatggaaag catcacagat tttggatagt ttctgagtct tctagaacgt tccaagtgca 2880
   gaagaaacct ggtggagact caggcgggca ctaggaacat ggcatcagtg gtcttagggt 2940
   agcactttgt caggaatgaa cagtcatcat ggttataatc cacatatcca ttgcaactca 3000
   tgaatgattc tctcctgttt tgtttttaac ttttcttttt acactgattt tctatttaga 3060
   cactaaaaca tataggggtg cttattcccc ctggatacat ttacctgtga accagctatt 3120
   ccggtgtcat agctgggtac ctaacttact tccatatgtg aagtgtgcta aacacaaacc 3180
   agtttacaga agagatgtat tttgtgtata gtaaactgta tatataccct tttaccacag    3240
   tcagtttttt aaacaaatga atactctaga tttttcttct aaatgaggtt actgttgggg    3300
   tggttgtgac ctagtgatgc tgtagaaagg agtctgcatt cactaaaagt gtgtcaacct    3360
   agagcaggca atgcccttcc ttgtggattt ctgtctgctc gttttggagc tacctgcggt    3420
   ttagaaatag aattcaagaa caatcacgga gtttcccact tgatgccact gccaaagtca    3480
   gaacaaggga tcttgagaga aggaactgtc gctcagctgg gagcggaatc attatcgcaa    3540
   tcacaggtcc tggttcacag tttagtggca ctctctggtt tgtaagaatg ggcattacgt    3600
   tcagtgtcat ctggtcatct gtgatgtgtg tcatcagcct gtcctgatgt tgagatttaa    3660
   aataaagcat gaatgaacag aaaaaaaaaa aaaaaaaaaa a                        3701
<210> 6
   <211> 842
   <212> PRT
   <213> Mus musculus
<400> 6
   Met Ala Leu Val Phe Ser Ala Leu Leu Leu Leu Gly Leu Cys Gly Lys
   1               5                   10                  15
   Ile Ser Ser Glu Gly Gln Pro Ala Phe His Asn Thr Pro Gly Ala Met
               20                  25                  30
   Asn Tyr Glu Leu Pro Thr Thr Lys Tyr Glu Thr Gln Asp Thr Phe Asn
           35                  40                  45
   Ala Gly Ile Val Gly Pro Leu Tyr Lys Met Val His Ile Phe Leu Asn
       50                  55                  60
   Val Val Gln Pro Asn Asp Phe Pro Leu Asp Leu Ile Lys Lys Leu Ile
   65                  70                  75                  80
   Gln Asn Lys Asn Phe Asp Ile Ser Val Asp Ser Lys Glu Pro Glu Ile
                   85                  90                  95
   Ile Val Leu Ala Leu Lys Ile Ala Leu Tyr Glu Ile Gly Val Leu Ile
               100                 105                 110
   Cys Ala Ile Leu Gly Leu Leu Phe Ile Ile Leu Met Pro Leu Val Gly
           115                 120                 125
   Cys Phe Phe Cys Met Cys Arg Cys Cys Asn Lys Cys Gly Gly Glu Met
       130                 135                 140
   His Gln Arg Gln Lys Gln Asn Ala Pro Cys Arg Arg Lys Cys Leu Gly
   145                 150                 155                 160
   Leu Ser Leu Leu Val Ile Cys Leu Leu Met Ser Leu Gly Ile Ile Tyr
                   165                 170                 175
   Gly Phe Val Ala Asn Gln Gln Thr Arg Thr Arg Ile Lys Gly Thr Gln
               180                 185                 190
   Lys Leu Ala Lys Ser Asn Phe Arg Asp Phe Gln Thr Leu Leu Thr Glu
           195                 200                 205
   Thr Pro Lys Gln Ile Asp Tyr Val Val Glu Gln Tyr Thr Asn Thr Lys
       210                 215                 220
   Asn Lys Ala Phe Ser Asp Leu Asp Gly Ile Gly Ser Val Leu Gly Gly
   225                 230                 235                 240
   Arg Ile Lys Asp Gln Leu Lys Pro Lys Val Thr Pro Val Leu Glu Glu
                   245                 250                 255
   Ile Lys Ala Met Ala Thr Ala Ile Lys Gln Thr Lys Asp Ala Leu Gln
               260                 265                 270
   Asn Met Ser Ser Ser Leu Lys Ser Leu Gln Asp Ala Ala Thr Gln Leu
           275                 280                 285
   Asn Thr Asn Leu Ser Ser Val Arg Asn Ser Ile Glu Asn Ser Leu Ser
       290                 295                 300
   Ser Ser Asp Cys Thr Ser Asp Pro Ala Ser Lys Ile Cys Asp Ser Ile
   305                 310                 315                 320
   Arg Pro Ser Leu Ser Ser Leu Gly Ser Ser Leu Asn Ser Ser Gln Leu
                   325                 330                 335
   Pro Ser Val Asp Arg Glu Leu Asn Thr Val Thr Glu Val Asp Lys Thr
               340                 345                 350
   Asp Leu Glu Ser Leu Val Lys Arg Gly Tyr Thr Thr Ile Asp Glu Ile
           355                 360                 365
   Pro Asn Thr Ile Gln Asn Gln Thr Val Asp Val Ile Lys Asp Val Lys
       370                 375                 380
   Asn Thr Leu Asp Ser Ile Ser Ser Asn Ile Lys Asp Met Ser Gln Ser
   385                 390                 395                 400
   Ile Pro Ile Glu Asp Met Leu Leu Gln Val Ser His Tyr Leu Asn Asn
                   405                 410                 415
   Ser Asn Arg Tyr Leu Asn Gln Glu Leu Pro Lys Leu Glu Glu Tyr Asp
               420                 425                 430
   Ser Tyr Trp Trp Leu Gly Gly Leu Ile Val Cys Phe Leu Leu Thr Leu
           435                 440                 445
   Ile Val Thr Phe Phe Phe Leu Gly Leu Leu Cys Gly Val Phe Gly Tyr
       450                 455                 460
   Asp Lys His Ala Thr Pro Thr Arg Arg Gly Cys Val Ser Asn Thr Gly
   465                 470                 475                 480
   Gly Ile Phe Leu Met Ala Gly Val Gly Phe Gly Phe Leu Phe Cys Trp
                   485                 490                 495
   Ile Leu Met Ile Leu Val Val Leu Thr Phe Val Val Gly Ala Asn Val
               500                 505                 510
   Glu Lys Leu Leu Cys Glu Pro Tyr Glu Asn Lys Lys Leu Leu Gln Val
           515                 520                 525
   Leu Asp Thr Pro Tyr Leu Leu Lys Glu Gln Trp Gln Phe Tyr Leu Ser
       530                 535                 540
   Gly Met Leu Phe Asn Asn Pro Asp Ile Asn Met Thr Phe Glu Gln Val
   545                 550                 555                 560
   Tyr Arg Asp Cys Lys Arg Gly Arg Gly Ile Tyr Ala Ala Phe Gln Leu
                   565                 570                 575
   Glu Asn Val Val Asn Val Ser Asp His Phe Asn Ile Asp Gln Ile Ser
               580                 585                 590
   Glu Asn Ile Asn Thr Glu Leu Glu Asn Leu Asn Val Asn Ile Asp Ser
           595                 600                 605
   Ile Glu Leu Leu Asp Asn Thr Gly Arg Lys Ser Leu Glu Asp Phe Ala
       610                 615                 620
   His Ser Gly Ile Asp Thr Ile Asp Tyr Ser Thr Tyr Leu Lys Glu Thr
   625                 630                 635                 640
   Glu Lys Ser Pro Thr Glu Val Asn Leu Leu Thr Phe Ala Ser Thr Leu
                   645                 650                 655
   Glu Ala Lys Ala Asn Gln Leu Pro Glu Gly Lys Leu Lys Gln Ala Phe
               660                 665                 670
   Leu Leu Asp Val Gln Asn Ile Arg Ala Ile His Gln His Leu Leu Pro
           675                 680                 685
   Pro Val Gln Gln Ser Leu Lys Phe Val Arg Val Arg Asn Thr Leu Arg
       690                 695                 700
   Gln Ser Val Trp Thr Leu Gln Gln Thr Ser Asn Lys Leu Pro Glu Lys
   705                 710                 715                 720
   Val Lys Lys Ile Leu Ala Ser Leu Asp Ser Val Gln His Phe Leu Thr
                   725                 730                 735
   Asn Asn Val Ser Leu Ile Val Ile Gly Glu Thr Lys Lys Phe Gly Lys
               740                 745                 750
   Thr Ile Leu Gly Tyr Phe Glu His Tyr Leu His Trp Val Phe Tyr Ala
           755                 760                 765
   Ile Thr Glu Lys Met Thr Ser Cys Lys Pro Met Ala Thr Ala Met Asp
       770                 775                 780
   Ser Ala Val Asn Gly Ile Leu Cys Gly Tyr Val Ala Asp Pro Leu Asn
   785                 790                 795                 800
   Leu Phe Trp Phe Gly Ile Gly Lys Ala Thr Val Leu Leu Leu Pro Ala
                   805                 810                 815
   Val Ile Ile Ala Ile Lys Leu Ala Lys Tyr Tyr Arg Arg Met Asp Ser
               820                 825                 830
   Glu Asp Val Tyr Asp Asp Pro Ser Arg Tyr
           835                 840
<210> 7
   <211> 3306
   <212> DNA
   <213> Mus musculus
<400> 7
   gaattcccgc gcggccgcca gagctccggc ccgggggctg cctgtgtgtt cctggcccgg      60
   cgtggcgact gctctccggg ctggcggggg ccgggcgtga gcccgggcct cagcgttcct     120
   gagcgctgcg agtgttcact actcgccagc aaagtttgga gtaggcaacg caagctccag     180
   tcctttcttc tgctgctgcc cagatccgag agcagctccg gtgtatgtct agctgttctg     240
   cgatcccggc gcgcgtgaag cctcggaacc ttggcgccgg ctgctaccca aggaatcgtt     300
   ctctttttgg agttttcctc cgagatcatc gcctgctcca tcccgatcca ctctgggctc     360
   cggcgcagca ccgagcgcag aggagcgctg ccattcaagt ggcagccaca gcagcagcag     420
   cagcagcagt gggagcagga acagcagtaa caacagcaac agcagcacag ccgcctcaga     480
   gctttgctcc tgagcccctg tgggctgaag gcattgcagg tagcccatgg tctcagaaga 540
   agtgtgcaga tgggattacc gtccacgtgg agatatggaa gaggaccagg gattggcact 600
   gtgaccatgg tcagctgggg gcgcttcatc tgcctggtct tggtcaccat ggcaaccttg 660
   tccctggccc ggccctcctt cagtttagtt gaggatacca ctttagaacc agaagagcca 720
   ccaaccaaat accaaatctc ccaaccagaa gcgtacgtgg ttgcccccgg ggaatcgcta 780
   gagttgcagt gcatgttgaa agatgccgcc gtgatcagtt ggactaagga tggggtgcac 840
   ttggggccca acaataggac agtgcttatt ggggagtatc tccagataaa aggtgccaca 900
   cctagagact ccggcctcta tgcttgtact gcagctagga cggtagacag tgaaacttgg 960
   atcttcatgg tgaatgtcac agatgccatc tcatctggag atgatgagga cgacacagat 1020
   agctccgaag acgttgtcag tgagaacagg agcaaccaga gagcaccgta ctggaccaac 1080
   accgagaaga tggagaagcg gctccacgct tgtcctgccg ccaacactgt gaagttccgc 1140
   tgtccggctg gggggaatcc aacgtccaca atgaggtggt taaaaaacgg gaaggagttt 1200
   aagcaggagc atcgcattgg aggctataag gtacgaaacc agcactggag ccttattatg 1260
   gaaagtgtgg tcccgtcaga caaaggcaac tacacctgcc tggtggagaa tgaatacggg 1320
   tccatcaacc acacctacca cctggatgtc gttgaacgtt caccacaccg tcccatcctc 1380
   caagctggac tgcctgcaaa tgcctccacg gtggtcggag gggatgtgga gtttgtctgc 1440
   aaggtttaca gcgatgccca gccccacatc cagtggatca agcacgtgga aaagaacggc 1500
   agtaaaaacg ggcctgatgg gctgccctac ctcaaggttc tgaaagctgc cggtgttaac 1560
   accacggaca aagagattga ggttctctat attcggaatg taacttttga ggatgctggg 1620
   gaatatacgt gcttggcggg taattctatc gggatatcct ttcactctgc atggttgaca 1680
   gttctgccag cgcctgtgag agagaaggag atcacggctt ccccagatta tctggagata 1740
   gctatttact gcataggggt cttcttaatc gcctgcatgg tggtgacagt catcttttgc 1800
   cgaatgaaga ccacgaccaa gaagccagac ttcagcagcc agccagctgt gcacaagctg 1860
   accaagcgca tccccctgcg gagacaggta acagtttcgg ccgagtccag ctcctccatg 1920
   aactccaaca ccccgctggt gaggataaca acgcgtctgt cctcaacagc ggacaccccg 1980
   atgctagcag gggtctccga gtatgagttg ccagaggatc caaagtggga attccccaga 2040
   gataagctga cgctgggcaa acccctgggg gaaggttgct tcgggcaagt agtcatggct 2100
   gaagcagtgg gaatcgataa agacaaaccc aaggaggcgg tcaccgtggc agtgaagatg 2160
   ttgaaagatg atgccacaga gaaggacctg tctgatctgg tatcagagat ggagatgatg 2220
   aagatgattg ggaaacataa gaacattatc aacctcctgg gggcctgcac gcaggatgga 2280
   cctctctacg tcatagttga atatgcatcg aaaggcaacc tccgggaata cctccgagcc 2340
   cggaggccac ctggcatgga gtactcctat gacattaacc gtgtccccga ggagcagatg 2400
   accttcaagg acttggtgtc ctgcacctac cagctggcta gaggcatgga gtacttggct     2460
   tcccaaaaat gtatccatcg agatttggct gccagaaacg tgttggtaac agaaaacaat 2520
   gtgatgaaga tagcagactt tggcctggcc agggatatca acaacataga ctactataaa 2580
   aagaccacaa atgggcgact tccagtcaag tggatggctc ctgaagccct ttttgataga 2640
   gtttacactc atcagagcga tgtctggtcc ttcggggtgt taatgtggga gatctttact 2700
   ttagggggct caccctaccc agggattccc gtggaggaac tttttaagct gctcaaagag 2760
   ggacacagga tggacaagcc caccaactgc accaatgaac tgtacatgat gatgagggat 2820
   tgctggcatg ctgtaccctc acagagaccc acattcaagc agttggtcga agacttggat 2880
   cgaattctga ctctcacaac caatgaggaa tacttggatc tcacccagcc tctcgaacag 2940
   tattctccta gttaccccga cacaagtagc tcttgttctt caggggacga ttctgtgttt 3000
   tctccagacc ccatgcctta tgaaccctgt ctgcctcagt atccacacat aaacggcagt 3060
   gttaaaacat gagtgaatgt gtcttcctgt ccccaaacag gacagcacca ggaacctact 3120
   tacactgagc agagaggctg tgctccagag cctgtgacac gcctccactt gtatatatgg 3180
   atcagaggag taaatagtgg gaagcatatt tgtcacgtgt gtaaagattt atacagttgg 3240
   aacatgtact acaggaagga gactgttctg atagtgacag ccgccaccat gccacctttg 3300
   accaca 3306
<210> 8
   <211> 821
   <212> PRT
   <213> Mus musculus
<400> 8
   Met Val Ser Trp Gly Arg Phe Ile Cys Leu Val Leu Val Thr Met Ala
   1               5                   10                  15
   Thr Leu Ser Leu Ala Arg Pro Ser Phe Ser Leu Val Glu Asp Thr Thr
               20                  25                  30
   Leu Glu Pro Glu Glu Pro Pro Thr Lys Tyr Gln Ile Ser Gln Pro Glu
           35                  40                  45
   Ala Tyr Val Val Ala Pro Gly Glu Ser Leu Glu Leu Gln Cys Met Leu
       50                  55                  60
   Lys Asp Ala Ala Val Ile Ser Trp Thr Lys Asp Gly Val His Leu Gly
   65                  70                  75                  80
   Pro Asn Asn Arg Thr Val Leu Ile Gly Glu Tyr Leu Gln Ile Lys Gly
                   85                  90                  95
   Ala Thr Pro Arg Asp Ser Gly Leu Tyr Ala Cys Thr Ala Ala Arg Thr
               100                 105                 110
   Val Asp Ser Glu Thr Trp Ile Phe Met Val Asn Val Thr Asp Ala Ile
           115                 120                 125
   Ser Ser Gly Asp Asp Glu Asp Asp Thr Asp Ser Ser Glu Asp Val Val
       130                 135                 140
   Ser Glu Asn Arg Ser Asn Gln Arg Ala Pro Tyr Trp Thr Asn Thr Glu
   145                 150                 155                 160
   Lys Met Glu Lys Arg Leu His Ala Cys Pro Ala Ala Asn Thr Val Lys
                   165                 170                 175
   Phe Arg Cys Pro Ala Gly Gly Asn Pro Thr Ser Thr Met Arg Trp Leu
               180                 185                 190
   Lys Asn Gly Lys Glu Phe Lys Gln Glu His Arg Ile Gly Gly Tyr Lys
           195                 200                 205
   Val Arg Asn Gln His Trp Ser Leu Ile Met Glu Ser Val Val Pro Ser
       210                 215                 220
   Asp Lys Gly Asn Tyr Thr Cys Leu Val Glu Asn Glu Tyr Gly Ser Ile
   225                 230                 235                 240
   Asn His Thr Tyr His Leu Asp Val Val Glu Arg Ser Pro His Arg Pro
                   245                 250                 255
   Ile Leu Gln Ala Gly Leu Pro Ala Asn Ala Ser Thr Val Val Gly Gly
               260                 265                 270
   Asp Val Glu Phe Val Cys Lys Val Tyr Ser Asp Ala Gln Pro His Ile
           275                 280                 285
   Gln Trp Ile Lys His Val Glu Lys Asn Gly Ser Lys Asn Gly Pro Asp
       290                 295                 300
   Gly Leu Pro Tyr Leu Lys Val Leu Lys Ala Ala Gly Val Asn Thr Thr
   305                 310                 315                 320
   Asp Lys Glu Ile Glu Val Leu Tyr Ile Arg Asn Val Thr Phe Glu Asp
                   325                 330                 335
   Ala Gly Glu Tyr Thr Cys Leu Ala Gly Asn Ser Ile Gly Ile Ser Phe
               340                 345                 350
   His Ser Ala Trp Leu Thr Val Leu Pro Ala Pro Val Arg Glu Lys Glu
           355                 360                 365
   Ile Thr Ala Ser Pro Asp Tyr Leu Glu Ile Ala Ile Tyr Cys Ile Gly
   370 375 380
   Val Phe Leu Ile Ala Cys Met Val Val Thr Val Ile Phe Cys Arg Met
   385 390 395 400
   Lys Thr Thr Thr Lys Lys Pro Asp Phe Ser Ser Gln Pro Ala Val His
   405 410 415
   Lys Leu Thr Lys Arg Ile Pro Leu Arg Arg Gln Val Thr Val Ser Ala
   420 425 430
   Glu Ser Ser Ser Ser Met Asn Ser Asn Thr Pro Leu Val Arg Ile Thr
   435 440 445
   Thr Arg Leu Ser Ser Thr Ala Asp Thr Pro Met Leu Ala Gly Val Ser
   450 455 460
   Glu Tyr Glu Leu Pro Glu Asp Pro Lys Trp Glu Phe Pro Arg Asp Lys
   465 470 475 480
   Leu Thr Leu Gly Lys Pro Leu Gly Glu Gly Cys Phe Gly Gln Val Val
   485 490 495
   Met Ala Glu Ala Val Gly Ile Asp Lys Asp Lys Pro Lys Glu Ala Val
   500 505 510
   Thr Val Ala Val Lys Met Leu Lys Asp Asp Ala Thr Glu Lys Asp Leu
   515 520 525
   Ser Asp Leu Val Ser Glu Met Glu Met Met Lys Met Ile Gly Lys His
   530 535 540
   Lys Asn Ile Ile Asn Leu Leu Gly Ala Cys Thr Gln Asp Gly Pro Leu
   545 550 555 560
   Tyr Val Ile Val Glu Tyr Ala Ser Lys Gly Asn Leu Arg Glu Tyr Leu
   565 570 575
   Arg Ala Arg Arg Pro Pro Gly Met Glu Tyr Ser Tyr Asp Ile Asn Arg
   580 585 590
   Val Pro Glu Glu Gln Met Thr Phe Lys Asp Leu Val Ser Cys Thr Tyr
   595 600 605
   Gln Leu Ala Arg Gly Met Glu Tyr Leu Ala Ser Gln Lys Cys Ile His
   610 615 620
   Arg Asp Leu Ala Ala Arg Asn Val Leu Val Thr Glu Asn Asn Val Met
   625                 630                 635                 640
   Lys Ile Ala Asp Phe Gly Leu Ala Arg Asp Ile Asn Asn Ile Asp Tyr
                   645                 650                 655
   Tyr Lys Lys Thr Thr Asn Gly Arg Leu Pro Val Lys Trp Met Ala Pro
               660                 665                 670
   Glu Ala Leu Phe Asp Arg Val Tyr Thr His Gln Ser Asp Val Trp Ser
           675                 680                 685
   Phe Gly Val Leu Met Trp Glu Ile Phe Thr Leu Gly Gly Ser Pro Tyr
       690                 695                 700
   Pro Gly Ile Pro Val Glu Glu Leu Phe Lys Leu Leu Lys Glu Gly His
   705                 710                 715                 720
   Arg Met Asp Lys Pro Thr Asn Cys Thr Asn Glu Leu Tyr Met Met Met
                   725                 730                 735
   Arg Asp Cys Trp His Ala Val Pro Ser Gln Arg Pro Thr Phe Lys Gln
               740                 745                 750
   Leu Val Glu Asp Leu Asp Arg Ile Leu Thr Leu Thr Thr Asn Glu Glu
           755                 760                 765
   Tyr Leu Asp Leu Thr Gln Pro Leu Glu Gln Tyr Ser Pro Ser Tyr Pro
       770                 775                 780
   Asp Thr Ser Ser Ser Cys Ser Ser Gly Asp Asp Ser Val Phe Ser Pro
   785                 790                 795                 800
   Asp Pro Met Pro Tyr Glu Pro Cys Leu Pro Gln Tyr Pro His Ile Asn
                   805                 810                 815
   Gly Ser Val Lys Thr
               820
<210> 9
   <211> 3037
   <212> DNA
   <213> Mus musculus
<400> 9
   ggcgagggga gagagccggg agaggcgagc ggcggcgcgg caggcgcgga acgggcgcac      60
   ggacgatcga acgcgcggcc gccagagctc cggcgcgggg gctgcctgtg tgttcctggc      120
   ccggcgtggc gactgctctc cgggctggcg ggggccgggc gtgagcccgg gcctcagcgt      180
   tcctgagcgc tgcgagtgtt cactactcgc cagcaaagtt tggagtaggc aacgccaagc 240
   tccagtcctt tcttctgctg ctgcccagat ccgagagcag ctccggtgtc atgtcctagc 300
   tgttctgcga tccccggcgc gcgtgaagcc tcggaacctt cgcgccggct gctacccaag 360
   gaatcgttct ctttttggag ttttcctccg agatcatcgc ctgctccatc ccgatccact 420
   ctgggctccg gcgcagaccg agcgcagagg agcgctgcca ttcaagtggc agccacagca 480
   gcagcagcag cagcagtggg agcaggaaca gcagtaacaa cagcaacagc agcacagccg 540
   cctcagagct ttggctcctg agccccctgt gggctgaagg cattgcaggt agcccatggt 600
   ctcagaagaa gtgtgcagat gggattaccg tccacgtgga gatatggaag aggaccaggg 660
   attggcactg tgaccatggt cagctggggg cgcttcatct gcctggtctt ggtcaccatg 720
   gcaaccttgt ccctggcccg gccctccttc agtttagttg aggataccac tttagaacca 780
   gaaggagcac cgtactggac caacaccgag aagatggaga agcggctcca cgctgtccct 840
   gccgccaaca ctgtgaagtt ccgctgtccg gctgggggga atccaacgcc cacaatgagg 900
   tggttaaaaa acgggaagga gtttaagcag gagcatcgca ttggaggcta taaggtacga 960
   aaccagcact ggagccttat tatggaaagt gtggtcccgt cagacaaagg caactacacc 1020
   tgcctggtgg agaatgaata cgggtccatc aaccacacct accacctcga tgtcgttgaa 1080
   cggtcaccac accggcccat cctccaagct ggactgcctg caaatgcctc cacggtggtc 1140
   ggaggggatg tggagtttgt ctgcaaggtt tacagcgatg cccagcccca catccagtgg 1200
   atcaagcacg tggaaaagaa cggcagtaaa tacgggcctg atgggctgcc ctacctcaag 1260
   gtcctgaagc actcggggat aaatagctcc aatgcagaag tgctggctct gttcaatgtg 1320
   acggagatgg atgctgggga atatatatgt aaggtctcca attatatagg gcaggccaac 1380
   cagtctgcct ggctcactgt cctgcccaaa cagcaagcgc ctgtgagaga gaaggagatc 1440
   acggcttccc cagattatct ggagatagct atttactgca taggggtctt cttaatcgcc 1500
   tgcatggtgg tgacagtcat cttttgccga atgaagacca cgaccaagaa gccagacttc 1560
   agcagccagc cagctgtgca caagctgacc aagcgcatcc ccctgcggag acaggtaaca 1620
   gtttcggccg agtccagctc ctccatgaac tccaacaccc cgctggtgag gataacaacg 1680
   cgtctgtcct caacagcgga caccccgatg ctagcagggg tctccgagta tgagttgcca 1740
   gaggatccaa agtgggaatt ccccagagat aagctgacgc tgggcaaacc cctgggggaa 1800
   ggttgcttcg ggcaagtagt catggctgaa gcagtgggaa tcgataaaga caaacccaag 1860
   gaggcggtca ccgtggcagt gaagatgttg aaagatgatg ccacagagaa ggacctgtct 1920
   gatctggtat cagagatgga gatgatgaag atgattggga aacataagaa cattatcaac 1980
   ctcctggggg cctgcacgca ggatggacct ctctacgtca tagttgaata tgcatcgaaa 2040
   ggcaacctcc gggaatacct ccgagcccgg aggccacctg gcatggagta ctcctatgac 2100
   attaaccgtg tccccgagga gcagatgacc ttcaaggact tggtgtcctg cacctaccag 2160
   ctggctagag gcatggagta cttggcttcc caaaaatgta tccatcgaga tttggctgcc     2220
   agaaacgtgt tggtaacaga aaacaatgtg atgaagatag cagactttgg cctggccagg     2280
   gatatcaaca acatagacta ctataaaaag accacaaatg ggcgacttcc agtcaagtgg     2340
   atggctcctg aagccctttt tgatagagtt tacactcatc agagcgatgt ctggtccttc     2400
   ggggtgttaa tgtgggagat ctttacttta gggggctcac cctacccagg gattcccgtg     2460
   gaggaacttt ttaagctgct caaagaggga cacaggatgg acaagcccac caactgcacc     2520
   aatgaactgt acatgatgat gagggattgc tggcatgctg taccctcaca gagacccaca     2580
   ttcaagcagt tggtcgaaga cttggatcga attctgactc tcacaaccaa tgaggaatac     2640
   ttggatctca cccagcctct cgaacagtat tctcctagtt accccgacac aaggagctct     2700
   tgttcttcag gggacgattc tgtgttttct ccagacccca tgccttatga accctgtctg     2760
   cctcagtatc cacacataaa cggcagtgtt aaaacatgag tgaatgtgtc ttcctgtccc     2820
   caaacaggac agcaccagga acctacttac actgagcaga gaggctgtct cagagcctgt     2880
   gacacgcctc cacttgtata tatggatcag aggagtaaat agtgggaagc atattgtcac     2940
   gtgtgtaaag atttatacag ttcggaaaca tgttacctaa ccaggaaagg aagactgttt     3000
   tcctgataag tggacagccg caagccacca tgccacc                              3037
<210> 10
   <211> 707
   <212> PRT
   <213> Mus musculus
<400> 10
   Met Val Ser Trp Gly Arg Phe Ile Cys Leu Val Leu Val Thr Met Ala
   1               5                   10                  15
   Thr Leu Ser Leu Ala Arg Pro Ser Phe Ser Leu Val Glu Asp Thr Thr
               20                  25                  30
   Leu Glu Pro Glu Gly Ala Pro Tyr Trp Thr Asn Thr Glu Lys Met Glu
           35                  40                  45
   Lys Arg Leu His Ala Val Pro Ala Ala Asn Thr Val Lys Phe Arg Cys
       50                  55                  60
   Pro Ala Gly Gly Asn Pro Thr Pro Thr Met Arg Trp Leu Lys Asn Gly
   65                  70                  75                  80
   Lys Glu Phe Lys Gln Glu His Arg Ile Gly Gly Tyr Lys Val Arg Asn
                   85                  90                  95
   Gln His Trp Ser Leu Ile Met Glu Ser Val Val Pro Ser Asp Lys Gly
               100                 105                 110
   Asn Tyr Thr Cys Leu Val Glu Asn Glu Tyr Gly Ser Ile Asn His Thr
           115                 120                 125
   Tyr His Leu Asp Val Val Glu Arg Ser Pro His Arg Pro Ile Leu Gln
       130                 135                 140
   Ala Gly Leu Pro Ala Asn Ala Ser Thr Val Val Gly Gly Asp Val Glu
   145                 150                 155                 160
   Phe Val Cys Lys Val Tyr Ser Asp Ala Gln Pro His Ile Gln Trp Ile
                   165                 170                 175
   Lys His Val Glu Lys Asn Gly Ser Lys Tyr Gly Pro Asp Gly Leu Pro
               180                 185                 190
   Tyr Leu Lys Val Leu Lys His Ser Gly Ile Asn Ser Ser Asn Ala Glu
           195                 200                 205
   Val Leu Ala Leu Phe Asn Val Thr Glu Met Asp Ala Gly Glu Tyr Ile
       210                 215                 220
   Cys Lys Val Ser Asn Tyr Ile Gly Gln Ala Asn Gln Ser Ala Trp Leu
   225                 230                 235                 240
   Thr Val Leu Pro Lys Gln Gln Ala Pro Val Arg Glu Lys Glu Ile Thr
                   245                 250                 255
   Ala Ser Pro Asp Tyr Leu Glu Ile Ala Ile Tyr Cys Ile Gly Val Phe
               260                 265                 270
   Leu Ile Ala Cys Met Val Val Thr Val Ile Phe Cys Arg Met Lys Thr
           275                 280                 285
   Thr Thr Lys Lys Pro Asp Phe Ser Ser Gln Pro Ala Val His Lys Leu
       290                 295                 300
   Thr Lys Arg Ile Pro Leu Arg Arg Gln Val Thr Val Ser Ala Glu Ser
   305                 310                 315                 320
   Ser Ser Ser Met Asn Ser Asn Thr Pro Leu Val Arg Ile Thr Thr Arg
                   325                 330                 335
   Leu Ser Ser Thr Ala Asp Thr Pro Met Leu Ala Gly Val Ser Glu Tyr
               340                 345                 350
   Glu Leu Pro Glu Asp Pro Lys Trp Glu Phe Pro Arg Asp Lys Leu Thr
           355                 360                 365
   Leu Gly Lys Pro Leu Gly Glu Gly Cys Phe Gly Gln Val Val Met Ala
       370                 375                 380
   Glu Ala Val Gly Ile Asp Lys Asp Lys Pro Lys Glu Ala Val Thr Val
   385                 390                 395                 400
   Ala Val Lys Met Leu Lys Asp Asp Ala Thr Glu Lys Asp Leu Ser Asp
                   405                 410                 415
   Leu Val Ser Glu Met Glu Met Met Lys Met Ile Gly Lys His Lys Asn
               420                 425                 430
   Ile Ile Asn Leu Leu Gly Ala Cys Thr Gln Asp Gly Pro Leu Tyr Val
           435                 440                 445
   Ile Val Glu Tyr Ala Ser Lys Gly Asn Leu Arg Glu Tyr Leu Arg Ala
       450                 455                 460
   Arg Arg Pro Pro Gly Met Glu Tyr Ser Tyr Asp Ile Asn Arg Val Pro
   465                 470                 475                 480
   Glu Glu Gln Met Thr Phe Lys Asp Leu Val Ser Cys Thr Tyr Gln Leu
                   485                 490                 495
   Ala Arg Gly Met Glu Tyr Leu Ala Ser Gln Lys Cys Ile His Arg Asp
               500                 505                 510
   Leu Ala Ala Arg Asn Val Leu Val Thr Glu Asn Asn Val Met Lys Ile
           515                 520                 525
   Ala Asp Phe Gly Leu Ala Arg Asp Ile Asn Asn Ile Asp Tyr Tyr Lys
       530                 535                 540
   Lys Thr Thr Asn Gly Arg Leu Pro Val Lys Trp Met Ala Pro Glu Ala
   545                 550                 555                 560
   Leu Phe Asp Arg Val Tyr Thr His Gln Ser Asp Val Trp Ser Phe Gly
                   565                 570                 575
   Val Leu Met Trp Glu Ile Phe Thr Leu Gly Gly Ser Pro Tyr Pro Gly
               580                 585                 590
   Ile Pro Val Glu Glu Leu Phe Lys Leu Leu Lys Glu Gly His Arg Met
           595                 600                 605
   Asp Lys Pro Thr Asn Cys Thr Asn Glu Leu Tyr Met Met Met Arg Asp
       610                 615                 620
   Cys Trp His Ala Val Pro Ser Gln Arg Pro Thr Phe Lys Gln Leu Val
   625                 630                 635                 640
   Glu Asp Leu Asp Arg Ile Leu Thr Leu Thr Thr Asn Glu Glu Tyr Leu
                   645                 650                 655
   Asp Leu Thr Gln Pro Leu Glu Gln Tyr Ser Pro Ser Tyr Pro Asp Thr
               660                 665                 670
   Arg Ser Ser Cys Ser Ser Gly Asp Asp Ser Val Phe Ser Pro Asp Pro
           675                 680                 685
   Met Pro Tyr Glu Pro Cys Leu Pro Gln Tyr Pro His Ile Asn Gly Ser
       690                 695                 700
   Val Lys Thr
   705
<210> 11
   <211> 3344
   <212> DNA
   <213> Xenopus tropicalis
<400> 11
   agagcaggga agtacagcgc tgcgctacaa gaactgagca aacgagcaga aaagtacaca 60
   ttcctgatcc ttcggtcttt ccaaaagtcc ccaatggggt cacacaggcc atggttactt 120
   ggtgctgtgg tgctgctggc actccttcag gtacagggag gactggcaga atggacacag 180
   tgtcaaatgg gattttccaa ggaaaggtac agcttttcgg tacctaagaa cttggagaca 240
   gacaaagcac tgggtagagt gatctttaac agctgtgagg gaccagtgag aattcagttt 300
   gcctctaaag atcctaattt tgaaattcac aaagatggca cagtttatgt taagaatcct 360
   accaagatga aagacaacag aaaaacattc cgtgtcctgg cttgggagaa tcaaggtcat 420
   gtatactcta ccagtgtaac cttgaaaggg gaagggcatc accataagca ggacatttct 480
   tctgtgaaac attcccacca cccaaaatct gagactggtt taaaaagaca aaaaagagac 540
   tgggtgattc caccaatcgt aacatctgag aatgaaaagg gcccatttcc caaacggctt 600
   gtgcagatca agtccagtaa tgcaaaggaa atcaaggttt tttacagtat cacaggccag 660
   ggtgccgata cccctccaga aggagtgttc actattggac gggaggatgg atggctaaat 720
   gtgacacgac ctttggacag agaagccatt gatagttaca ctcttttttc tcatgctgtg 780
   tcagtaaatg ggcaaaatgt ggaagatccc atggaaatcc aaattaaagt acaagatcag 840
   aatgataatg acccagtttt cacacaggag gtctttgaag gctatgtgcc tgaagggtct 900
   aagccaggta cgcccgtcat gactgtatct gcaacagatg ccgatgatgc tatagacatg 960
   tacaatggtg tgattactta ctccattctc aaccaagacc ctaaagagcc caacaatcaa 1020
   atgttcacta ttgattccca gtctgggttg atcagcgtag ttacaactgg attagacaga 1080
   gagaaaatac cagtgtacac actgactatt caagctgcag atggagaatt tgggaaagat 1140
   cgcacaacaa ctgcaaaagc tgtgatcatt gtgacagaca ccaatgataa ccctcctgtg 1200
   tttaacccaa cgcaatacat tgcagaggtt cctgaaaatg aagttggata tgaggttgca 1260
   cgtcttacgg taacagatgc agatattgaa gggtcagatg cctggaatgc tgtgtacaag 1320
   atcattaaag gaaatgaggc tggctttttc agcatccaaa cagatattga caacattggg 1380
   ctactgaaaa cagtgaaggg tctggactat gagctgaaga agcagtatat tctgtcagtc 1440
   attgtgacaa acaaagctaa cttttctgtt ccactacaaa cttcaactgc aacggtcact 1500
   gtaactgtca cagatgtgaa tgaggcccca gtatttgtac cagtgttgaa agacgtgtct 1560
   gtgccagagg atctgcccag tggccaagtt gttgctacct ataccgcaca ggatccagac 1620
   aaggaacaga accagaaaat aagttacttc attggaaatg acccagcagg gtgggtgtct 1680
   gtgaacagag ataatgggat tgtcactgga aatggaaact tggatcggga atcaaagttt 1740
   gtgctaaaca acacctacaa agtcataatc ttggccgctg acagtggcac tccttctgcc 1800
   actgggactg gaacccttgt gcttaatctc attgatgtta atgataatgg cccatttttg 1860
   gatccccaac aaaatagttt ctgccagaag gatccaggct ttcgtgtatt taatatcatt 1920
   gacaaagatc tttaccctaa cacataccca tatacagtag acctgactgg tgaatccaat 1980
   gaaaactgga ctgctacagt gacagaacag agtttacttg agctgagacc taaaaaggaa 2040
   ctggatattg gacgatacga agttttgatc tcattgagag acaatcaggg actgacagat 2100
   gtgacaaagc tacagattac aatctgtcaa tgtaatggtg accaaatgca atgtgaggaa 2160
   aaggctgctc aagcaggagg tttggggata tcagccatag ttggaatcct tggagggatc 2220
   ctagcgcttc ttttattgtt gttgctgctc ttactgtttg tacgacgaaa gaaagtggta 2280
   aaagaacctt tattaccacc agaagatgag actcgggaca atgtattttt ctatgatgaa 2340
   gaaggcggtg gtgaggaaga ccaggatttt gatctaagcc agcttcaccg tggtctagat 2400
   gctcgtccag atataatccg taatgatgtc gttccagttt tagctgctcc ccagtatcga 2460
   ccccgtcctg ccaatccaga tgaaattgga aatttcattg atgagaactt gcatgcagct 2520
   gacaatgacc ccactgctcc tccatacgac tcgctccttg tgttcgatta cgaaggcagt 2580
   ggctctgagg ccgcatcact cagctctctt aactcttcca actctgattt agatcaggat 2640
   tacagtgctt tgaataactg gggacctcgt ttcaccaaac tggcagaaat gtatggagga 2700
   gatgaggatt agaatgtgca ctgcaatacc attttgattc taaacagtaa actaaaaacc 2760
   ataattgtgt atgcagtctt tggaattcac tttgttttct cctgctctta aaacagagat 2820
   aaggactgct caaaagttac tcctcctgct tttgtaaaat cgttcaaaaa tattttatgt 2880
   atatgtatat atgaaaaaat cgtatttttt gtactatttg tgttcttata tccctgcaat 2940
   ttgtaataca agaggatctt tatctgctta attataaata taaaatgccc gatatgattc 3000
   actatgattt taatgtgttg agaaatcttt ttttaaaaag gtttccagac acctgacgct     3060
   tggaagggaa ttccataaaa atataattga attgggggga gattgtgttt tgccatggtc     3120
   tgatatacat tttcatatat atacatatga tcattcacag agtacagtca acatttggaa     3180
   tttgatgagc ttgctggtcg aactgaaaaa aaaatgtatt atagctgggg taaaaattaa     3240
   tgtatgagct aaatggggca caattttgat atctctgcat ttgtatttta cttggcatgt     3300
   atacttttgt aataaaataa agatatacat taatatacaa cata                      3344
<210> 12
   <211> 872
   <212> PRT
   <213> Xenopus tropicalis
<400> 12
   Met Gly Ser His Arg Pro Trp Leu Leu Gly Ala Val Val Leu Leu Ala
   1               5                   10                  15
   Leu Leu Gln Val Gln Gly Gly Leu Ala Glu Trp Thr Gln Cys Gln Met
               20                  25                  30
   Gly Phe Ser Lys Glu Arg Tyr Ser Phe Ser Val Pro Lys Asn Leu Glu
           35                  40                  45
   Thr Asp Lys Ala Leu Gly Arg Val Ile Phe Asn Ser Cys Glu Gly Pro
       50                  55                  60
   Val Arg Ile Gln Phe Ala Ser Lys Asp Pro Asn Phe Glu Ile His Lys
   65                  70                  75                  80
   Asp Gly Thr Val Tyr Val Lys Asn Pro Thr Lys Met Lys Asp Asn Arg
                   85                  90                  95
   Lys Thr Phe Arg Val Leu Ala Trp Glu Asn Gln Gly His Val Tyr Ser
               100                 105                 110
   Thr Ser Val Thr Leu Lys Gly Glu Gly His His His Lys Gln Asp Ile
           115                 120                 125
   Ser Ser Val Lys His Ser His His Pro Lys Ser Glu Thr Gly Leu Lys
       130                 135                 140
   Arg Gln Lys Arg Asp Trp Val Ile Pro Pro Ile Val Thr Ser Glu Asn
   145                 150                 155                 160
   Glu Lys Gly Pro Phe Pro Lys Arg Leu Val Gln Ile Lys Ser Ser Asn
                   165                 170                 175
   Ala Lys Glu Ile Lys Val Phe Tyr Ser Ile Thr Gly Gln Gly Ala Asp
   180 185 190
   Thr Pro Pro Glu Gly Val Phe Thr Ile Gly Arg Glu Asp Gly Trp Leu
   195 200 205
   Asn Val Thr Arg Pro Leu Asp Arg Glu Ala Ile Asp Ser Tyr Thr Leu
   210 215 220
   Phe Ser His Ala Val Ser Val Asn Gly Gln Asn Val Glu Asp Pro Met
   225 230 235 240
   Glu Ile Gln Ile Lys Val Gln Asp Gln Asn Asp Asn Asp Pro Val Phe
   245 250 255
   Thr Gln Glu Val Phe Glu Gly Tyr Val Pro Glu Gly Ser Lys Pro Gly
   260 265 270
   Thr Pro Val Met Thr Val Ser Ala Thr Asp Ala Asp Asp Ala Ile Asp
   275 280 285
   Met Tyr Asn Gly Val Ile Thr Tyr Ser Ile Leu Asn Gln Asp Pro Lys
   290 295 300
   Glu Pro Asn Asn Gln Met Phe Thr Ile Asp Ser Gln Ser Gly Leu Ile
   305 310 315 320
   Ser Val Val Thr Thr Gly Leu Asp Arg Glu Lys Ile Pro Val Tyr Thr
   325 330 335
   Leu Thr Ile Gln Ala Ala Asp Gly Glu Phe Gly Lys Asp Arg Thr Thr
   340 345 350
   Thr Ala Lys Ala Val Ile Ile Val Thr Asp Thr Asn Asp Asn Pro Pro
   355 360 365
   Val Phe Asn Pro Thr Gln Tyr Ile Ala Glu Val Pro Glu Asn Glu Val
   370 375 380
   Gly Tyr Glu Val Ala Arg Leu Thr Val Thr Asp Ala Asp Ile Glu Gly
   385 390 395 400
   Ser Asp Ala Trp Asn Ala Val Tyr Lys Ile Ile Lys Gly Asn Glu Ala
   405 410 415
   Gly Phe Phe Ser Ile Gln Thr Asp Ile Asp Asn Ile Gly Leu Leu Lys
   420 425 430
   Thr Val Lys Gly Leu Asp Tyr Glu Leu Lys Lys Gln Tyr Ile Leu Ser
   435 440 445
   Val Ile Val Thr Asn Lys Ala Asn Phe Ser Val Pro Leu Gln Thr Ser
       450                 455                 460
   Thr Ala Thr Val Thr Val Thr Val Thr Asp Val Asn Glu Ala Pro Val
   465                 470                 475                 480
   Phe Val Pro Val Leu Lys Asp Val Ser Val Pro Glu Asp Leu Pro Ser
                   485                 490                 495
   Gly Gln Val Val Ala Thr Tyr Thr Ala Gln Asp Pro Asp Lys Glu Gln
               500                 505                 510
   Asn Gln Lys Ile Ser Tyr Phe Ile Gly Asn Asp Pro Ala Gly Trp Val
           515                 520                 525
   Ser Val Asn Arg Asp Asn Gly Ile Val Thr Gly Asn Gly Asn Leu Asp
       530                 535                 540
   Arg Glu Ser Lys Phe Val Leu Asn Asn Thr Tyr Lys Val Ile Ile Leu
   545                 550                 555                 560
   Ala Ala Asp Ser Gly Thr Pro Ser Ala Thr Gly Thr Gly Thr Leu Val
                   565                 570                 575
   Leu Asn Leu Ile Asp Val Asn Asp Asn Gly Pro Phe Leu Asp Pro Gln
               580                 585                 590
   Gln Asn Ser Phe Cys Gln Lys Asp Pro Gly Phe Arg Val Phe Asn Ile
           595                 600                 605
   Ile Asp Lys Asp Leu Tyr Pro Asn Thr Tyr Pro Tyr Thr Val Asp Leu
       610                 615                 620
   Thr Gly Glu Ser Asn Glu Asn Trp Thr Ala Thr Val Thr Glu Gln Ser
   625                 630                 635                 640
   Leu Leu Glu Leu Arg Pro Lys Lys Glu Leu Asp Ile Gly Arg Tyr Glu
                   645                 650                 655
   Val Leu Ile Ser Leu Arg Asp Asn Gln Gly Leu Thr Asp Val Thr Lys
               660                 665                 670
   Leu Gln Ile Thr Ile Cys Gln Cys Asn Gly Asp Gln Met Gln Cys Glu
           675                 680                 685
   Glu Lys Ala Ala Gln Ala Gly Gly Leu Gly Ile Ser Ala Ile Val Gly
       690                 695                 700
   Ile Leu Gly Gly Ile Leu Ala Leu Leu Leu Leu Leu Leu Leu Leu Leu
   705                 710                 715                 720
   Leu Phe Val Arg Arg Lys Lys Val Val Lys Glu Pro Leu Leu Pro Pro
                   725                 730                 735
   Glu Asp Glu Thr Arg Asp Asn Val Phe Phe Tyr Asp Glu Glu Gly Gly
               740                 745                 750
   Gly Glu Glu Asp Gln Asp Phe Asp Leu Ser Gln Leu His Arg Gly Leu
           755                 760                 765
   Asp Ala Arg Pro Asp Ile Ile Arg Asn Asp Val Val Pro Val Leu Ala
       770                 775                 780
   Ala Pro Gln Tyr Arg Pro Arg Pro Ala Asn Pro Asp Glu Ile Gly Asn
   785                 790                 795                 800
   Phe Ile Asp Glu Asn Leu His Ala Ala Asp Asn Asp Pro Thr Ala Pro
                   805                 810                 815
   Pro Tyr Asp Ser Leu Leu Val Phe Asp Tyr Glu Gly Ser Gly Ser Glu
               820                 825                 830
   Ala Ala Ser Leu Ser Ser Leu Asn Ser Ser Asn Ser Asp Leu Asp Gln
           835                 840                 845
   Asp Tyr Ser Ala Leu Asn Asn Trp Gly Pro Arg Phe Thr Lys Leu Ala
       850                 855                 860
   Glu Met Tyr Gly Gly Asp Glu Asp
   865                 870
<210> 13
   <211> 1862
   <212> DNA
   <213> Homo sapiens
<400> 13
   gtccccgcgc cagagacgca gccgcgctcc caccacccac acccaccgcg ccctcgttcg      60
   cctcttctcc gggagccagt ccgcgccacc gccgccgccc aggccatcgc caccctccgc     120
   agccatgtcc accaggtccg tgtcctcgtc ctcctaccgc aggatgttcg gcggcccggg     180
   caccgcgagc cggccgagct ccagccggag ctacgtgact acgtccaccc gcacctacag     240
   cctgggcagc gcgctgcgcc ccagcaccag ccgcagcctc tacgcctcgt ccccgggcgg     300
   cgtgtatgcc acgcgctcct ctgccgtgcg cctgcggagc agcgtgcccg gggtgcggct     360
   cctgcaggac tcggtggact tctcgctggc cgacgccatc aacaccgagt tcaagaacac     420
   ccgcaccaac gagaaggtgg agctgcagga gctgaatgac cgcttcgcca actacatcga      480
   caaggtgcgc ttcctggagc agcagaataa gatcctgctg gccgagctcg agcagctcaa      540
   gggccaaggc aagtcgcgcc tgggggacct ctacgaggag gagatgcggg agctgcgccg      600
   gcaggtggac cagctaacca acgacaaagc ccgcgtcgag gtggagcgcg acaacctggc      660
   cgaggacatc atgcgcctcc gggagaaatt gcaggaggag atgcttcaga gagaggaagc      720
   cgaaaacacc ctgcaatctt tcagacagga tgttgacaat gcgtctctgg cacgtcttga      780
   ccttgaacgc aaagtggaat ctttgcaaga agagattgcc tttttgaaga aactccacga      840
   agaggaaatc caggagctgc aggctcagat tcaggaacag catgtccaaa tcgatgtgga      900
   tgtttccaag cctgacctca cggctgccct gcgtgacgta cgtcagcaat atgaaagtgt      960
   ggctgccaag aacctgcagg aggcagaaga atggtacaaa tccaagtttg ctgacctctc     1020
   tgaggctgcc aaccggaaca atgacgccct gcgccaggca aagcaggagt ccactgagta     1080
   ccggagacag gtgcagtccc tcacctgtga agtggatgcc cttaaaggaa ccaatgagtc     1140
   cctggaacgc cagatgcgtg aaatggaaga gaactttgcc gttgaagctg ctaactacca     1200
   agacactatt ggccgcctgc aggatgagat tcagaatatg aaggaggaaa tggctcgtca     1260
   ccttcgtgaa taccaagacc tgctcaatgt taagatggcc cttgacattg agattgccac     1320
   ctacaggaag ctgctggaag gcgaggagag caggatttct ctgcctcttc caaacttttc     1380
   ctccctgaac ctgagggaaa ctaatctgga ttcactccct ctggttgata cccactcaaa     1440
   aaggacactt ctgattaaga cggttgaaac tagagatgga caggttatca acgaaacttc     1500
   tcagcatcac gatgaccttg aataaaaatt gcacacactc agtgcagcaa tatattacca     1560
   gcaagaataa aaaagaaatc catatcttaa agaaacagct ttcaagtgcc tttctgcagt     1620
   ttttcaggag cgcaagatag atttggaata ggaataagct ctagttctta acaaccgaca     1680
   ctcctacaag atttagaaaa aagtttacaa cataatctag tttacagaaa aatcttgtgc     1740
   tagaatactt tttaaaaggt attttgaata ccattaaaac tgcttttttt tttccagcaa     1800
   gtatccaacc aacttggttc tgcttcaata aatctttgga aaaactcaaa aaaaaaaaaa     1860
   aa                                                                    1862
<210> 14
   <211> 466
   <212> PRT
   <213> Homo sapiens
<400> 14
   Met Ser Thr Arg Ser Val Ser Ser Ser Ser Tyr Arg Arg Met Phe Gly
   1               5                   10                  15
   Gly Pro Gly Thr Ala Ser Arg Pro Ser Ser Ser Arg Ser Tyr Val Thr
               20                  25                  30
   Thr Ser Thr Arg Thr Tyr Ser Leu Gly Ser Ala Leu Arg Pro Ser Thr
           35                  40                  45
   Ser Arg Ser Leu Tyr Ala Ser Ser Pro Gly Gly Val Tyr Ala Thr Arg
       50                  55                  60
   Ser Ser Ala Val Arg Leu Arg Ser Ser Val Pro Gly Val Arg Leu Leu
   65                  70                  75                  80
   Gln Asp Ser Val Asp Phe Ser Leu Ala Asp Ala Ile Asn Thr Glu Phe
                   85                  90                  95
   Lys Asn Thr Arg Thr Asn Glu Lys Val Glu Leu Gln Glu Leu Asn Asp
               100                 105                 110
   Arg Phe Ala Asn Tyr Ile Asp Lys Val Arg Phe Leu Glu Gln Gln Asn
           115                 120                 125
   Lys Ile Leu Leu Ala Glu Leu Glu Gln Leu Lys Gly Gln Gly Lys Ser
       130                 135                 140
   Arg Leu Gly Asp Leu Tyr Glu Glu Glu Met Arg Glu Leu Arg Arg Gln
   145                 150                 155                 160
   Val Asp Gln Leu Thr Asn Asp Lys Ala Arg Val Glu Val Glu Arg Asp
                   165                 170                 175
   Asn Leu Ala Glu Asp Ile Met Arg Leu Arg Glu Lys Leu Gln Glu Glu
               180                 185                 190
   Met Leu Gln Arg Glu Glu Ala Glu Asn Thr Leu Gln Ser Phe Arg Gln
           195                 200                 205
   Asp Val Asp Asn Ala Ser Leu Ala Arg Leu Asp Leu Glu Arg Lys Val
       210                 215                 220
   Glu Ser Leu Gln Glu Glu Ile Ala Phe Leu Lys Lys Leu His Glu Glu
   225                 230                 235                 240
   Glu Ile Gln Glu Leu Gln Ala Gln Ile Gln Glu Gln His Val Gln Ile
                   245                 250                 255
   Asp Val Asp Val Ser Lys Pro Asp Leu Thr Ala Ala Leu Arg Asp Val
               260                 265                 270
   Arg Gln Gln Tyr Glu Ser Val Ala Ala Lys Asn Leu Gln Glu Ala Glu
           275                 280                 285
   Glu Trp Tyr Lys Ser Lys Phe Ala Asp Leu Ser Glu Ala Ala Asn Arg
       290                 295                 300
   Asn Asn Asp Ala Leu Arg Gln Ala Lys Gln Glu Ser Thr Glu Tyr Arg
   305                 310                 315                 320
   Arg Gln Val Gln Ser Leu Thr Cys Glu Val Asp Ala Leu Lys Gly Thr
                   325                 330                 335
   Asn Glu Ser Leu Glu Arg Gln Met Arg Glu Met Glu Glu Asn Phe Ala
               340                 345                 350
   Val Glu Ala Ala Asn Tyr Gln Asp Thr Ile Gly Arg Leu Gln Asp Glu
           355                 360                 365
   Ile Gln Asn Met Lys Glu Glu Met Ala Arg His Leu Arg Glu Tyr Gln
       370                 375                 380
   Asp Leu Leu Asn Val Lys Met Ala Leu Asp Ile Glu Ile Ala Thr Tyr
   385                 390                 395                 400
   Arg Lys Leu Leu Glu Gly Glu Glu Ser Arg Ile Ser Leu Pro Leu Pro
                   405                 410                 415
   Asn Phe Ser Ser Leu Asn Leu Arg Glu Thr Asn Leu Asp Ser Leu Pro
               420                 425                 430
   Leu Val Asp Thr His Ser Lys Arg Thr Leu Leu Ile Lys Thr Val Glu
           435                 440                 445
   Thr Arg Asp Gly Gln Val Ile Asn Glu Thr Ser Gln His His Asp Asp
       450                 455                 460
   Leu Glu
   465
<210> 15
   <211> 2721
   <212> DNA
   <213> Homo sapiens
<400> 15
   atgtgccgga tagcgggagc gctgcggacc ctgctgccgc tgctggcggc cctgcttcag      60
   gcgtctgtag aggcttctgg tgaaatcgca ttatgcaaga ctggatttcc tgaagatgtt     120
   tacagtgcag tcttatcgaa ggatgtgcat gaaggacagc ctcttctcaa tgtgaagttt     180
   agcaactgca atggaaaaag aaaagtacaa tatgagagca gtgagcctgc agattttaag     240
   gtggatgaag atggcatggt gtatgccgtg agaagctttc cactctcttc tgagcatgcc     300
   aagttcctga tatatgccca agacaaagag acccaggaaa agtggcaagt ggcagtaaaa     360
   ttgagcctga agccaacctt aactgaggag tcagtgaagg agtcagcaga agttgaagaa 420
   atagtgttcc caagacaatt cagtaagcac agtggccacc tacaaaggca gaagagagac 480
   tgggtcatcc ctccaatcaa cttgccagaa aactccaggg gaccttttcc tcaagagctt 540
   gtcaggatca ggtctgatag agataaaaac ctttcactgc ggtacagtgt aactgggcca 600
   ggagctgacc agcctccaac tggtatcttc attatcaacc ccatctcggg tcagctgtcg 660
   gtgacaaagc ccctggatcg cgagcagata gcccggtttc atttgagggc acatgcagta 720
   gatattaatg gaaatcaagt ggagaacccc attgacattg tcatcaatgt tattgacatg 780
   aatgacaaca gacctgagtt cttacaccag gtttggaatg ggacagttcc tgagggatca 840
   aagcctggaa catatgtgat gaccgtaaca gcaattgatg ctgacgatcc caatgccctc 900
   aatgggatgt tgaggtacag aatcgtgtct caggctccaa gcaccccttc acccaacatg 960
   tttacaatca acaatgagac tggtgacatc atcacagtgg cagctggact tgatcgagaa 1020
   aaagtgcaac agtatacgtt aataattcaa gctacagaca tggaaggcaa tcccacatat 1080
   ggcctttcaa acacagccac ggccgtcatc acagtgacag atgtcaatga caatcctcca 1140
   gagtttactg ccatgacgtt ttatggtgaa gttcctgaga acagggtaga catcatagta 1200
   gctaatctaa ctgtgaccga taaggatcaa ccccatacac cagcctggaa cgcagtgtac 1260
   agaatcagtg gcggagatcc tactggacgg ttcgccatcc agaccgaccc aaacagcaac 1320
   gacgggttag tcaccgtggt caaaccaatc gactttgaaa caaataggat gtttgtcctt 1380
   actgttgctg cagaaaatca agtgccatta gccaagggaa ttcagcaccc ccctcagtca 1440
   actgcaaccg tgtctgttac agttattgac gtaaatgaaa acccttattt tgcccccaat 1500
   cctaagatca ttcgccaaga agaagggctt catgccggta ccatgttgac aacattcact 1560
   gctcaggacc cagatcgata tatgcagcaa aatattagat acactaaatt atctgatcct 1620
   gccaattggc taaaaataga tcctgtgaat ggacaaataa ctacaattgc tgttttggac 1680
   cgagaatcac caaatgtgaa aaacaatata tataatgcta ctttccttgc ttctgacaat 1740
   ggaattcctc ctatgagtgg aacaggaacg ctgcagatct atttacttga tattaatgac 1800
   aatgcccctc aagtgttacc tcaagaggca gagacttgcg aaactccaga ccccaattca 1860
   attaatatta cagcacttga ttatgacatt gatccaaatg ctggaccatt tgcttttgat 1920
   cttcctttat ctccagtgac tattaagaga aattggacca tcactcggct taatggtgat 1980
   tttgctcagc ttaatttaaa gataaaattt cttgaagctg gtatctatga agttcccatc 2040
   ataatcacag attcgggtaa tcctcccaaa tcaaatattt ccatcctgcg tgtgaaggtt 2100
   tgccagtgtg actccaacgg ggactgcaca gatgtggaca ggattgtggg tgcggggctt 2160
   ggcaccggtg ccatcattgc catcctgctc tgcatcatca tcctgcttat ccttgtgctg 2220
   atgtttgtgg tatggatgaa acgccgggat aaagaacgcc aggccaaaca acttttaatt 2280
   gatccagaag atgatgtaag agataatatt ttaaaatatg atgaagaagg tggaggagaa     2340
   gaagaccagg actatgactt gagccagctg cagcagcctg acactgtgga gcctgatgcc     2400
   atcaagcctg tgggaatccg acgaatggat gaaagaccca tccacgccga gccccagtat     2460
   ccggtccgat ctgcagcccc acaccctgga gacattgggg acttcattaa tgagggcctt     2520
   aaagcggctg acaatgaccc cacagctcca ccatatgact ccctgttagt gtttgactat     2580
   gaaggcagtg gctccactgc tgggtccttg agctccctta attcctcaag tagtggtggt     2640
   gagcaggact atgattacct gaacgactgg gggccacggt tcaagaaact tgctgacatg     2700
   tatggtggag gtgatgactg a                                               2721
<210> 16
   <211> 906
   <212> PRT
   <213> Homo sapiens
<400> 16
<210> 17
   <211> 2391
   <212> DNA
   <213> Homo sapiens
<400> 17
   atgaaggaga actactgttt acaagccgcc ctggtgtgcc tgggcatgct gtgccacagc 60
   catgcctttg ccccagagcg gcgggggcac ctgcggccct ccttccatgg gcaccatgag 120
   aagggcaagg aggggcaggt gctacagcgc tccaagcgtg gctgggtctg gaaccagttc 180
   ttcgtgatag aggagtacac cgggcctgac cccgtgcttg tgggcaggct tcattcagat 240
   attgactctg gtgatgggaa cattaaatac attctctcag gggaaggagc tggaaccatt 300
   tttgtgattg atgacaaatc agggaacatt catgccacca agacgttgga tcgagaagag 360
   agagcccagt acacgttgat ggctcaggcg gtggacaggg acaccaatcg gccactggag 420
   ccaccgtcgg aattcattgt caaggtccag gacattaatg acaaccctcc ggagttcctg 480
   cacgagacct atcatgccaa cgtgcctgag aggtccaatg tgggaacgtc agtaatccag 540
   gtgacagctt cagatgcaga tgaccccact tatggaaata gcgccaagtt agtgtacagt 600
   atcctcgaag gacaacccta tttttcggtg gaagcacaga caggtatcat cagaacagcc 660
   ctacccaaca tggacaggga ggccaaggag gagtaccacg tggtgatcca ggccaaggac 720
   atgggtggac atatgggcgg actctcaggg acaaccaaag tgacgatcac actgaccgat 780
   gtcaatgaca acccaccaaa gtttccgcag agcgtatacc agatgtctgt gtcagaagca 840
   gccgtccctg gggaggaagt aggaagagtg aaagctaaag atccagacat tggagaaaat 900
   ggcttagtca catacaatat tgttgatgga gatggtatgg aatcgtttga aatcacaacg 960
   gactatgaaa cacaggaggg ggtgataaag ctgaaaaagc ctgtagattt tgaaaccaaa 1020
   agagcctata gcttgaaggt agaggcagcc aacgtgcaca tcgacccgaa gtttatcagc 1080
   aatggccctt tcaaggacac tgtgaccgtc aagatctcag tagaagatgc tgatgagccc 1140
   cctatgttct tggccccaag ttacatccac gaagtccaag aaaatgcagc tgctggcacc 1200
   gtggttggga gagtgcatgc caaagaccct gatgctgcca acagcccgat aaggtattcc 1260
   atcgatcgtc acactgacct cgacagattt ttcactatta atccagagga tggttttatt 1320
   aaaactacaa aacctctgga tagagaggaa acagcctggc tcaacatcac tgtctttgca 1380
   gcagaaatcc acaatcggca tcaggaagcc aaagtcccag tggccattag ggtccttgat 1440
   gtcaacgata atgctcccaa gtttgctgcc ccttatgaag gtttcatctg tgagagtgat 1500
   cagaccaagc cactttccaa ccagccaatt gttacaatta gtgcagatga caaggatgac 1560
   acggccaatg gaccaagatt tatcttcagc ctaccccctg aaatcattca caatccaaat 1620
   ttcacagtca gagacaaccg agataacaca gcaggcgtgt acgcccggcg tggagggttc 1680
   agtcggcaga agcaggactt gtaccttctg cccatagtga tcagcgatgg cggcatcccg 1740
   cccatgagta gcaccaacac cctcaccatc aaagtctgcg ggtgcgacgt gaacggggca 1800
   ctgctctcct gcaacgcaga ggcctacatt ctgaacgccg gcctgagcac aggcgccctg 1860
   atcgccatcc tcgcctgcat cgtcattctc ctggtcattg tagtattgtt tgtgaccctg 1920
   agaaggcaaa agaaagaacc actcattgtc tttgaggaag aagatgtccg tgagaacatc 1980
   attacttatg atgatgaagg gggtggggaa gaagacacag aagcctttga tattgccacc 2040
   ctccagaatc ctgatggtat caatggattt atcccccgca aagacatcaa acctgagtat    2100
   cagtacatgc ctagacctgg gctccggcca gcgcccaaca gcgtggatgt cgatgacttc    2160
   atcaacacga gaatacagga ggcagacaat gaccccacgg ctcctcctta tgactccatt    2220
   caaatctacg gttatgaagg caggggctca gtggccgggt ccctgagctc cctagagtcg    2280
   gccaccacag attcagactt ggactatgat tatctacaga actggggacc tcgttttaag    2340
   aaactagcag atttgtatgg ttccaaagac acttttgatg acgattctta a            2391
<210> 18
   <211> 796
   <212> PRT
   <213> Homo sapiens
<400> 18
   Met Lys Glu Asn Tyr Cys Leu Gln Ala Ala Leu Val Cys Leu Gly Met
   1               5                   10                  15
   Leu Cys His Ser His Ala Phe Ala Pro Glu Arg Arg Gly His Leu Arg
               20                  25                  30
   Pro Ser Phe His Gly His His Glu Lys Gly Lys Glu Gly Gln Val Leu
           35                  40                  45
   Gln Arg Ser Lys Arg Gly Trp Val Trp Asn Gln Phe Phe Val Ile Glu
       50                  55                  60
   Glu Tyr Thr Gly Pro Asp Pro Val Leu Val Gly Arg Leu His Ser Asp
   65                  70                  75                  80
   Ile Asp Ser Gly Asp Gly Asn Ile Lys Tyr Ile Leu Ser Gly Glu Gly
                   85                  90                  95
   Ala Gly Thr Ile Phe Val Ile Asp Asp Lys Ser Gly Asn Ile His Ala
               100                 105                 110
   Thr Lys Thr Leu Asp Arg Glu Glu Arg Ala Gln Tyr Thr Leu Met Ala
           115                 120                 125
   Gln Ala Val Asp Arg Asp Thr Asn Arg Pro Leu Glu Pro Pro Ser Glu
       130                 135                 140
   Phe Ile Val Lys Val Gln Asp Ile Asn Asp Asn Pro Pro Glu Phe Leu
   145                 150                 155                 160
   His Glu Thr Tyr His Ala Asn Val Pro Glu Arg Ser Asn Val Gly Thr
                   165                 170                 175
   Ser Val Ile Gln Val Thr Ala Ser Asp Ala Asp Asp Pro Thr Tyr Gly
   180 185 190
   Asn Ser Ala Lys Leu Val Tyr Ser Ile Leu Glu Gly Gln Pro Tyr Phe
   195 200 205
   Ser Val Glu Ala Gln Thr Gly Ile Ile Arg Thr Ala Leu Pro Asn Met
   210 215 220
   Asp Arg Glu Ala Lys Glu Glu Tyr His Val Val Ile Gln Ala Lys Asp
   225 230 235 240
   Met Gly Gly His Met Gly Gly Leu Ser Gly Thr Thr Lys Val Thr Ile
   245 250 255
   Thr Leu Thr Asp Val Asn Asp Asn Pro Pro Lys Phe Pro Gln Ser Val
   260 265 270
   Tyr Gln Met Ser Val Ser Glu Ala Ala Val Pro Gly Glu Glu Val Gly
   275 280 285
   Arg Val Lys Ala Lys Asp Pro Asp Ile Gly Glu Asn Gly Leu Val Thr
   290 295 300
   Tyr Asn Ile Val Asp Gly Asp Gly Met Glu Ser Phe Glu Ile Thr Thr
   305 310 315 320
   Asp Tyr Glu Thr Gln Glu Gly Val Ile Lys Leu Lys Lys Pro Val Asp
   325 330 335
   Phe Glu Thr Lys Arg Ala Tyr Ser Leu Lys Val Glu Ala Ala Asn Val
   340 345 350
   His Ile Asp Pro Lys Phe Ile Ser Asn Gly Pro Phe Lys Asp Thr Val
   355 360 365
   Thr Val Lys Ile Ser Val Glu Asp Ala Asp Glu Pro Pro Met Phe Leu
   370 375 380
   Ala Pro Ser Tyr Ile His Glu Val Gln Glu Asn Ala Ala Ala Gly Thr
   385 390 395 400
   Val Val Gly Arg Val His Ala Lys Asp Pro Asp Ala Ala Asn Ser Pro
   405 410 415
   Ile Arg Tyr Ser Ile Asp Arg His Thr Asp Leu Asp Arg Phe Phe Thr
   420 425 430
   Ile Asn Pro Glu Asp Gly Phe Ile Lys Thr Thr Lys Pro Leu Asp Arg
           435                 440                 445
   Glu Glu Thr Ala Trp Leu Asn Ile Thr Val Phe Ala Ala Glu Ile His
       450                 455                 460
   Asn Arg His Gln Glu Ala Lys Val Pro Val Ala Ile Arg Val Leu Asp
   465                 470                 475                 480
   Val Asn Asp Asn Ala Pro Lys Phe Ala Ala Pro Tyr Glu Gly Phe Ile
                   485                 490                 495
   Cys Glu Ser Asp Gln Thr Lys Pro Leu Ser Asn Gln Pro Ile Val Thr
               500                 505                 510
   Ile Ser Ala Asp Asp Lys Asp Asp Thr Ala Asn Gly Pro Arg Phe Ile
           515                 520                 525
   Phe Ser Leu Pro Pro Glu Ile Ile His Asn Pro Asn Phe Thr Val Arg
       530                 535                 540
   Asp Asn Arg Asp Asn Thr Ala Gly Val Tyr Ala Arg Arg Gly Gly Phe
   545                 550                 555                 560
   Ser Arg Gln Lys Gln Asp Leu Tyr Leu Leu Pro Ile Val Ile Ser Asp
                   565                 570                 575
   Gly Gly Ile Pro Pro Met Ser Ser Thr Asn Thr Leu Thr Ile Lys Val
               580                 585                 590
   Cys Gly Cys Asp Val Asn Gly Ala Leu Leu Ser Cys Asn Ala Glu Ala
           595                 600                 605
   Tyr Ile Leu Asn Ala Gly Leu Ser Thr Gly Ala Leu Ile Ala Ile Leu
       610                 615                 620
   Ala Cys Ile Val Ile Leu Leu Val Ile Val Val Leu Phe Val Thr Leu
   625                 630                 635                 640
   Arg Arg Gln Lys Lys Glu Pro Leu Ile Val Phe Glu Glu Glu Asp Val
                   645                 650                 655
   Arg Glu Asn Ile Ile Thr Tyr Asp Asp Glu Gly Gly Gly Glu Glu Asp
               660                 665                 670
   Thr Glu Ala Phe Asp Ile Ala Thr Leu Gln Asn Pro Asp Gly Ile Asn
           675                 680                 685
   Gly Phe Ile Pro Arg Lys Asp Ile Lys Pro Glu Tyr Gln Tyr Met Pro
       690                 695                 700
   Arg Pro Gly Leu Arg Pro Ala Pro Asn Ser Val Asp Val Asp Asp Phe
   705                 710                 715                 720
   Ile Asn Thr Arg Ile Gln Glu Ala Asp Asn Asp Pro Thr Ala Pro Pro
                   725                 730                 735
   Tyr Asp Ser Ile Gln Ile Tyr Gly Tyr Glu Gly Arg Gly Ser Val Ala
               740                 745                 750
   Gly Ser Leu Ser Ser Leu Glu Ser Ala Thr Thr Asp Ser Asp Leu Asp
           755                 760                 765
   Tyr Asp Tyr Leu Gln Asn Trp Gly Pro Arg Phe Lys Lys Leu Ala Asp
       770                 775                 780
   Leu Tyr Gly Ser Lys Asp Thr Phe Asp Asp Asp Ser
   785                 790                 795
<210> 19
   <211> 2598
   <212> DNA
   <213> Homo sapiens
<400> 19
   atggccctcg tactcggctc cctgttgctg ctggggctgt gcgggaactc cttttcagga      60
   gggcagcctt catccacaga tgctcctaag gcttggaatt atgaattgcc tgcaacaaat     120
   tatgagaccc aagactccca taaagctgga cccattggca ttctctttga actagtgcat     180
   atctttctct atgtggtaca gccgcgtgat ttcccagaag atactttgag aaaattctta     240
   cagaaggcat atgaatccaa aattgattat gacaagccag aaactgtaat cttaggtcta     300
   aagattgtct actatgaagc agggattatt ctatgctgtg tcctggggct gctgtttatt     360
   attctgatgc ctctggtggg gtatttcttt tgtatgtgtc gttgctgtaa caaatgtggt     420
   ggagaaatgc accagcgaca gaaggaaaat gggcccttcc tgaggaaatg ctttgcaatc     480
   tccctgttgg tgatttgtat aataataagc attggcatct tctatggttt tgtggcaaat     540
   caccaggtaa gaacccggat caaaaggagt cggaaactgg cagatagcaa tttcaaggac     600
   ttgcgaactc tcttgaatga aactccagag caaatcaaat atatattggc ccagtacaac     660
   actaccaagg acaaggcgtt cacagatctg aacagtatca attcagtgct aggaggcgga     720
   attcttgacc gactgagacc caacatcatc cctgttcttg atgagattaa gtccatggca     780
   acagcgatca aggagaccaa agaggcgttg gagaacatga acagcacctt gaagagcttg     840
   caccaacaaa gtacacagct tagcagcagt ctgaccagcg tgaaaactag cctgcggtca     900
   tctctcaatg accctctgtg cttggtgcat ccatcaagtg aaacctgcaa cagcatcaga     960
   ttgtctctaa gccagctgaa tagcaaccct gaactgaggc agcttccacc cgtggatgca    1020
   gaacttgaca acgttaataa cgttcttagg acagatttgg atggcctggt ccaacagggc 1080
   tatcaatccc ttaatgatat acctgacaga gtacaacgcc aaaccacgac tgtcgtagca 1140
   ggtatcaaaa gggtcttgaa ttccattggt tcagatatcg acaatgtaac tcagcgtctt 1200
   cctattcagg atatactctc agcattctct gtttatgtta ataacactga aagttacatc 1260
   cacagaaatt tacctacatt ggaagagtat gattcatact ggtggctggg tggcctggtc 1320
   atctgctctc tgctgaccct catcgtgatt ttttactacc tgggcttact gtgtggcgtg 1380
   tgcggctatg acaggcatgc caccccgacc acccgaggct gtgtctccaa caccggaggc 1440
   gtcttcctca tggttggagt tggattaagt ttcctctttt gctggatatt gatgatcatt 1500
   gtggttctta cctttgtctt tggtgcaaat gtggaaaaac tgatctgtga accttacacg 1560
   agcaaggaat tattccgggt tttggataca ccctacttac taaatgaaga ctgggaatac 1620
   tatctctctg ggaagctatt taataaatca aaaatgaagc tcacttttga acaagtttac 1680
   agtgactgca aaaaaaatag aggcacttac ggcactcttc acctgcagaa cagcttcaat 1740
   atcagtgaac atctcaacat taatgagcat actggaagca taagcagtga attggaaagt 1800
   ctgaaggtaa atcttaatat ctttctgttg ggtgcagcag gaagaaaaaa ccttcaggat 1860
   tttgctgctt gtggaataga cagaatgaat tatgacagct acttggctca gactggtaaa 1920
   tcccccgcag gagtgaatct tttatcattt gcatatgatc tagaagcaaa agcaaacagt 1980
   ttgcccccag gaaatttgag gaactccctg aaaagagatg cacaaactat taaaacaatt 2040
   caccagcaac gagtccttcc tatagaacaa tcactgagca ctctatacca aagcgtcaag 2100
   atacttcaac gcacagggaa tggattgttg gagagagtaa ctaggattct agcttctctg 2160
   gattttgctc agaacttcat cacaaacaat acttcctctg ttattattga ggaaactaag 2220
   aagtatggga gaacaataat aggatatttt gaacattatc tgcagtggat cgagttctct 2280
   atcagtgaga aagtggcatc gtgcaaacct gtggccaccg ctctagatac tgctgttgat 2340
   gtctttctgt gtagctacat tatcgacccc ttgaatttgt tttggtttgg cataggaaaa 2400
   gctactgtat ttttacttcc ggctctaatt tttgcggtaa aactggctaa gtactatcgt 2460
   cgaatggatt cggaggacgt gtacgatgat gttgaaacta tacccatgaa aaatatggaa 2520
   aatggtaata atggttatca taaagatcat gtatatggta ttcacaatcc tgttatgaca 2580
   agcccatcac aacattga 2598
<210> 20
   <211> 865
   <212> PRT
   <213> Homo sapiens
<400> 20
<210> 21
   <211> 2466
   <212> DNA
   <213> Homo sapiens
<400> 21
   atggtcagct ggggtcgttt catctgcctg gtcgtggtca ccatggcaac cttgtccctg      60
   gcccggccct ccttcagttt agttgaggat accacattag agccagaaga gccaccaacc     120
   aaataccaaa tctctcaacc agaagtgtac gtggctgcgc caggggagtc gctagaggtg     180
   cgctgcctgt tgaaagatgc cgccgtgatc agttggacta aggatggggt gcacttgggg     240
   cccaacaata ggacagtgct tattggggag tacttgcaga taaagggcgc cacgcctaga     300
   gactccggcc tctatgcttg tactgccagt aggactgtag acagtgaaac ttggtacttc     360
   atggtgaatg tcacagatgc catctcatcc ggagatgatg aggatgacac cgatggtgcg     420
   gaagattttg tcagtgagaa cagtaacaac aagagagcac catactggac caacacagaa     480
   aagatggaaa agcggctcca tgctgtgcct gcggccaaca ctgtcaagtt tcgctgccca     540
   gccgggggga acccaatgcc aaccatgcgg tggctgaaaa acgggaagga gtttaagcag     600
   gagcatcgca ttggaggcta caaggtacga aaccagcact ggagcctcat tatggaaagt     660
   gtggtcccat ctgacaaggg aaattatacc tgtgtagtgg agaatgaata cgggtccatc     720
   aatcacacgt accacctgga tgttgtggag cgatcgcctc accggcccat cctccaagcc     780
   ggactgccgg caaatgcctc cacagtggtc ggaggagacg tagagtttgt ctgcaaggtt     840
   tacagtgatg cccagcccca catccagtgg atcaagcacg tggaaaagaa cggcagtaaa     900
   tacgggcccg acgggctgcc ctacctcaag gttctcaagg ccgccggtgt taacaccacg     960
   gacaaagaga ttgaggttct ctatattcgg aatgtaactt ttgaggacgc tggggaatat    1020
   acgtgcttgg cgggtaattc tattgggata tcctttcact ctgcatggtt gacagttctg    1080
   ccagcgcctg gaagagaaaa ggagattaca gcttccccag actacctgga gatagccatt 1140
   tactgcatag gggtcttctt aatcgcctgt atggtggtaa cagtcatcct gtgccgaatg 1200
   aagaacacga ccaagaagcc agacttcagc agccagccgg ctgtgcacaa gctgaccaaa 1260
   cgtatccccc tgcggagaca ggtaacagtt tcggctgagt ccagctcctc catgaactcc 1320
   aacaccccgc tggtgaggat aacaacacgc ctctcttcaa cggcagacac ccccatgctg 1380
   gcaggggtct ccgagtatga acttccagag gacccaaaat gggagtttcc aagagataag 1440
   ctgacactgg gcaagcccct gggagaaggt tgctttgggc aagtggtcat ggcggaagca 1500
   gtgggaattg acaaagacaa gcccaaggag gcggtcaccg tggccgtgaa gatgttgaaa 1560
   gatgatgcca cagagaaaga cctttctgat ctggtgtcag agatggagat gatgaagatg 1620
   attgggaaac acaagaatat cataaatctt cttggagcct gcacacagga tgggcctctc 1680
   tatgtcatag ttgagtatgc ctctaaaggc aacctccgag aatacctccg agcccggagg 1740
   ccacccggga tggagtactc ctatgacatt aaccgtgttc ctgaggagca gatgaccttc 1800
   aaggacttgg tgtcatgcac ctaccagctg gccagaggca tggagtactt ggcttcccaa 1860
   aaatgtattc atcgagattt agcagccaga aatgttttgg taacagaaaa caatgtgatg 1920
   aaaatagcag actttggact cgccagagat atcaacaata tagactatta caaaaagacc 1980
   accaatgggc ggcttccagt caagtggatg gctccagaag ccctgtttga tagagtatac 2040
   actcatcaga gtgatgtctg gtccttcggg gtgttaatgt gggagatctt cactttaggg 2100
   ggctcgccct acccagggat tcccgtggag gaacttttta agctgctgaa ggaaggacac 2160
   agaatggata agccagccaa ctgcaccaac gaactgtaca tgatgatgag ggactgttgg 2220
   catgcagtgc cctcccagag accaacgttc aagcagttgg tagaagactt ggatcgaatt 2280
   ctcactctca caaccaatga ggaatacttg gacctcagcc aacctctcga acagtattca 2340
   cctagttacc ctgacacaag aagttcttgt tcttcaggag atgattctgt tttttctcca 2400
   gaccccatgc cttacgaacc atgccttcct cagtatccac acataaacgg cagtgttaaa 2460
   acatga 2466
<210> 22
   <211> 821
   <212> PRT
   <213> Homo sapiens
<400> 22
<210> 23
   <211> 2649
   <212> DNA
   <213> Homo sapiens
<400> 23
   atgggccctt ggagccgcag cctctcggcg ctgctgctgc tgctgcaggt ctcctcttgg 60
   ctctgccagg agccggagcc ctgccaccct ggctttgacg ccgagagcta cacgttcacg 120
   gtgccccggc gccacctgga gagaggccgc gtcctgggca gagtgaattt tgaagattgc 180
   accggtcgac aaaggacagc ctatttttcc ctcgacaccc gattcaaagt gggcacagat 240
   ggtgtgatta cagtcaaaag gcctctacgg tttcataacc cacagatcca tttcttggtc 300
   tacgcctggg actccaccta cagaaagttt tccaccaaag tcacgctgaa tacagtgggg 360
   caccaccacc gccccccgcc ccatcaggcc tccgtttctg gaatccaagc agaattgctc 420
   acatttccca actcctctcc tggcctcaga agacagaaga gagactgggt tattcctccc 480
   atcagctgcc cagaaaatga aaaaggccca tttcctaaaa acctggttca gatcaaatcc 540
   aacaaagaca aagaaggcaa ggttttctac agcatcactg gccaaggagc tgacacaccc 600
   cctgttggtg tctttattat tgaaagagaa acaggatggc tgaaggtgac agagcctctg 660
   gatagagaac gcattgccac atacactctc ttctctcacg ctgtgtcatc caacgggaat 720
   gcagttgagg atccaatgga gattttgatc acggtaaccg atcagaatga caacaagccc 780
   gaattcaccc aggaggtctt taaggggtct gtcatggaag gtgctcttcc aggaacctct 840
   gtgatggagg tcacagccac agacgcggac gatgatgtga acacctacaa tgccgccatc 900
   gcttacacca tcctcagcca agatcctgag ctccctgaca aaaatatgtt caccattaac 960
   aggaacacag gagtcatcag tgtggtcacc actgggctgg accgagagag tttccctacg 1020
   tataccctgg tggttcaagc tgctgacctt caaggtgagg ggttaagcac aacagcaaca 1080
   gctgtgatca cagtcactga caccaacgat aatcctccga tcttcaatcc caccacgtac 1140
   aagggtcagg tgcctgagaa cgaggctaac gtcgtaatca ccacactgaa agtgactgat 1200
   gctgatgccc ccaatacccc agcgtgggag gctgtataca ccatattgaa tgatgatggt 1260
   ggacaatttg tcgtcaccac aaatccagtg aacaacgatg gcattttgaa aacagcaaag 1320
   ggcttggatt ttgaggccaa gcagcagtac attctacacg tagcagtgac gaatgtggta 1380
   ccttttgagg tctctctcac cacctccaca gccaccgtca ccgtggatgt gctggatgtg 1440
   aatgaagccc ccatctttgt gcctcctgaa aagagagtgg aagtgtccga ggactttggc 1500
   gtgggccagg aaatcacatc ctacactgcc caggagccag acacatttat ggaacagaaa 1560
   ataacatatc ggatttggag agacactgcc aactggctgg agattaatcc ggacactggt 1620
   gccatttcca ctcgggctga gctggacagg gaggattttg agcacgtgaa gaacagcacg 1680
   tacacagccc taatcatagc tacagacaat ggttctccag ttgctactgg aacagggaca 1740
   cttctgctga tcctgtctga tgtgaatgac aacgccccca taccagaacc tcgaactata 1800
   ttcttctgtg agaggaatcc aaagcctcag gtcataaaca tcattgatgc agaccttcct 1860
   cccaatacat ctcccttcac agcagaacta acacacgggg cgagtgccaa ctggaccatt 1920
   cagtacaacg acccaaccca agaatctatc attttgaagc caaagatggc cttagaggtg 1980
   ggtgactaca aaatcaatct caagctcatg gataaccaga ataaagacca agtgaccacc 2040
   ttagaggtca gcgtgtgtga ctgtgaaggg gccgctggcg tctgtaggaa ggcacagcct 2100
   gtcgaagcag gattgcaaat tcctgccatt ctggggattc ttggaggaat tcttgctttg 2160
   ctaattctga ttctgctgct cttgctgttt cttcggagga gagcggtggt caaagagccc 2220
   ttactgcccc cagaggatga cacccgggac aacgtttatt actatgatga agaaggaggc 2280
   ggagaagagg accaggactt tgacttgagc cagctgcaca ggggcctgga cgctcggcct 2340
   gaagtgactc gtaacgacgt tgcaccaacc ctcatgagtg tcccccggta tcttccccgc 2400
   cctgccaatc ccgatgaaat tggaaatttt attgatgaaa atctgaaagc ggctgatact 2460
   gaccccacag ccccgcctta tgattctctg ctcgtgtttg actatgaagg aagcggttcc 2520
   gaagctgcta gtctgagctc cctgaactcc tcagagtcag acaaagacca ggactatgac 2580
   tacttgaacg aatggggcaa tcgcttcaag aagctggctg acatgtacgg aggcggcgag 2640
   gacgactag 2649
<210> 24
   <211> 882
   <212> PRT
   <213> Homo sapiens
<400> 24